# FASCICULE DE BREVET EUROPEEN

(11) **EP 1 435 227 B1**
(45) Date de publication et mention de la délivrance du brevet: **03.12.2008**
(21) Numéro de dépôt: 03104982.8
(22) Date de dépôt: 26.12.2003
(51) Int. Cl.: A61Q 5/06, A61Q 5/10, A61K 8/41, A61K 8/49

(54) **Composition de teinture des fibres kératiniques comprenant un colorant direct monohétéroyldiarylméthane ou un précurseur leuco de celui-ci et procédé de teinture la mettant en oeuvre**
Mittel zur Färbung von Keratinfasern enthaltend einen direktziehenden Farbstoff von typ Monoheteroyldiarylmethan oder eine Leucovorstufe davon sowie Färbemethode damit
Composition for the dyeing of keratinous fibres comprising a direct dye of the monoheteroyldiarylmethane type or a leuco precursor thereof and a dyeing process using it

(30) Priorité: 30.12.2002 FR 0216851
(43) Date de publication de la demande: 07.07.2004
(73) Titulaire: L'ORÉAL, 75008 Paris (FR)
(72) Inventeur: Guerin, Frédéric, 75009 PARIS (FR); Lagrange, Alain, 77700 COUPVRAY (FR)
(74) Mandataire: Poulin, Gérard

(56) Documents cités:
- DE-A- 1 620 564
- US-A- 3 423 427
- US-A- 3 627 893
- US-A- 3 995 088
- US-A- 4 407 960

## Description

La présente invention concerne une composition de teinture des fibres kératiniques, en particulier des fibres kératiniques humaines et, plus particulièrement, des cheveux, qui comprend au moins un composé choisi parmi les colorants directs de type hétéroyldiarylméthane, plus précisément monohétéroyldiarylméthane et les précurseurs leuco de ceux-ci.

L'invention concerne, en outre, un procédé de teinture des fibres kératiniques, en particulier des fibres kératiniques humaines et, plus particulièrement, des cheveux qui met en oeuvre ladite composition.

Il existe de nombreuses compositions et de nombreux procédés pour teindre les fibres kératiniques et, en particulier, les cheveux humains.

Ainsi, il est connu de teindre les fibres kératiniques et, en particulier, les cheveux humains avec des compositions tinctoriales contenant des précurseurs de colorant d'oxydation, en particulier des ortho ou paraphénylènediamines, des ortho ou paraaminophénols, des composés hétérocycliques, tels que des dérivés de diaminopyrazole, appelés généralement « bases d'oxydation ». Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu, d'une part, au niveau des bases d'oxydation et, d'autre part, au niveau des coupleurs, permet l'obtention d'une riche palette de couleurs.

La coloration, dite « permanente », obtenue grâce à ces colorants d'oxydation, doit, par ailleurs, satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, l'ondulation permanente, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles, tout au long d'une même fibre kératinique, qui peut être en effet différemment sensibilisée, c'est-à-dire abîmée entre sa pointe et sa racine.

Il s'avère, en conclusion, que si les associations base-coupleur classiques permettent d'obtenir une palette étendue de couleurs, elles ne permettent pas de satisfaire aux critères énumérés plus haut et, notamment, conduisent souvent à la génération dans la fibre de produits de couplage variés et mal définis, ce qui pose des problèmes d'innocuité et/ou des problèmes de ténacité difficiles à maîtriser, comme par exemple un virage sélectif.

Une autre manière de teindre les fibres kératiniques, en particulier les cheveux, est d'utiliser des colorants directs.

Les colorants classiques qui sont utilisés sont en particulier des colorants du type nitrés benzèniques, anthraquinoniques, nitropyridiniques, azoïques, azoïques cationiques, xanthéniques, acridiniques aziniques, triarylméthanes, benzéniques nitrés ou des colorants naturels.

Ces colorants qui sont des molécules colorées et colorantes ayant une affinité pour les fibres sont appliqués sur les fibres kératiniques pendant un temps nécessaire à l'obtention de la coloration désirée, puis rincés.

L'utilisation des colorants directs est de ce fait très répandue car ils présentent, en outre, certains avantages par rapport aux précurseurs de colorants d'oxydation et, notamment, une diminution des risques potentiels d'allergie et l'absence de sensibilisation du cheveu due au processus oxydatif.

Les colorations obtenues sont cependant temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

Il existe donc un besoin pour une composition de teinture des fibres kératiniques, en particulier des fibres kératiniques humaines et, plus particulièrement, des cheveux, qui soit d'une innocuité, d'une compatibilité, totale, vis-à-vis des fibres kératiniques, qui soit peu sélective, qui donne une grande variété de couleurs, puissantes, et qui permette, en outre, d'obtenir une coloration des fibres kératiniques tenace, stable, résistante aux agents extérieurs, tels que la lumière, les intempéries, le lavage, la transpiration, et les frottements et aux traitements ultérieurs, tels que l'ondulation permanente.

Il existe encore un besoin pour une composition de teinture qui permette le traitement de toutes sortes de fibres kératiniques, de tous types de cheveux, par exemple des cheveux blancs, même si ces cheveux ont subi préalablement un traitement, tel qu'un traitement de décoloration ou d'ondulation permanente.

Il existe enfin un besoin pour une composition qui réalise la teinture sans qu'il soit nécessaire de réaliser, au préalable, une sensibilisation de la fibre kératinique, du cheveu.

Le but de la présente invention est de fournir une composition de teinture des fibres kératiniques, en particulier des fibres kératiniques humaines et, plus particulièrement, des cheveux qui réponde, entre autres, aux besoins énumérés ci-dessus.

Le but de la présente invention est encore de fournir une composition de teinture des fibres kératiniques qui ne présente pas les inconvénients, défauts, limitations et désavantages des compositions de teinture de l'art antérieur, qu'il s'agisse des compositions de teinture mettant en oeuvre une association base-coupleur, ou des compositions mettant en oeuvre un colorant direct.

Ce but et d'autres encore sont atteints, conformément à l'invention, par une composition pour la teinture des fibres kératiniques comprenant dans un milieu cosmétique approprié pour la teinture, au moins un colorant choisi parmi les composés répondant à la formule (I), (Ibis), (II) ou (IIbis) suivante ou leurs formes tautomères, et leurs sels d'addition : 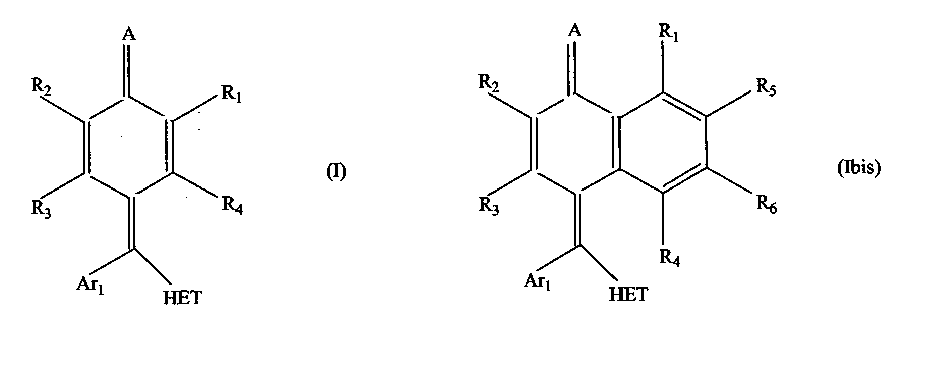 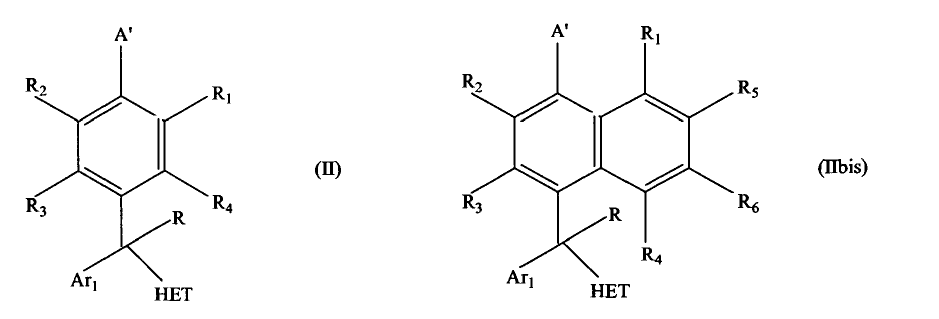 dans lesquelles :
- Ar₁ représente un radical aryle, tel que phényle ou naphtyle, éventuellement substitué par un ou plusieurs groupements Z ;
- HET représente un hétérocycle éventuellement substitué par un ou plusieurs groupements Z' ;
- A représente O ; NH ; N-alkyle ; N-hydroxyalkyle ; ammonium, N-alkylammonium, N-hydroxyalkylammonium, N,N-dialkylammonium, N,N-di(hydroxyalkyl) ammonium ou N-(hydroxyalkyl), N-(alkyl)ammonium, dans lesquels les deux groupements alkyle peuvent former avec l'atome d'azote auquel ils sont liés un cycle pouvant être interrompu par un ou plusieurs atomes d'azote, d'oxygène ou de soufre ;
- R₁ à R₆ et Z, Z' désignent indépendamment l'un de l'autre un groupement choisi parmi l'atome d'hydrogène, les atomes d'halogène, tels que F, Cl, Br et I, les radicaux -NHSO₃H ; hydroxyle ; alkyle ; alcoxy ; alkylthio ; monoalkylamino ; dialkylamino, dans lesquels les deux groupements alkyle peuvent conjointement, avec l'atome d'azote, auquel ils sont liés, former un cycle pouvant être interrompu par un ou plusieurs atomes d'azote, d'oxygène ou de soufre ; les hétérocycles et les radicaux nitro ; aryle ; acyle ; alcoxycarbonyle ; carboxamido ; cyano ; -CO₂H ; -SO₃H ; -PO₃H₂ ; -PO₄H₂ ;
- A' représente un atome d'hydrogène ; un groupe hydroxyle ; amino ; (hydroxyalkyl)amino ; monoalkylamino ; di(hydroxyalkyl)amino, (hydroxyalkyl)(alkyl)amino, (dialkyl)amino, dans lesquels les deux groupements alkyle peuvent former avec l'atome d'azote auquel ils sont liés un cycle pouvant être interrompu par un ou plusieurs atomes d'azote, d'oxygène ou de soufre ;
- R représente un atome d'hydrogène ou d'halogène ; un radical hydroxyle ; un radical alcoxy ; ou un radical alkylthio, à l'exception du bis-(4-hydroxyphényl) (quinolyl-2)méthane et du chlorydrate de bis -(4-hydroxyphényl)-(quinolyl-2) méthane.

Selon une forme de réalisation avantageuse, HET représente un hétérocycle insaturé, de préférence un hétérocycle aromatique, éventuellement substitué par un ou plusieurs groupements Z'.

Dans les formules (I), (Ibis) et (II), (IIbis) ci -dessus, le terme alkyle utilisé pour les radicaux alkyle, ainsi que pour les groupements comportant une partie alkyle, signifie, sauf indication différente, une chaîne carbonée, linéaire ou ramifiée, comportant de 1 à 30 atomes de carbone, de préférence de 1 à 8, mieux de 1 à 4, pouvant être portée et/ou interrompue par un ou plusieurs atomes d'oxygène de soufre ou d'azote, pouvant être substituée par un ou plusieurs groupes choisis parmi les atomes d'halogène, tel le chlore, le brome, l'iode et le fluor ; les hétérocycles ; les radicaux aryle ; hydroxyle ; alcoxy ; amino ; acyle ; carboxamido ; -CO₂H ; -SO₃H ; -PO₃H₂ ; -PO₄H₂ ; -NHSO₃H ; sulfonamide ; monoalkylamino ; trialkylammonium ; ou bien encore par un radical dialkylamino, dans lequel les deux groupements alkyle peuvent former, conjointement avec l'atome d'azote dudit groupement dialkyl(C₁-C₄)amino, auquel ils sont liés, un cycle pouvant être interrompu par un ou plusieurs atomes d'azote, d'oxygène ou de soufre.

De même, selon l'invention, le terme alcoxy utilisé pour les radicaux alcoxy ainsi que pour les groupements comportant une partie alcoxy, signifie, sauf indication différente, une chaîne O-alkyle, le terme alkyle ayant la signification indiquée ci-dessus. Les radicaux alcoxy des groupes alcoxycarbonyle ont, de préférence, de 1 à 4 atomes de carbone. Les groupes acyle ont, de préférence, de 2 à 4 atomes de carbone.

Selon l'invention, on entend par hétérocycle, un cycle aromatique ou non contenant 5, 6 ou 7 sommets, et de 1 à 3 hétéroatomes choisis parmi les atomes d'azote, de soufre et d'oxygène. Ces hétérocycles peuvent être condensés sur d'autres hétérocycles, ou sur d'autres cycles notamment aromatiques tels qu'un groupement phényle. Ces hétérocycles peuvent, en outre, être quaternisés par un radical alkyle. Les termes alkyle et alcoxy ont les significations indiqués ci-dessus.

Parmi, les hétérocycles, et notamment ceux de HET, on peut notamment citer à titre d'exemple les cycles : thiophène, benzothiophène, furane, benzofurane, indole, indoline, carbazole, pyridine, déhydroquinoléine, chromone, julodinine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

Selon l'invention, on entend par aryle, sauf spécifié autrement, un radical aryle en C₆ à C₃₀ pouvant être substitué par un ou plusieurs radicaux alkyle ; alcoxy ; acyle ; cyano ; carboxamido ; -CO₂H ; -SO₃H ; -PO₃H₂ ; -PO₄H₂ ; hydroxyle ; amino ; monoalkyl(C₁-C₄)amino ; ou dialkyl(C₁-C₄) amino ; dans lequel les deux groupements alkyle peuvent former, conjointement avec l'atome d'azote dudit groupement dialkyl(C₁-C₄)amino, auquel ils sont liés, un cycle pouvant être interrompu par un ou plusieurs atomes d'azote, d'oxygène ou de soufre. De préférence, le groupe aryle est un groupe phényle ou un groupe naphtyle pouvant être substitué comme indiqué ci-dessus.

Les composés (I) et (Ibis) peuvent être définis comme étant des colorants directs du type hétéroyldiarylméthane et les composés de formules (II), (IIbis) sont les précurseurs leuco des hétéroyldiarylméthane de formule (I) et (Ibis).

Les composés leuco (II) ou (IIbis) sont généralement peu ou pas colorés et peuvent être transformés par simple oxydation à l'air ou en présence d'un agent oxydant en un composé hétéroyldiarylméthane de formule (I) ou (Ibis).

A titre de composés de formule (I), (Ibis), (II), (IIbis), utilisables dans le cadre de la présente invention, on peut citer les composés suivants pour lesquels les contre ions sont ou non spécifiés : Méthanaminium, N-[4-[[4-(diméthylamino)phényl](2,2-diméthyl-2H-1-benzopyran-3-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-3-yl)méthylène]-2,5-cyclohexadièn-1-ylidène]-N-méthyl-Méthanaminium, N-[4-[[4-(diméthylamino)phényl](2,2-diméthyl-2H-1-benzopyran-5-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-5-yl)méthylène]-2,5-cyclohexadièn-1-ylidène]-N-méthyl-Méthanaminium, N-[4-[[4-(diméthylamino)phényl](2,2-diméthyl-2H-1-benzopyran-4-yl)méthylène]-2,5-cyclohexadièn-1-ylidène]-Nméthyl-, acétate Méthanaminium, N-[4-[[4-(diméthylamino)phényl](2,2-diméthyl-2H-1-benzopyran-4-yl)méthylène]-2,5-cyclohexadièn-1-ylidène]-N-méthyl-Méthanaminium, N-[4-[[4-(diméthylamino)phényl](2,2-diméthyl-2H-1-benzopyran-8-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-8-yl)méthylène]-2,5-cyclohexadièn-1-ylidène]-N-méthyl-Méthanaminium, N-[4-[[4-(diméthylamino)phényl](2,2-diméthyl-2H-1-benzopyran-7-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-7-yl)méthylène]-2,5-cyclohexadièn-1-ylidène]-N-méthyl-Méthanaminium, N-[4-[[4-(diméthylamino)phényl](2,2-diméthyl-2H-1-benzopyran-6-yl)méthylène]-2,5-cyclohexadièn-1-ylidène]-N-méthyl-, acétate Méthanaminium, N-[4-[4-(diméthylamino)phényl](2,2-diméthyl-2H-1-benzopyran-6-yl)méthylène]-2,5-cyclohexadièn-1-ylidène]-N-méthyl-2,5-Cyclohexadièn-1-one, 4-[[4-amino-3,5-bis(1-méthyléthyl)phényl](4-éthyl-3,4-dihydro-2H-1,benzoxazin-7-yl)méthylène]-2,6-diméthoxy 2,5-Cyclohexadièn-1-one, 4-[[3,4-dihydro-4-(2-pyridinylméthyl)-2H-1,4-benzoxazin-7-yl](4-éthyl-3,4-dihydro-2H-1,4-benzoxazin-7-yl)méthylène]-2,6-diméthoxy-2,5-Cyclohexadièn-1-one, 4-[[4-(diméthylamino)phényl](4-éthyl-3,4-dihydro-2H-1,4-benzoxazin-7-yl)méthylène]-2,6-diméthoxy-2,5-Cyclohexadièn-1-one, 2,6-bis(1,1-diméthyléthyl)-4-[(5-éthyl-2-thiènyl)phénylméthylène]-Éthanaminium, N-[3-carboxy-4-[[2-carboxy-4-[éthyl (2-méthoxyéthyl)amino]phényl](7-carboxy-4-oxo-4H-1-benzothiopyran-3-yl)méthylène]-2,5-cyclohexadièn-1-ylidène]-N-éthyl-2-méthoxy Éthanaminium, N-[4-[[4-[bis(2-méthoxyéthyl)amino]-2-carboxyphényl](5-carboxy-2-pyridinyl)méthylène]-3-carboxy-2,5-cyclohexadièn-1-ylidène]-2-méthoxy-N-(2-méthoxyéthyl)-, Éthanaminium, N-[4-[3-benzofuranyl[2-carboxy-4-[éthyl[2-[(méthylsulfonyl)amino]éthyl]amino]phényl]méthylène]-3-carboxy-2,5-cyclohexadièn-1-ylidène]-N-éthyl-2-[(méthylsulfonyl)amino]-, perchlorate Éthanaminium, N-[4-[3-benzofuranyl[2-carboxy-4-[éthyl[2-[(méthylsulfonyl)amino]éthyl]amino]phényl]méthylène]-3-carboxy-2,5-cyclohexadièn-1-ylidène]-N-éthyl-2-[(méthylsulfonyl)amino]-Éthanaminium, N-[4-[[4-[bis(2-méthoxyéthyl)amino]-2-carboxyphényl](4-oxo-4H-1-benzothiopyran-3-yl)méthylène]-3-carboxy-2,5-cyclohexadièn-1-ylidène]-2-méthoxy-N-(2-méthoxyéthyl)-, acétate Éthanaminium, N-[4-[[4-[bis(2-méthoxyéthyl)amino]-2-carboxyphényl](4-oxo-4H-1-benzothiopyran-3-yl)méthylène]-3-carboxy-2,5-cyclohexadièn-1-ylidène]-2-méthoxy-N-(2-méthoxyéthyl)-Sel d'acide Benzoique et de, 5-[(3-carboxy-4-oxo-2,5-cyclohexadièn-1-ylidène)-3-pyridinylméthyl]-2-hydroxy-Méthanaminium, N-[2-carboxy-4-[[3-carboxy-4-(diméthylamino)phényl](4-oxo-4H-1-benzothiopyran-3-yl)méthylène]-2,5-cyclohexadièn-1-ylidène]-N-méthyl-, perchlorate Méthanaminium, N-[2-carboxy-4-[[3-carboxy-4-(diméthylamino)phényl](4-oxo-4H-1-benzothiopyran-3-yl)méthylène]-2,5-cyclohexadièn-1-ylidène]-N-méthyl-Sel d'acide benzoique et de 5-[(3-carboxy-4-oxo-2,5-cyclohexadièn-1-ylidène)[3,5-diméthyl-1-[2-[(méthylsulfonyl)amino]éthyl]-1H-pyrazol-4-yl]méthyl]-2-hydroxy-Sel d'acide benzoique et de 5-[[2-[bis(2-méthoxyéthyl)amino]-5-thiazolyl](3-carboxy-4-oxo-2,5-cyclohexadièn-1-ylidène)méthyl]-2-hydroxy-Éthanaminium, N-[4-[(7-carboxy-2-oxo-2H-1-benzopyran-4-yl)[4-(diéthylamino)phényl]méthylène]-2,5-cyclohexadièn-1-ylidène]-N-éthyl-, perchlorate Éthanaminium, N-[4-[(7-carboxy-2-oxo-2H-1-benzopyran-4-yl)[4-(diéthylamino)phényl]méthylène]-2,5-cyclohexadièn-1-ylidène]-N-éthyl-Éthanaminium, N-[4-[2-benzofuranyl[4-[éthyl[2-[(méthylsulfonyl)amino] éthyl]amino]phényl]méthylène]-2,5-cyclohexadièn-1-ylidène]-N-éthyl-2-[(méthylsulfonyl)amino]-, perchlorate Éthanaminium, N-[4-[2-benzofuranyl[4-[éthyl[2-[(méthylsulfonyl)amino]éthyl]amino]phényl]méthylène]-2,5-cyclohexadièn-1-ylidène]-N-éthyl-2-[(méthylsulfonyl)amino] - Éthanaminium, N-[4-[(6-carboxybenzo[b]thièn-2-yl)[4-(diéthylamino)phényl]méthylène]-2,5-cyclohexadièn-1-ylidène]-N-éthyl-, tétrafluoroborate(1-) Éthanaminium, N-[4-[(6-carboxybenzo[b]thièn-2-yl)[4-(diéthylamino)phényl]méthylène]-2,5-cyclohexadièn-1-ylidène]-N-éthyl-Éthanaminium, N-[4-[[5-[bis(2-méthoxyéthyl)amino]-2-furanyl][4-[éthyl[2-[(méthylsulfonyl)amino] éthyl] amino]phényl] méthylène]-2,5-cyclohexadièn-1-ylidène]-N-éthyl-2-[(méthylsulfonyl)amino]-, perchlorate Éthanaminium, N-[4-[[5-[bis(2-méthoxyéthyl)amino]-2-furanyl][4-[éthyl[2-[(méthylsulfonyl)amino]éthyl]amino]phényl]méthylène]-2,5-cyclohexadièn-1-ylidène]-N-éthyl-2-[(méthylsulfonyl)amino]-2,5-Cyclohexadièn-1-one, 4-[(4-hydroxyphényl)-4-quinolinylméthylène]-Éthanaminium,N-[4-[[4-[bis(2-méthoxyéthyl)aminolphényl](7-carboxy-4-oxo-4H-1-benzopyran-3-yl)méthylène]-2,5-cyclohexadièn-1-ylidène]-2-méthoxy-N-(2-méthoxyéthyl)-, sulfate (1:1) Éthanaminium, N-[4-[[4-[bis(2-méthoxyéthyl)amino]phényl](7-carboxy-4-oxo-4H-1-benzopyran-3-yl)méthylène]-2,5-cyclohexadièn-1-ylidène]-2-méthoxy-N-(2-méthoxyéthyl)-Méthanaminium, N-[4-[(5-carboxy-2-pyridinyl)[4-(diméthylamino)phényl]méthylène] 2,5-cyclohexadièn-1-ylidène]-N-méthyl-, perchlorate Méthanaminium, N-[4-[(5-carboxy-2-pyridinyl)[4-(diméthylamino)phényl]méthylène] -2,5-cyclohexadièn-1-ylidène]-N-méthyl-Acide 2-Thiophénecarboxylique, 5-[(4-hydroxyphényl)(4-oxo-2,5-cyclohexadièn-1-ylidène)méthyl]-2,5-Cyclohexadièn-1-one, 4-[2-furanyl(4-hydroxyphényl) méthylène]- 2,5-Cyclohexadièn-1-one, 4-[(4-hydroxyphényl)-2-pyridinylméthylène]-Méthanaminium, N-[3-carboxy-4-[[2-carboxy-4-(diméthylamino)phényl](4-oxo-4H-1-benzopyran-3-yl)méthylène]-2,5-cyclohexadièn-1-ylidène]-N-méthyl, perchlorate Méthanaminium, N-[3-carboxy-4-[[2-carboxy-4-(diméthylamino)phényl](4-oxo-4H-1-benzopyran-3-yl)méthylène]-2,5-cyclohexadièn-1-ylidène]-N-méthyl-Éthanaminium, N-[4-[(5-carboxy-2-thiènyl)[4-(diéthylamino)phényl]méthylène]-2,5-cyclohexadièn-1-ylidène]-N-éthyl-, perchlorate Éthanaminium, N-[4-[(5-carboxy-2-thiènyl)[4-(diéthylamino)phényl]méthylène]-2,5-cyclohexadièn-1-ylidène]-N-éthyl-2,5-Cyclohexadièn-1-one, 4-[(4-méthoxyphényl)-3,6,9,12,15-pentaoxabicyclo[15.3.1]héneicosa-1(21),17,19-trièn-21-ylméthylène]-3,6,9,12,15-Pentaoxabicyclo[15.3.1]héneicosane, 2,5-cyclohexadièn-1-one Méthanaminium, N-[4-[(2,3-dihydro-3-méthyl-2-benzothiazolyl)[4-(diméthylamino)phényl]méthylène]-2,5-cyclohexadièn-1-ylidène]-N-méthyl-, chlorure [4-(α-Benzo[b]thièn-2-yl-p-diméthylaminobenzylidène)-2,5-cyclohexadièn-1-ylidène] diméthylammonium chlorure [4-(α-Benzo[b]thièn-3-yl-p-diméthylaminobenzylidène)-2,5-cyclohexadièn-1-ylidène] diméthylammonium chlorure Méthanaminium, N-[4-[[9-(diméthylamino)benzo[a]phénoxazin-7-ium-5-yl][4-(diméthylamino)phényl]méthylène]-2,5-cyclohexadièn-1-ylidène]-N-méthyl-, bis[tétraphénylborate(1-)] Méthanaminium Benzo[a]phénoxazin-7-ium, Méthanaminium, N-[4-[[9-(diméthylamino)benzo[a]phénoxazin-7-ium-5-yl][4-(diméthylamino) phényl]méthylène]-2,5-cyclohexadièn-1-ylidène]-N-méthyl-[4-(α-3-Bromobenzo[b]thièn-2-yl-p-diméthylaminobenzylidène)-2,5-cyclohexadièn-1-ylidène]diméthylammonium chlorure [4-(α-3-Chlorobenzo[b]thièn-2-yl-p-diméthylminobenzylidène)-2,5-cyclohexadièn-1-ylidène]diméthylammonium chlorure [4-(p-Diméthylamino-α-3-méthylbenzo[b]thièn-2-ylbenzylidène)-2,5-cyclohexadièn-1-ylidène]diméthylammonium chlorure [4-[2-Bromo-α-(p-diméthylaminophényl)-3-thénylidène]-2,5-cyclohexadièn-1-ylidène]diméthylammonium chlorure [4-[2-Chloro-α-(p-diméthylaminophényl)-3-thénylidène]-2,5-cyclohexadièn-1-ylidène] diméthylammonium chlorure [4-[α-(p-Diméthylaminophényl)-3-thénylidène]-2,5-cyclohexadièn-1-ylidène]diméthylammonium chlorure [4-[α-(p-Diméthylaminophényl)-2,5-diméthyl-3-thénylidène]-2,5-cyclohexadièn-1-ylidène] diméthylammonium chlorure Pyridinium, 1-[[4-(diméthylamino)phényl][4-(diméthyliminio)-2,5-cyclohexadièn-1-ylidène]méthyl]-, bis[tétrafluoroborate(1-)] 2,5-Cyclohexadièn-1-one, 2,3,5,6-tétrachloro-4-[(pentachlorophényl)(2,3,5,6-tétrachloro-4-pyridinyl)méthylène]-Éthanaminium, N-[4-[[4-(diéthylamino)phényl](4-oxo-4H-1-benzothiopyran-3-yl)méthylène]-2,5-cyclohexadièn-1-ylidène]-N-éthyl-, perchlorate Éthanaminium, N-[4-[[4-(diéthylamino)phényl](4-oxo-4H-1-benzothiopyran-3-yl)méthylène]-2,5-cyclohexadièn-1-ylidène]-N-éthyl-4H-1-Benzothiopyran, éthanaminium deriv. Benzènaminium, N-méthyl-N-[4-[[4-(méthylphénylamino)phényl](4-oxo-4H-1-benzothiopyran-3-yl)méthylène]-2,5-cyclohexadièn-1-ylidène]-, perchlorate Benzènaminium, N-méthyl-N-[4-[[4-(méthylphénylamino)phényl](4-oxo-4H-1-benzothiopyran-3-yl)méthylène]-2,5-cyclohexadièn-1-ylidène]-Éthanaminium, N-[4-[[4-(diéthylamino)-2-méthylphényl](4-oxo-4H-1-benzothiopyran-3-yl)méthylène]-3-méthyl-2,5-cyclohexadièn-1-ylidène]-N-éthyl-, perchlorate Éthanaminium, N-[4-[[4-(diéthylamino)-2-méthylphényl](4-oxo-4H-1-benzothiopyran-3-yl)méthylène]-3-méthyl-2,5-cyclohexadièn-1-ylidène]-N-éthyl-Ethanaminium 4H-1-Benzothiopyran, Methanaminium, N-[4-[[4-(diméthylamino)phényl][5-(4-iodophényl)-2-furanyl]méthylène]-2,5-cyclohexadièn-1-ylidène]-N-méthyl-, chlorure Méthanaminium, N-[4-[[5-(4-bromophényl)-2-furanyl][4-(diméthylamino)phényl]méthylène]-2,5-cyclohexadièn-1-ylidène]-N-méthyl-, chlorure Méthanaminium, N-[4-[[4-(diméthylamino)phényl][5- (4-nitrophényl) -2-furanyl]méthylène]-2,5-cyclohexadièn-1-ylidène]-N-méthyl-, chlorure [Oxybis[méthylène-5,2-furandiyl[p-(N-méthylanilino)benzylidyne]-2,5-cyclohexadiène-4,1-diylidène]]bis[méthylphénylammonium chlorure] [Oxybis[méthylène-5,2-furandiyl[p-(N-méthylanilino)benzylidyne]-2,5-cyclohexadiène-4,1-diylidène]]bis[méthylphénylammonium hydrogènosulfate] Benzènaminium, N,N'-[oxybis[méthylène-5,2-furandiyl[[4-(méthylphénylamino)phényl]méthylidyne]-2,5-cyclohexadiène-4,1-diylidène]]bis[N-méthyl-Sel d'acide 1,4-Cyclohexadiène-1-sulfonique et de, 3-[4-anilino-α-(1-méthyl-2-phénylindol-3-yl)-3-sulfobenzylidène]-6-phénylimino-Sel d'acide trifluorométhane sulfonique et de Pyridinium, 1-[[4-(diméthylamino)phényl][4-(diméthyliminio)-2,5-cyclohexadièn-1-ylidène]méthyl Sel d'acide trifluorométhane sulfonique et de 1-[[4-(diméthylamino)phényl][4-(diméthyliminio)-2,5-cyclohexadièn-1-ylidène]méthyl]pyridinium Pyridinium, 1-[[4-(diméthylamino)phényl][4-(diméthyliminio)-2,5-cyclohexadièn-1-ylidène]méthyl]-[4-[p-(Diméthylamino)-α-(9-méthylcarbazol-3-yl)benzylidène]-2,5-cyclohexadièn-1-ylidène]diméthylammonium chlorure [4-[α-[p-(Diméthylamino)phényl]furfurylidène]-2,5-cyclohexadièn-1-ylidène]diméthylammonium chlorure Méthanaminium, N-[4-[bis(1,2,3,4,10,14b-hexahydro-2-méthyldibenzo[c,f]pyrazino [1,2-a]azépin-8-yl)méthylène]-2,5-cyclohexadièn-1-ylidène]-N-méthyl-, perchlorate, diperchlorate Méthanaminium, N-[4-[bis(1,2,3,4,10,14b-hexahydro-2-méthyldibenzo[c,f]pyrazino[1,2-a]azépin-8-yl)méthylène]-2,5-cyclohexadièn-1-ylidène]-N-méthyl-, perchlorate Méthanaminium, N-[4-[bis(1,2,3,4,10,14b-hexahydro-2-méthyldibenzo[c,f]pyrazino [1,2-a]azépin-8-yl)méthylène]-2,5-cyclohexadièn-1-ylidène]-N-méthyl-1H-Indolizinium, 3-[[4-(diméthyliminio)-2,5-cyclohexadièn-1-ylidène](4-méthoxyphényl)méthyl]-2- (2-hydroxy-5-méthylphényl)-1-méthyl-, 1H-Indolizinium, 3-[[4-(diméthyliminio)-2,5-cyclohexadièn-1-ylidène]phénylméthyl]-2-(2-hydroxy-5-méthylphényl)-1-méthyl-, 1H-Indolizinium, 3-[[4-(diméthylamino)phényl][4-(diméthyliminio)-2,5-cyclohexadièn-1-ylidène]méthyl]-2-(2-hydroxy-5-méthylphényl)-1-méthyl-, 1H-Indolizinium, 3-[[4-(diméthylamino)phényl][4-(diméthyliminio)-2,5-cyclohexadièn-1-ylidène]méthyl]-2-(2-hydroxy-5-méthylphényl)-1-méthyl-1-Propanaminium, N-[4-[2-benzofuranyl[3-chloro-4-[éthyl(3-sulfopropyl)amino] phényl]méthylène]-2-chloro-2,5-cyclohexadièn-1-ylidène]-N-éthyl-3-sulfo-, sel interne, sel de sodium 1-Prcpanaminium, N-[4-[2-benzofuranyl[4-[éthyl(3-sulfopropyl)amino]-3-méthylphényl]méthylène]-2-méthyl-2,5-cyclohexadièn-1-ylidène]-N-éthyl-3-1-Butanaminium, N-[4-[2-benzofuranyl[4-[éthyl(4-sulfobutyl)amino]phényl]méthylène]-2,5-cyclohexadièn-1-ylidènel-N-éthyl-4-1-Propanaminium, N-[4-[(5-chlorobenzo[b]thièn-2-yl)[4-[méthyl(3-sulfopropyl)amino]phényl]méthylène]-2,5-cyclohexadièn-1-ylidène]-N-méthyl-3-sulfo-, 1-Propanaminium, N-[4-[(3-chlorobenzo[b]thièn-2-yl)[4-[méthyl(3-sulfopropyl)amino]phényl]méthylène]-2,5-cyclohexadièn-1-ylidène]-N-méthyl-3-sulfo-, sel de sodium 1-Propanaminium, N-[4-[benzo[b]thièn-2-yl[4-[méthyl(3-sulfopropyl)amino]phényl]méthylène]-2,5-cyclohexadièn-1-ylidène]-N-méthyl-3-sulfo-, sel de sodium 1-Propanaminiun, N-méthyl-N-[4-[(3-méthylbenzo[b]thièn-2-yl)[4-[méthyl(3-sulfopropyl)amino]phényl]méthylène]-2,5-cyclohexadièn-1-ylidène]-3-sulfo-, sel de sodium 1-Propanaminium, N-[4-[(3-bromobenzo[b]thièn-2-yl)[4-[méthyl(3-sulfopropyl)amino]phényl]méthylène]-2,5-cyclohexadièn-1-ylidène]-N-méthyl-3-sulfo-, sel de sodium 1-Propanaminium, N-[4-[(6-chlorobenzo[b]thièn-2-yl)[4-[méthyl(3-sulfopropyl)amino]phényl]méthylène]-2,5-cyclohexadièn-1-ylidène]-N-méthyl-3-sulfo-, sel de sodium 1-Propanaminium, N-méthyl-N-[4-[[4-[méthyl(3-sulfopropyl)amino]phényl](5-nitro-2-benzofuranyl)méthylène]-2,5-cyclohexadièn-1-ylidène]-3-sulfo-, sel de sodium 1-Propanaminium, N-méthyl-N-[4-[(5-méthyl-2-benzofuranyl)[4-[méthyl(3-sulfopropyl)amino]phényl]méthylène]-2,5-cyclohexadièn-1-ylidène]-3-sulfo-, sel de sodium 1-Propanaminium, N-[4-[(5-chloro-2-benzofuranyl)[4-[méthyl(3-sulfopropyl)amino]phényl]méthylène]-2,5-cyclohexadièn-1-ylidène]-N-méthyl-3-sulfo-, sel de sodium 1-Propanaminium, N-[4-[2-benzofuranyl[4-[méthyl(3-sulfopropyl)amino]phényl]méthylène]-2,5-cyclohexadièn-1-ylidène]-N-méthyl-3-sulfo-, sel interne, sel de sodium Éthanaminium, N-[4-[(2-chlorophényl)(1-méthyl-2-phényl-1H-indol-3-yl)méthylène]-2,5-cyclohexadièn-1-ylidène]-N-éthyl-, trichlorozincate(1-) Zincate(1-), trichloro-, N-[4-[(2-chlorophényl)(1-méthyl-2-phényl-1H-indol-3-yl)méthylène]-2,5-cyclohexadièn-1-ylidène]-N-éthyléthanaminium Benzènesulfonic acid, 5-[(1,2-diméthyl-1H-indol-3-yl)[4-[(2-méthylphényl)imino]-3-sulfo-2,5-cyclohexadièn-1-ylidène]méthyl]-2- [(2-méthylphényl)amino]-, monosel de sodium Éthanaminium, N-[4-[(2-chlorophényl)(1-méthyl-2-phényl-1H-indol-3-yl)méthylène]-2,5-cyclohexadièn-1-ylidène]-N-éthyl-, méthyl sulfate 3H-Pyrazol-3-one, 1,2-dihydro-4-[(4-hydroxy-3-méthoxyphényl)(3-méthoxy-4-oxo-2,5-cyclohexadièn-1-ylidène)méthyl]-1,5-diméthyl-2-phényl-, ion(1-) Éthanaminium, N-[4-[(2-chlorophényl)(1-méthyl-2-phényl-1H-indol-3-yl)méthylène]-2,5-cyclohexadièn-1-ylidène]-N-éthyl-, acétate Éthanaminium, N-[4-[(2-chlorophényl)(1-méthyl-2-phényl-1H-indol-3-yl)méthylène]-2,5-cyclohexadièn-1-ylidène]-N-éthyl-, phosphate (1:1) Éthanaminium, N-[4-[(2-chlorophényl)(1-méthyl-2-phényl-1H-indol-3-yl)méthylène]-2,5-cyclohexadièn-1-ylidène]-N-éthyl-Éthanaminium, N-[4-[(2-chlorophényl)(1-méthyl-2-phényl-1H-indol-3-yl)méthylène]-2,5-cyclohexadièn-1-ylidène]-N-éthyl-, chlorure, composé avec le chlorure de zinc (ZnCl₂) N-[4-[(2-chlorophényl)(1-méthyl-2-phényl-1H-indol-3-yl)méthylène]-2,5-cyclohexadièn-1-ylidène]-N-éthyléthanaminium chlorure 1-Pipéridinyloxy, 4-[[3,5-bis(1,1-diméthyléthyl)-4-oxo-2,5-cyclohexadièn-1-ylidène][3,5-bis(1,1-diméthyléthyl)-4-oxyphényl]méthyl]-2,2,6,6-tétraméthyl-1H-Pyrrol-1-yloxy, 3-[[3,5-bis(1,1-diméthyléthyl)-4-oxo-2,5-cyclohexadièn-1-ylidène][3,5-bis(1,1-diméthyléthyl)-4-oxyphényl]méthyl]-2,5-dihydro-2,2,5,5-tétraméthyl-1H-Pyrrol-1-yloxy, 3-[[3,5-bis(1,1-diméthyléthyl)-4-hydroxyphényl][3,5-bis(1,1-diméthyléthyl)-4-oxo-2,5-cyclohexadièn-1-ylidène]méthyl]-2,5-dihydro-2,2,5,5-tétraméthyl-1-Pipéridinyloxy, 4-[[3,5-bis(1,1-diméthyléthyl)-4-hydroxyphényl][3,5-bis(1,1-diméthyléthyl)-4-oxo-2,5-cyclohexadièn-1-ylidène]méthyl]-2,2,6,6-tétraméthyl-Acétamide, N-méthyl-N-[[2-[[4-oxo-3-[2,2,2-trifluoro-1-hydroxy-1-(trifluorométhyl)éthyl]-1(4H)-naphthalènylidène](2,3,6,7-tétrahydro-1H,5H-benzo[ij]quinolizin-9-yl)méthyl]phényl]sulfonyl]-Ester d'acide carbamique et de [[2-[(3,5-diméthoxy-4-oxo-2,5-cyclohexadièn-1-ylidène)(2,3,6,7-tétrahydro-1H,5H-benzo[ij]quinolizin-9-yl)méthyl]phényl]sulfonyl]méthyl-, 2-(méthylsulfonyl)éthyl Ester d'acide carbamique et de, méthyl[[2-[[4-oxo-3-[2,2,2-trifluoro-1-hydroxy-1-(trifluorométhyl)éthyl]-1(4H)-naphthalènylidène](2,3,6,7-tétrahydro-1H,5H-benzo[ij]quinolizin-9-yl)méthyl]phényl]sulfonyl]-, 2-cyanoéthyl Ester d'acide carbamique et de méthyl[[2-[[4-oxo-3-[2,2,2-trifluoro-1-hydroxy-1-(trifluorométhyl)éthyl]-1(4H)-naphthalènylidène](2,3,6,7-tétrahydro-1H,5H-benzo[ij]quinolizin-9-yl)méthyl]phényl]sulfonyl]-, 2-(méthylsulfonyl)éthylester Ester d'acide Carbamique et de méthyl[[2-[[4-oxo-3-[2,2,2-trifluoro-1-hydroxy-1- (trifluorométhyl)éthyl]-1 (4H) -naphthalènylidène](2,3,6,7-tétrahydro-1H,5H-benzo[ij]quinolizin-9-yl)méthyl]phényl]sulfonyl]-, 2-(phénylsulfonyl)éthyl Acétamide, N-[[2-[(3,5-diméthyl-4-oxo-2,5-cyclohexadièn-1-ylidène)(2,3,6,7-tétrahydro-1H,5H-benzo[ij]quinolizin-9-yl)méthyl]sulfonyl]-N-méthyl-2,5-Cyclohexadièn-1-one, 4-[(4-hydroxy-3,5-diméthylphényl)(2,3,5,6,8,9,11,12-octahydro-1,4,7,10,13-benzopentaoxacyclopentadecin-15-yl)méthylène]-2,6-diméthyl-Phénoxy, 4-[[3,5-bis(1,1-diméthyléthyl)-4-oxo-2,5-cyclohexadièn-1-ylidène](2,3,5,6,8,9,11,12-octahydro-1,4,7,10,13-benzopentaoxacyclopentadecin-15-yl)méthyl]-2,6-bis(1,1-diméthyléthyl)-Éthanaminium, N-[4-[(2-chlorophényl)(1-méthyl-2-phényl-1H-indol-3-yl)méthylène]-2,5-cyclohexadièn-1-ylidène]-N-éthyl-, chlorure Phénoxy, 4-[[3,5-bis(1,1-diméthyléthyl)-4-oxo-2,5-cyclohexadièn-1-ylidène]- 3-pyridinylméthyl]-2,6-bis(1,1-diméthyléthyl)-2,5-Cyclohexadièn-1-one, 4-[[3,5-bis(1,1-diméthyléthyl)-4-hydroxyphényl]-3-pyridinylméthylène]-2,6-bis(1,1-diméthyléthyl)-, ion(1-) 2,5-Cyclohexadièn-1-one, 4-[[3,5-bis(1,1-diméthyléthyl)-4-hydroxyphényl]-3-pyridinylméthylène]-2,6-bis(1,1-diméthyléthyl)-Phénoxy, 4,4'-[3,5-pyridinediylbis[[3,5-bis(1,1-diméthyléthyl)-4-oxo-2,5-cyclohexadièn-1-ylidène]méthylène]]bis[2,6-bis(1,1-diméthyléthyl)-Phénoxy, 4,4'-[2,4-pyridinediylbis[[3,5-bis(1,1-diméthyléthyl)-4-oxo-2,5-cyclohexadièn-1-ylidène]méthylène]]bis[2,6-bis(1,1-diméthyléthyl)-Phénoxy, 4,4'-[2,5-pyridinediylbis[[3,5-bis(1,1-diméthyléthyl)-4-oxo-2,5-cyclohexadièn-1-ylidène]méthylène]]bis[2,6-bis(1,1-diméthyléthyl)-Méthanaminium, N-[4-[9H-carbazol-3-yl[4-(diméthylamino)phényl]méthylène]-2,5-cyclohexadièn-1-ylidène]-N-méthyl-, chlorure [4-[α-Carbazol-3-yl-p-(diméthylamino)benzylidène]-2,5-cyclohexadièn-1-ylidène]diméthylammonium chlorure Benzènamine, 4-éthoxy-N-[4-[[4-[(4-éthoxyphényl)amino]phényl](1-méthyl-2-phényl-1H-indol-3-yl)méthylène]-2,5-cyclohexadièn-1-ylidène]-, acétate Benzènamine, 4-éthoxy-N-[4-[[4-[(4-éthoxyphényl)amino]phényl](1-méthyl-2-phényl-1H-indol-3-yl)méthylène]-2,5-cyclohexadièn-1-ylidène]-(2,5-Cyclohexadièn-1-one, 4,4'-[3,5-pyridinediylbis[[3,5-bis(1,1-diméthyléthyl)-4-hydroxyphényl]méthylidyne]]bis[2,6-bis(1,1-diméthyléthyl)-, ion(2-)-2,5-Cyclohexadièn-1-one, 4,4'-[2,6-pyridinediylbis[[3,5-bis(1,1-diméthyléthyl)-4-hydroxyphényl]méthylidyne]]bis[2,6-bis(1,1-diméthyléthyl)-, ion(2-)-2,5-Cyclohexadièn-1-one, 4,4'-[2,5-pyridinediylbis[[3,5-bis(1,1-diméthyléthyl)-4-hydroxyphényl]méthylidyne]]bis [2,6-bis(1,1-diméthyléthyl)-, ion(2-)-2,5-Cyclohexadièn-1-one, 4,4'-[2,4-pyridinediylbis[[3,5-bis(1,1-diméthyléthyl)-4-hydroxyphényl]méthylidyne]]bis [2,6-bis(1,1-diméthyléthyl)-, ion(2-)-2,5-Cyclohexadièn-1-one, 4,4'-[3,5-pyridinediylbis[[3,5-bis(1,1-diméthyléthyl)-4-hydroxyphényl]méthylidyne]]bis [2,6-bis(1,1-diméthyléthyl)-2,5-Cyclohexadièn-1-one, 4,4'-[2,5-pyridinediylbis[[3,5-bis(1,1-diméthyléthyl)-4-hydroxyphényl]méthylidyne]]bis[2,6-bis(1,1-diméthyléthyl)-2,5-Cyclohexadièn-1-one, 4,4'-[2,4-pyridinediylbis[[3,5-bis(1,1-diméthyléthyl)-4-hydroxyphényl]méthylidyne]]bis[2,6-bis(1,1-diméthyléthyl)-Cyclohexanaminium, N-méthyl-N-[4-[(2-méthyl-1H-indol-3-yl)phénylméthylène]-2,5-cyclohexadièn-1-ylidène]-, trichlorozincate(1-) Zincate(1-), trichloro-, N-méthyl-N-[4-[(2-méthyl-1H-indol-3-yl)phénylméthylène]-2,5-cyclohexadièn-1-ylidène]cyclohexanaminium Cyclohexanaminium, N-méthyl-N-[4-[(2-méthyl-1H-indol-3-yl)phénylméthylène]-2,5-cyclohexadièn-1-ylidène]-Cyclohexanaminium, N-[4-[(2-chlorophényl)(1-méthyl-2-phényl-1H-indol-3-yl)méthylène]-2,5-cyclohexadièn-1-ylidène]-N-méthyl-, trichlorozincate(1-) Zincate(1-), trichloro-, N-[4-[(2-chlorophényl)(1-méthyl-2-phényl-1H-indol-3-yl)méthylène]-2,5-cyclohexadièn-1-ylidène]-N-méthylcyclohexanaminium Cyclohexanaminium, N-[4-[(2-chlorophényl)(1-méthyl-2-phényl-1H-indol-3-yl)méthylène]-2,5-cyclohexadièn-1-ylidène]-N-méthyl-Cyclohexanaminium, N-[4-[(2-chlorophényl)[2-(4-chlorophényl)-1-méthyl-1H-indol-3-yl]méthylène]-2,5-cyclohexadièn-1-ylidène]-N-méthyl-,trichlorozincate(1-) Trichlorozincate de-, N-[4-[(2-chlorophényl)[2-(4-chlorophényl)-1-méthyl-1H-indol-3-yl]méthylène]-2,5-cyclohexadièn-1-ylidène]-N-méthylcyclohexanaminium Cyclohexanaminium, N-[4-[(2-chlorophényl)[2-(4-chlorophényl)-1-méthyl-1H-indol-3-yl]méthylène]-2,5-cyclohexadièn-1-ylidène]-N-méthyl-Benzèneméthanaminium, N-cyclohexyl-N-[4-[[1-(2-éthoxyéthyl)-2-phényl-1H-indol-3-yl](4-méthylphényl)méthylène]-2,5-cyclohexadièn-1-ylidène]-, trichlorozincate(1-) TrichloroZincate de N-cyclohexyl-N-[4-[[1-(2-éthoxyéthyl)-2-phényl-1H-indol-3-yl] (4-méthylphényl)méthylène]-2,5-cyclohexadièn-1-ylidène]benzèneméthanaminium Benzèneméthanaminium, N-cyclohexyl-N-[4-[[1-(2-éthoxyéthyl)-2-phényl-1H-indol-3-yl](4-méthylphényl)méthylène]-2,5-cyclohexadièn-1-ylidène]-Cyclohexanaminium, N-méthyl-N-[4-[(1-méthyl-2-phényl-1H-indol-3-yl)phénylméthylène]-2,5-cyclohexadièn-1-ylidène]-, trichlorozincate(1-) TrichloroZincate de N-méthyl-N-[4-[(1-méthyl-2-phényl-1H-indol-3-yl)phénylméthylène]-2,5-cyclohexadièn-1-ylidène]cyclohexanaminium Cyclohexanaminium, N-méthyl-N-[4-[(1-méthyl-2-phényl-1H-indol-3-yl)phénylméthylène]-2,5-cyclohexadièn-1-ylidène]-Cyclohexanaminium, N-[4-[(2-bromophényl)(1,2-diméthyl-1H-indol-3-yl)méthylène]-2,5-cyclohexadièn-1-ylidène]-N-éthyl-, trichlorozincate(1-) TrichloroZincate de N-[4-[(2-bromophényl)(1,2-diméthyl-1H-indol-3-yl)méthylène]-2,5-cyclohexadièn-1-ylidène]-N-éthylcyclohexanaminium Cyclohexanaminium, N-[4-[(2-bromophényl)(1,2-diméthyl-1H-indol-3-yl)méthylène]-2,5-cyclohexadièn-1-ylidène]-N-éthyl-Éthanaminium, N-[3-chloro-4-[(1-méthyl-2-phényl-1H-indol-3-yl)phénylméthylène]-2,5-cyclohexadièn-1-ylidène]-N-éthyl-, sulfate (1:1) Éthanaminium, N-[3-chloro-4-[(1-méthyl-2-phényl-1H-indol-3-yl)phénylméthylène]-2,5-cyclohexadièn-1-ylidène]-N-éthyl-Éthanaminium, N-éthyl-N-[4-[(2-méthyl-1H-indol-3-yl)(2-nitrophényl)méthylène]-2,5-cyclohexadièn-1-ylidène]-, sulfate (1:1) Éthanaminium, N-éthyl-N-[4-[(2-méthyl-1H-indol-3-yl)(2-nitrophényl)méthylène]-2,5-cyclohexadièn-1-ylidène]-Méthanamine, N-[4-[(1-méthyl-2-phényl-1H-indol-3-yl)phénylméthylène]-2,5-cyclohexadièn-1-ylidène]-, sulfate (1:1) Méthanamine, N-[4-[(1-méthyl-2-phényl-1H-indol-3-yl)phénylméthylène]-2,5-cyclohexadièn-1-ylidène]-Méthanaminium, N-[4-[[4-(diméthylamino)phényl]-2-furanylméthylène]-2,5-cyclohexadièn-1-ylidène]-N-méthyl-, (T-4)-tétrabromothallate(1-) Thallate(1-), tétrabromo-, (T-4)-, N-[4-[[4-(diméthylamino)phényl]-2-furanylméthylène]-2,5-cyclohexadièn-1-ylidène]-N-méthylméthanaminium Phénoxy, 4-[[3,5-bis(1,1-diméthyléthyl)-4-oxo-2,5-cyclohexadièn-1-ylidène][6-[[3,5-bis(1,1-diméthyléthyl)-4-oxo-2,5-cyclohexadièn-1-ylidène][3,5-bis(1,1-diméthyléthyl)-4-oxyphényl]méthyl]-2-pyridinyl]méthyl]-2,6-bis(1,1-diméthyléthyl)-2,5-Cyclohexadièn-1-one, 4,4'-[2,6-pyridinediylbis[[3,5-bis(1,1-diméthyléthyl)-4-hydroxyphényl]méthylidyne]]bis[2,6-bis(1,1-diméthyléthyl)-Méthanaminium, N-[4-[(2,3-dihydro-1,5-diméthyl-3-oxo-2-phényl-1H-pyrazol-4-yl) (3-nitrophényl)méthylène]-2,5-cyclohexadièn-1-ylidène]-N-méthyl-,chlorure Méthanaminium, N-[4-[(3-bromophényl)(2,3-dihydro-1,5-diméthyl-3-oxo-2-phényl-1H-pyrazol-4-yl)méthylène]-2,5-cyclohexadièn-1-ylidène]-N-méthyl-,chlorure Méthanaminium, N-[4-[(2,3-dihydro-1,5-diméthyl-3-oxo-2-phényl-1H-pyrazol-4-yl)(3-méthoxyphényl)méthylène]-2,5-cyclohexadièn-1-ylidène]-N-méthyl-,chlorure Méthanaminium, N-[4-[(2,3-dihydro-1,5-diméthyl-3-oxo-2-phényl-1H-pyrazol-4-yl)phénylméthylène]-2,5-cyclohexadièn-1-ylidène]-N-méthyl-, chlorure [[4-[(1-méthyl-2-phényl-1H-indol-3-yl)[4-[(sulfophényl)amino]phényl]méthylène] -2,5-cyclohexadièn-1-ylidène]amino]-Méthanaminium, N-[4-[(2,3-dihydro-1,5-diméthyl-3-oxo-2-phényl-1H-pyrazol-4-yl)[4- (diméthylamino)phényl]méthylène] -2,5-cyclohexadièn-1-ylidène]-N-méthyl-, chlorure Benzènamine, 4-[(2-[1,1'-biphényl]-4-yl-1-butyl-1H-indol-3-yl)[4-[(4-éthoxyphényl)imino]-2,5-cyclohexadièn-1-ylidèneméthyl]-N-(4-éthoxyphényl)-Benzènaminium, N-méthyl-N-[4-[[4-(méthylphénylamino)phényl](4-oxo-4H-1-benzopyran-3-yl)méthylène]-2,5-cyclohexadièn-1-ylidène]-Éthanaminium, N-[4-[[4-(diéthylamino)phényl](4-oxo-4H-1-benzopyran-3-yl)méthylène]-2,5-cyclohexadièn-1-ylidène]-N-éthyl-Benzènaminium, 4-éthoxy-N-méthyl-N-[4-[(1-méthyl-2-phényl-1H-indol-3-yl)phénylméthylène]-2,5-cyclohexadièn-1-ylidène]-, chlorure Méthanaminium, N-[4-[12H-benzo[a]phénoxazin-5-yl[4-(diméthylamino)phényl]méthylène]-2,5-cyclohexadièn-1-ylidène]-N-méthyl-, chlorure Benzènaminium, N-méthyl-N-[4-[(1-méthyl-2-phényl-1H-indol-3-yl)phénylméthylène]-2,5-cyclohexadièn-1-ylidène]-Éthanaminium, N-[4-[[4-(diéthylamino)phényl](7-méthoxy-2,1,3-benzothiadiazol-4-yl)méthylène]-2,5-cyclohexadièn-1-ylidène]-N-éthyl-Éthanaminium, N-[4-[[4-(diéthylamino)phényl](5-méthoxy-2,1,3-benzothiadiazol-4-yl)méthylène]-2,5-cyclohexadièn-1-ylidène]-N-éthyl-Éthanaminium, N-[4-[[4-(diéthylamino)phényl](7-méthyl-2,1,3-benzothiadiazol-4-yl)méthylène]-2,5-cyclohexadièn-1-ylidène]-N-éthyl-Éthanaminium, N-[4-[2,1,3-benzothiadiazol-5-yl[4-(diéthylamino)phényl]méthylène]-2,5-cyclohexadièn-1-ylidène]-N-éthyl-Éthanaminium, N-[4-[2,1,3-benzothiadiazol-4-yl[4-(diéthylamino)phényl]méthylène]-2,5-cyclohexadièn-1-ylidène]-N-éthyl-Méthanaminium, N-méthyl-N-[4-[(1-méthyl-2-phényl-1H-indol-3-yl)phénylméthylène]-2,5-cyclohexadièn-1-ylidène]-, chlorure Méthanaminium, N-méthyl-N-[4-[(1-méthyl-2-phényl-1H-indol-3-yl)phénylméthylène]-2,5-cyclohexadièn-1-ylidène]-Méthanaminium, N-[4-[[4-(diméthylamino)phényl](7-méthoxy-2,1,3-benzothiadiazol-4-yl)méthylène]-2,5-cyclohexadièn-1-ylidène]-N-méthyl-Méthanaminium, N-[4-[[4-(diméthylamino)phényl](5-méthoxy-2,1,3-benzothiadiazol-4-yl)méthylène]-2,5-cyclohexadièn-1-ylidène]-N-méthyl-Méthanaminium, N-[4-[[4-(diméthylamino)phényl](7-méthyl-2,1,3-benzothiadiazol-4-yl)méthylène]-2,5-cyclohexadièn-1-ylidène]-N-méthyl-Méthanaminium, N-[4-[2,1,3-benzothiadiazol-5-yl[4-(diméthylamino)phényl]méthylène]-2,5-cyclohexadièn-1-ylidène]-N-méthyl-Méthanaminium, N-[4-[2,1,3-benzothiadiazol-4-yl[4-(diméthylamino)phényl]méthylène]-2,5-cyclohexadièn-1-ylidène]-N-méthyl-Méthanaminium, N-[4-[[4-(diméthylamino)phényl]-3-pyridinylméthylène]-2,5-cyclohexadièn-1-ylidène]-N-méthyl-Méthanaminium, N-[4-[[4-(diméthylamino)phényl]-4-pyridinylméthylène]-2,5-cyclohexadièn-1-ylidène]-N-méthyl-Methanaminium, N-[4-[[4- (diméthylamino)phényl]-2-thiènylméthylène]-2,5-cyclohexadièn-1-ylidène]-N-méthyl-Méthanaminium, N-[4-[[4-(diméthylamino)phényl]-2-furanylméthylène]-2, 5-cyclohexadièn-1-ylidène]-N-méthyl-Méthanaminium, N-[4-[[4-(diméthylamino)phényl]-3-thiènylméthylène]-2,5-cyclohexadièn-1-ylidène]-N-méthyl-[[4-[(1-méthyl-2-phényl-1H-indol-3-yl)[4-[(sulfophényl)amino]phényl]méthylène]-2,5-cyclohexadièn-1-ylidène]amino]-, Ethoxy[[4-[[4-[(éthoxysulfophényl)amino]phényl](1-méthyl-2-phényl-1H-indol-3-yl)méthylène]-2,5-cyclohexadièn-1-ylidène]amino]-Benzènamine, N-méthyl-4-[[4-(méthylimino)-2,5-cyclohexadièn-1-ylidène](2-méthyl-1H-indol-3-yl)méthyl]-, Méthanaminium, N-[4-[[4-(diméthylamino)phényl](2-méthyl-1H-indol-3-yl)méthylène]-2,5-cyclohexadièn-1-ylidène]-N-méthyl-Benzènamine, N-méthyl-4-[[4-(méthylimino)-2,5-cyclohexadièn-1-ylidène](1-méthyl-2-phényl-1H-indol-3-yl)méthyl]-, monoacide conjugué Méthanaminium, N-[4-[[4-(diméthylamino)phényl](1-méthyl-2-phényl-1H-indol-3-yl)méthylène]-2,5-cyclohexadièn-1-ylidène]-N-méthyl-Benzènamine, 4-éthoxy-N-[4-[[4-[(4-éthoxyphényl)amino]phényl](2-phényl-1H-indol-3-yl)méthylène]-2,5-cyclohexadièn-1-ylidène]-, Benzènamine, 4-éthoxy-N-[4-[[4-[(4-éthoxyphényl)amino]phényl](1-méthyl-1H-indol-3-yl)méthylène]-2,5-cyclohexadièn-1-ylidène]-, Benzènamine, N-[4-[(1,2-diméthyl-1H-indol-3-yl)[4-[(4-éthoxyphényl)amino]phényl]méthylène]-2,5-cyclohexadièn-1-ylidène]-4-éthoxy-Benzènamine, 4-[(1-méthyl-2-phényl-1H-indol-3-yl)[4- (phénylimino)-2,5-cyclohexadièn-1-ylidène]méthyl]-N-phényl-, monoacide conjugué Benzènamine, 4-éthoxy-N-[4-[[4-[(4-éthoxyphényl)amino]phényl](1-méthyl-2-phényl-1H-indol-3-yl)méthylène]-2,5-cyclohexadièn-1-ylidène]-, Méthanaminium, N-[4-[[4-(diméthylamino)phényl](2-phényl-1H-indol-3-yl)méthylène]-2,5-cyclohexadièn-1-ylidène]-N-méthyl-Benzènaminium, N-méthyl-N-[4-[[4-(méthylphénylamino)phényl](4-oxo-4H-1-benzopyran-3-yl)méthylène]-2,5-cyclohexadièn-1-ylidène]-, chlorure Benzènaminium, N-méthyl-N-[4-[[4-(méthylphénylamino)phényl](4-oxo-4H-1-benzopyran-3-yl)méthylène]-2,5-cyclohexadièn-1-ylidène]-, perchlorate Éthanaminium, N-[4-[[4-(diéthylamino)phényl](4-oxo-4H-1-benzopyran-3-yl)méthylène]-2,5-cyclohexadièn-1-ylidène]-N-éthyl-, perchlorate Éthanaminium, N-[4-[[4-(diéthylamino)phényl](4-oxo-4H-1-benzopyran-3-yl)méthylène]-2,5-cyclohexadièn-1-ylidène]-N-éthyl-, chlorure Benzènaminium, N-méthyl-N-[4-[[4- (méthylphénylamino)phényl](4-oxo-4H-1-benzopyran-3-yl)méthylène]-2,5-cyclohexadièn-1-ylidène]-, acétate Éthanaminium, N-[4-[[4-(diéthylamino)phényl](4-oxo-4H-1-benzopyran-3-yl)méthylène]-2,5-cyclohexadièn-1-ylidène]-N-éthyl-, acétate Méthanaminium, N-[4-[(2,3-dihydro-1,5-diméthyl-3-oxo-2-phényl-1H-pyrazol-4-yl)(3-nitrophényl)méthylène]-2,5-cyclohexadièn-1-ylidène]-N-méthyl-Méthanaminium, N-[4-[(3-bromophényl)(2,3-dihydro-1,5-diméthyl-3-oxo-2-phényl-1H-pyrazol-4-yl)méthylène]-2,5-cyclohexadièn-1-ylidène]-N-méthyl-Méthanaminium, N-[4-[(2,3-dihydro-1,5-diméthyl-3-oxo-2-phényl-1H-pyrazol-4-yl) (3-méthoxyphényl)méthylène]-2,5-cyclohexadièn-1-ylidène]-N-méthyl-Méthanaminium, N-[4-[(2,3-dihydro-1,5-diméthyl-3-oxo-2-phényl-1H-pyrazol-4-yl)phénylméthylène]-2,5-cyclohexadièn-1-ylidène]-N-méthyl-Méthanaminium, N-[4-[(2,3-dihydro-1,5-diméthyl-3-oxo-2-phényl-1H-pyrazol-4-yl) (3-méthylphényl)méthylène]-2,5-cyclohexadièn-1-ylidène]-N-méthyl-Benzènamine, N-[4-[(1-éthyl-2-phényl-1H-indol-3-yl)[4-[(2-méthoxyphényl)amino]phényl]méthylène]-2,5-cyclohexadièn-1-ylidène]-2-méthoxy-, monochlorhydrate 2-hydroxy-5-[[4-[[4-[(4-hydroxy-3-sulfophényl)amino]phényl](1-méthyl-2-phényl-1H-indol-3-yl)méthylène]-2,5-cyclohexadièn-1-ylidène]amino]-Benzènamine, 2-éthoxy-N-[4- [[4-[(2-éthoxyphényl)amino]phényl](1-méthyl-2-phényl-1H-indol-3-yl)méthylène]-2,5-cyclohexadièn-1-ylidène]-, monochlorhydrate Méthanaminium, N-[4-[[4-(diméthylamino)phényl]-2-furanylméthylène]-2,5-cyclohexadièn-1-ylidène]-N-méthyl-, perchlorate 2-éthoxy-5-[[4-[[4-[(4-éthoxy-3-sulfophényl)amino]phényl](1-méthyl-2-phényl-1H-indol-3-yl)méthyl]-2,5-cyclohexadièn-1-ylidène]amino]-Ammonium, [4-[α-[p-(diméthylamino)phényl]furfurylidène]-2,5-cyclohexadièn-1-ylidène]diméthyl-, tétrachlorogallate(1-) Méthanaminium, N-[4-[[4-(diméthylamino)phényl]-2-thiènylméthyl]-2,5-cyclohexadièn-1-ylidène]-N-méthyl-, perchlorate Ammonium, [4-[p-(diméthylamino)-α-2-thiènylbenzylidène]-2,5-cyclohexadièn-1-ylidène]diméthyl-, perchlorate Ammonium, (2-cyanoéthyl) [4-[α-[4-[(2-cyanoéthyl)éthylamino]-o-tolyl]pipéronylidène]-3-méthyl-2,5-cyclohexadièn-1-ylidène]éthyl-Ammonium, diméthyl[4-[α-(1-méthyl-2-phénylindol-3-yl)benzylidène]-2,5-cyclohexadièn-1-ylidène]-, chlorure, Ammonium, (p-éthoxyphényl)méthyl[4-[α-(1-méthyl-2-phénylindol-3-yl)benzylidène]-2,5-cyclohexadièn-1-ylidène]-, chlorure, (p-éthoxyphényl)méthyl[4-[α-(1-méthyl-2-phénylindol-3-yl)benzylidène]-2,5-cyclohexadièn-1-ylidène]ammonium chlorure Ammonium, [4-[α-2,1,3-benzothiadiazol-4-yl-p-(diméthylamino)benzylidène]-2,5-cyclohexadièn-1-ylidène]diméthyl-,perchlorate Ammonium, [4-[p-(diméthylamino)-α-(5-méthoxy-2,1,3-benzothiadiazol-4-yl)benzylidène]-2,5-cyclohexadièn-1-ylidène]diméthyl-, perchlorate Ammonium, [4-[p-(diéthylamino)-α-(5-méthoxy-2,1,3-benzothiadiazol-4-yl)benzylidène]-2,5-cyclohexadièn-1-ylidène]diéthyl-, perchlorate Ammonium, [4-[p-(diéthylamino)-α-(7-méthoxy-2,1,3-benzothiadiazol-4-yl)benzylidène]-2,5-cyclohexadièn-1-ylidène]diéthyl-, perchlorate Ammonium, [4-[p-(diméthylamino)-α-(7-méthoxy-2,1,3-benzothiadiazol-4-yl)benzylidène]-2,5-cyclohexadièn-1-ylidène]diméthyl-, perchlorate Ammonium, [4-[p-(diéthylamino)-α-(7-méthyl-2,1,3-benzothiadiazol-4-yl)benzylidène]-2,5-cyclohexadièn-1-ylidène]diéthyl-, perchlorate Ammonium, [4-[p-(diméthylamino)-α-(7-méthyl-2,1,3-benzothiadiazol-4-yl)benzylidène]-2,5-cyclohexadièn-1-ylidène]diméthyl-, perchlorate Ammonium, [4-[α-2,1,3-benzothiadiazol-4-yl-p-(diéthylamino)benzylidène]-2,5-cyclohexadièn-1-ylidène]diéthyl-,perchlorate Ammonium, [4-[α-2,1,3-benzothiadiazol-5-yl-p-(diéthylamino)benzylidène]-2,5-cyclohexadièn-1-ylidène]diéthyl-,perchlorate Ammonium, [4-[α-2,1,3-benzothiadiazol-5-yl-p-(diméthylamino)benzylidène]-2,5-cyclohexadièn-1-ylidène]diméthyl-,perchlorate Ammonium, méthyl[4-[α-(1-méthyl-2-phénylindol-3-yl)benzylidène]-2,5-cyclohexadièn-1-ylidène]phényl-, trichlorozincate(1-) Trichlorozincate de-, méthyl[4-[α-(1-méthyl-2-phénylindol-3-yl)benzylidène]-2,5-cyclohexadièn-1-ylidènelphénylammonium Trichlorozincate de diméthyl[4-[α-(1-méthyl-2-phénylindol-3-yl)benzylidène]-2,5-cyclohexadièn-1-ylidène] ammonium, Trichloro zincate de diméthyl[4-[α-(1-méthyl-2-phénylindol-3-yl)benzylidène]-2,5-cyclohexadièn-1-ylidène]ammonium Indole, 3-[α-(4-imino-2,5-cyclohexadièn-1-ylidène)benzyl]-1,2-diméthyl-, monochlorhydrate Indole, 3-[α-[4-[(p-éthoxyphényl)imino]-2,5-cyclohexadièn-1-ylidène]benzyl]-1-méthyl-2-phényl-, monochlorhydrate Ammonium, [4-[α-[9-(2-cyanoéthyl)carbazol-3-yl]-p-(diméthylamino)benzylidène]-2,5-cyclohexadièn-1-ylidène]diméthylAmmonium, [4-[α-carbazol-3-yl-p-(diméthylamino)benzylidène]-2,5-cyclohexadièn-1-ylidène]diméthylAmmonium, [4-[α-[9-(2-cyanoéthyl)carbazol-3-yl]benzylidène]-2,5-cyclohexadièn-1-ylidène]diméthylAmmonium, [4-(α-carbazol-3-ylbenzylidène)-2,5-cyclohexadièn-1-ylidène]diméthyl-2,5-Cyclohexadièn-1-one, 2,6-di-tert-butyl-4-(α-morpholinobenzylidène)-2,5-Cyclohexadièn-1-one, 2,6-di-tert-butyl-4-[α-(2,2-diméthyl-1-aziridinyl)benzylidène]-2,5-Cyclohexadièn-1-one, 4-(α-1-aziridinylbenzylidène)-2,6-di-tert-butyl-2,5-Cyclohexadièn-1-one, 2,6-bis(1,1-diméthyléthyl)-4-(phényl-1-pipéridinylméthylène)-2,5-Cyclohexadièn-1-one, 2, 6-di-tert-butyl-4- (α-pipéridinobenzylidène)-Ammonium, [4-[α-[4-(diéthylamino)-o-tolyl]pipéronyl]-3-méthyl-2,5-cyclohexadièn-1-ylidène]diéthyl-, chlorure Ammonium, [4-[α-[4-(diéthylamino)-o-tolyl]-2-thénylidène]-3-méthyl-2,5-cyclohexadièn-1-ylidène]diéthyl-, chlorure Ammonium, [4-[α-[p-(diméthylamino)phényl]-5-nitrofurfurylidène]-2,5-cyclohexadièn-1-ylidène]diméthyl-, chlorure Ammonium, [4-[5-bromo-α-[p-(diéthylmino)phényl]furfurylidène]-2,5-cyclohexadièn-1-ylidène]diéthyl-, chlorure Ammonium, [4-[5-bromo-α-[p-(diméthylmino)phényl]furfurylidène]-2,5-cyclohexadièn-1-ylidène]diméthyl-, chlorure Ammonium, [4-[α-[p-(diméthylamino)phényl]-2-furfurylidène]-2,5-cyclohexadièn-1-ylidène]diéthyl-, chlorure 2-hydroxy-5-[[4-[[4-(4-hydroxy-3-sulfophényl)amino]phényl](1-méthyl-2-phényl-1H-indol-3-yl)méthylène]-2,5-cyclohexadièn-1-ylidène]amino]-, Ammonium, [4-[α-12H-benzo[a]phénoxazin-5-yl-p-(diméthylamino)benzylidène]-2,5-cyclohexadièn-1-ylidène]diméthyl-,chlorure, composé avec le chlorure de zinc [4-[α-12H-benzo[a]phénoxazin-5-yl-p-(diméthylamino)benzylidène]-2,5-cyclohexadièn-1-ylidène]diméthylammonium chlorure Benzènamine, N,2-diméthyl-4-[(2-méthyl-1H-indol-3-yl)[3-méthyl-4-(méthylimino)-2,5-cyclohexadièn-1-ylidène]méthyl]-, monochlorhydrate Indole, 2-méthyl-3-[3-méthyl-4-méthylamino)-α-[3-méthyl-4-(méthylimino)-2,5-cyclohexadièn-1-ylidène]benzyl]-, monochlorhydrate Méthanaminium, N-[4-[[4-(diméthylamino)phényl]-2-furanylméthylène]-2,5-cyclohexadièn-1-ylidène]-N-méthyl-, chlorure Ammonium, [4-[α-[p-(diméthylamino)phényl]furfurylidène]-2,5-cyclohexadièn-1-ylidène]diméthyl-, chlorure Benzènaminium, N-[4-[[2-(4-chlorophényl)-4,6-diméthyl-1-(2-méthylpropyl)-1H-indol-3-yl](2,4-disulfophényl)méthylène]-2,5-cyclohexadièn-1-ylidène]-4-méthoxy-N-méthyl-, Ammonium, [4-[α-[2-(p-chlorophényl)-1-isobutyl-4,6-diméthylindol-3-yl]-2,4-disulfobenzylidène]-2,5-cyclohexadièn-1-ylidène](p-méthoxyphényl)méthyl-, hydroxyde, 2-éthoxy-5-[[4-[[[4-[(4-éthoxy-3-sulfophényl)amino]phényl](1-méthyl-2-phényl-1H-indol-3-yl)méthylène]-2,5-cyclohexadién-1-ylidène]amino]-, 5-[(3-carboxy-5-méthyl-4-oxo-2,5-cyclohexadièn-1-ylidène)(4,5-dihydro-3-méthyl-5-oxo-1-phényl-1H-pyrazol-4-yl)méthyl]-2-hydroxy-3-méthyl-5-[[4-[[2-(4-chlorophényl)-4,6-diméthyl-1-(2-méthylpropyl)-1H-indol-3-yl][4-[(4-éthoxy-3-sulfophényl)amino]phényl]méthylène]-2,5-cyclohexadièn-1-ylidène]amino]-2-éthoxy-, N-[α-[2-(p-chlorophényl)-1-isobutyl-4,6-diméthylindol-3-yl]-α-[4-[(4-éthoxy-3-sulfophényl)imino]-2,5-cyclohexadièn-1-ylidène]-p-tolyl]-6-éthoxy-, 6-[(4-éthoxyphényl)imino]-3-[(1-méthyl-2-phényl-1H-indol-3-yl)[4-[(2-sulfoéthyl)amino]phényl]méthylène]-, 6-[(p-éthoxyphényl)imino]-3-[α-(1-méthyl-2-phénylindol-3-yl)-p-[(2-sulfoéthyl)amino]benzylidène]-, Méthanaminium, N-[4-[[4-(diméthylamino)phényl]-3-thiènylméthylène]-2,5-cyclohexadièn-1-ylidène]-N-méthyl-, perchlorate [4-[α-[p-(Diméthylamino)phényl]-3-thénylidène]-2,5-cyclohexadièn-1-ylidène]diméthylammonium perchlorate Ammonium, 4-[α-[p-(diméthylamino)phényl]furfurylidène]-2,5-cyclohexadièn-1-ylidène]diméthyl-, bromure [4-[α-[p-(Diméthylamino)phényl]furfurylidène]-2,5-cyclohexadièn-1-ylidène]diméthylammonium bromure Méthanaminium, N-[4-[[4-(diméthylamino)phényl]-3-pyridinylméthylène]-2,5-cyclohexadièn-1-ylidène]-N-méthyl-, perchlorate Ammonium, [4-[p-(diméthylamino)-α-3-pyridylbenzylidène]-2,5-cyclohexadièn-1-ylidène]diméthyl-, perchlorate [4-[p-(Diméthylamino)-α-3-pyridylbenzylidène]-2,5-cyclohexadièn-1-ylidène]diméthylammonium perchlorate Ammonium, [4-[p-(diméthylamino)-α-4-pyridylbenzylidène]-2,5-cyclohexadièn-1-ylidène]diméthyl-, perchlorate [4-[p-(Diméthylamino)-α-4-pyridylbenzylidène]-2,5-cyclohexadièn-1-ylidène]diméthylammonium perchlorate Ammonium, [4-[p-(diméthylamino)-α-2-pyridylbenzylidène]-2,5-cyclohexadièn-1-ylidène]diméthyl-, chlorure [4-[p-(Diméthylamino)-α-2-pyridylbenzylidène]-2,5-cyclohexadièn-1-ylidène]diméthylammonium chlorure,
et leurs sels d'addition.

Les monohétéroyldiarylméthanes de formule (I) ou (Ibis) et les composés leuco de formule (II) ou (IIbis) sont des composés connus. Ces composés peuvent être préparés selon des procédés de synthèses bien connus dans la littérature et tels que décrits, par exemple, dans les documents DE-A-1 509 750, GB-A-1 139 407, GB-A-1 188 605, US-A-3 652 556 et US-A-3 685 956, GB-822 846, DE-1 254 118, GB-1 047 796, US-3 423 427, DE-1 569 749, BE-702 839, BE-A-702 240, US-3 995 088, US-A-4 054 718, DE-2 917 271, US-4 340 540, GB-A-2 075 539, US-A-4 460 385, US-A-5 094 688 et US-A-5 097 034.

Il s'avère que les compositions de l'invention permettent, de manière surprenante, d'obtenir des colorations intenses, même sur des cheveux non sensibilisés.

Les compositions selon l'invention permettent d'obtenir des reflets variés chromatiques ou sombres, très puissants, peu sélectifs et tenaces.

Ainsi, les colorants (I), (Ibis), (II) et (IIbis) inclus dans les compositions de l'invention permettent d'obtenir toutes les nuances du vert au bleu, en passant par les rouges et, en particulier, ils permettent d'obtenir aussi les nuances noires.

Les colorations obtenues avec les compositions de l'invention sont tenaces, stables, résistantes, vis-à-vis des intempéries, du lavage, de la transpiration, des frottements et des traitements ultérieurs, tels que l'ondulation permanente.

Ces colorations sont, en particulier, particulièrement tenaces à la lumière.

Le ou les composés de formules (I), (Ibis), (II), (IIbis) ci-dessus et/ou leur ou leurs sel(s) d'addition représentent généralement de 0,0001 à 10 % en poids du poids total de la composition de teinture, de préférence de 0,005 à 10 % en poids et, de préférence encore, de 0,01 à 6 % de poids du poids total de la composition de teinture selon l'invention.

La composition tinctoriale conforme à l'invention peut en outre contenir un ou plusieurs colorant(s) direct(s) différent(s) des composés de formule (I), (Ibis), (II) ou (IIbis). Le ou ces colorant(s) direct (s) utile (s) selon l'invention sont par exemple choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants :
- 1,4-diamino-2-nitrobenzène ;
- 1-amino-2 nitro-4-β- hydroxyéthylaminobenzène ;
- 1-amino-2 nitro-4-bis(β-hydroxyéthyl)-aminobenzène ;
- 1,4-Bis(β-hydroxyéthylamino)-2-nitrobenzène ;
- 1-β-hydroxyéthylamino-2-nitro-4-bis-(β-hydroxyéthylamino)-benzène ;
- 1-β-hydroxyéthylamino-2-nitro-4-aminobenzène ;
- 1-β-hydroxyéthylamino-2-nitro-4-(éthyl)(β-hydroxyéthyl) -aminobenzène ;
- 1-amino-3-méthyl-4-β-hydroxyéthylamino-6-nitrobenzène ;
- 1-amino-2-nitro-4-β-hydroxyéthylamino-5-chlorobenzène ;
- 1,2-Diamino-4-nitrobenzène ;
- 1-amino-2-β-hydroxyéthylamino-5-nitrobenzène ;
- 1,2-Bis-(β-hydroxyéthylamino)-4-nitrobenzène ;
- 1-amino-2-tris-(hydroxyméthyl)-méthylamino-5-nitrobenzène ;
- 1-Hydroxy-2-amino-5-nitrobenzène ;
- 1-Hydroxy-2-amino-4-nitrobenzène ;
- 1-Hydroxy-3-nitro-4-aminobenzène ;
- 1-Hydroxy-2-amino-4,6-dinitrobenzène ;
- 1-β-hydroxyéthyloxy-2-β-hydroxyéthylamino-5-nitrobenzène ;
- 1-Méthoxy-2-β-hydroxyéthylamino-5-nitrobenzène ;
- 1-β-hydroxyéthyloxy-3-méthylamino-4-nitrobenzène ;
- 1-β,γ-dihydroxypropyloxy-3-méthylamino-4-nitrobenzène ;
- 1-β-hydroxyéthylamino-4-β,γ-dihydroxypropyloxy-2-nitrobenzène ;
- 1-β,γ-dihydroxypropylamino-4-trifluorométhyl-2-nitrobenzène ;
- 1-β-hydroxyéthylamino-4-trifluorométhyl-2-nitrobenzène ;
- 1-β-hydroxyéthylamino-3-méthyl-2-nitrobenzène ;
- 1-β-aminoéthylamino-5-méthoxy-2-nitrobenzène ;
- 1-Hydroxy-2-chloro-6-éthylamino-4-nitrobenzène ;
- 1-Hydroxy-2-chloro-6-amino-4-nitrobenzène ;
- 1-Hydroxy-6-bis-(β-hydroxyéthyl)-amino-3-nitrobenzène ;
- 1-β-hydroxyéthylamino-2-nitrobenzène ;
- 1-Hydroxy-4-β-hydroxyéthylamino-3-nitrobenzène.

Parmi les colorants directs azoïques, on peut citer les colorants azoïques cationiques décrits dans les demandes de brevets WO -A-95/15144, WO-A-95/01772, EP-A-714954 et WO-A-01/66646, dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1H-Imidazolium ;
- chlorure de 1,3-diméthyl-2-[(4-aminophényl)azo]-1H-Imidazolium ;
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
- Disperse Red 17 ;
- Acid Yellow 9 ;
- Acid Black 1 ;
- Basic Red 22 ;
- Basic Red 76 ;
- Basic Yellow 57 ;
- Basic Brown 16 ;
- Acid Yellow 36 ;
- Acid Orange 7 ;
- Acid Red 33 ;
- Acid Red 35 ;
- Basic Brown 17 ;
- Acid Yellow 23 ;
- Acid Orange 24 ;
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15 ;
- Solvent Violet 13 ;
- Acid Violet 43 ;
- Disperse Violet 1 ;
- Disperse Violet 4 ;
- Disperse Blue 1 ;
- Disperse Violet 8 ;
- Disperse Blue 3 ;
- Disperse Red 11 ;
- Acid Blue 62 ;
- Disperse Blue 7 ;
- Basic Blue 22 ;
- Disperse Violet 15 ;
- Basic Blue 99,
ainsi que les composés suivants :
- 1-N-méthylmorpholiniumpropylamino-4-hydroxyanthraquinone ;
- 1-Aminopropylamino-4-méthylaminoanthraquinone ;
- 1-Aminopropylaminoanthraquinone ;
- 5-β-hydroxyéthyl-1,4-diaminoanthraquinone ;
- 2-Aminoéthylaminoanthraquinone ;
- 1,4-Bis-(β,γ-dihydroxypropylamino)-anthraquinone.

Parmi les colorants aziniques, on peut citer les composés suivants :
- Basic Blue 17 ;
- Basic Red 2.

Parmi les colorants triarylméthaniques, on peut citer les composés suivants :
- Basic Green 1 ;
- Acid blue 9 ;
- Basic Violet 3 ;
- Basic Violet 14 ;
- Basic Blue 7 ;
- Acid Violet 49 ;
- Basic Blue 26 ;
- Acid Blue 7.

Parmi les colorants indoaminiques, on peut citer les composés suivants :
- 2-β-hydroxyéthlyamino-5-[bis-(β-4'-hydroxyéthyl)amino]anilino-1,4-benzoquinone ;
- 2-β-hydroxyéthylamino-5-(2'-méthoxy-4'-amino)anilino-1,4-benzoqinone ;
- 3-N(2'-Chloro-4'-hydroxy)phényl-acétylamino-6-méthoxy-1,4-benzoquinone imine ;
- 3-N(3'-Chloro-4'-méthylamino)phényl-uréido-6-méthyl-1,4-benzoquinone imine ;
- 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs, additionnels différents des composants de formules (I), (Ibis), (II) et (IIbis) représentent de préférence de 0,001 à 20% en poids environ du poids total de la composition prête à l'emploi et encore plus préférentiellement de 0,005 à 10% en poids environ.

La composition de la présente invention peut de plus contenir une ou plusieurs base(s) d'oxydation, et éventuellement un ou plusieurs coupleur(s) classiquement utilisés pour la coloration par oxydation.

A titre d'exemple de base d'oxydation, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, le ou les base(s) d'oxydation et/ou le ou les coupleurs sont chacun généralement présents en une quantité comprise entre 0,001 et 10 % en poids environ du poids total de la composition tinctoriale, de préférence entre 0,005 et 6 %.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

De préférence, les compositions contiennent un solvant choisi parmi les alcanols en C₂-C₄ et les polyols de poids moléculaire inférieur à 1 000.

De préférence, les compositions contiennent au moins un agent tensioactif et/ou au moins un agent épaississant minéral ou organique.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, de préférence entre 5 et 11 environ, de préférence encore de 6 à 8,5.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di-et triéthanolamines ainsi que leurs dérivés, les hydroxydes de sodium ou de potassium et les composés de formule (III) suivante : 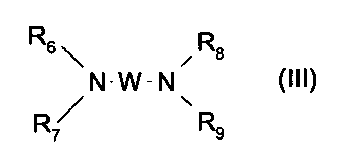 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe des fibres kératiniques et, en particulier, des fibres kératiniques humaines, telles que les cheveux, qui comprend l'application d'au moins une composition tinctoriale contenant au moins un composé, colorant, de formule (I), (Ibis), (II) ou (IIbis) telles que définies précédemment sur les fibres kératiniques. Après un temps de pause, les fibres kératiniques sont rincées laissant apparaître des fibres colorées. Le temps de pause est généralement compris entre 3 à 50 minutes environ, de préférence 5 à 30 minutes environ.

Par teinture ou coloration directe, on entend une teinture ou coloration effectuée sans agent oxydant autre que l'oxygène de l'air.

Lorsque la composition tinctoriale comprend soit au moins un composé de formule (II) ou (IIbis), soit au moins un composé de formule (I) ou (Ibis) et au moins une base d'oxydation, et éventuellement un ou plusieurs coupleurs, la composition tinctoriale contient de préférence un oxydant.

L'invention a donc également pour objet un procédé de teinture des fibres kératiniques et, en particulier, des fibres kératiniques humaines, telles que les cheveux, dans lequel on applique sur lesdites fibres au moins une composition de teinture comprenant soit au moins un composé de formule (II) ou (IIbis), soit au moins un composé de formule (I) ou (Ibis) et au moins une base d'oxydation et éventuellement un ou plusieurs coupleurs, la couleur étant révélée à pH acide, neutre ou alcalin à l'aide d'un agent oxydant.

Les agents oxydants utilisables sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment.

En cas de mélange avec la composition tinctoriale, le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11, mieux entre 6 et 8,5. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou « kit » de teinture, dans lequel un premier compartiment renferme une composition tinctoriale définie ci-dessus, comprenant soit au moins un composé de formule (II) ou (IIbis), soit au moins un composé de formule (I) ou (Ibis) et au moins une base d'oxydation et éventuellement un ou plusieurs coupleurs, et un deuxième compartiment renferme une composition oxydante.

Ce dispositif peut être équipé d'un moyen permettant de délivrer sur les cheveux le mélange souhaité, tel que les dispositifs décrits dans le brevet FR-2 586 913 au nom de la demanderesse.

Dans le cas d'une composition contenant au moins un composé de formule (II) ou (IIbis), il est souhaitable d'éviter le contact entre lesdits composés et l'oxygène de l'air. Un mode de conditionnement adapté peut être alors un dispositif aérosol.

Avec les colorants de l'invention, on peut également réaliser de la coloration directe éclaircissante en l'absence de colorant d'oxydation et en présence d'un agent oxydant dans des conditions telles (par exemple peroxyde d'hydrogène en milieu alcalin) que celui-ci soit apte à éclaircir les fibres kératiniques par action sur les pigments initialement présents dans lesdites fibres.

Les exemples qui suivent sont destinés à illustrer l'invention.

### Exemple 1

On a préparé la composition de teinture suivante :

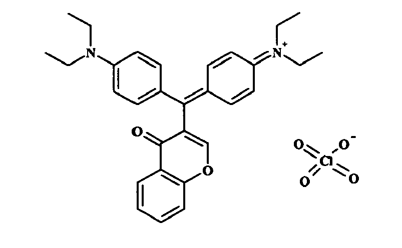

| | | |
|---|---|---|
| Ethanaminium,N-[4-[[4-(diéthylamino)phényl] (4-oxo-4H-1-benzopyran-3-yl)méthylène]-2,5-cyclohexadièn-1-ylidène]-N-éthyl-, perchlorate | | 0,553 g |
| Diéthanolamide oléïque | | 3 g |
| Acide laurique | | 1 g |
| Monoéthyléther de l'éthylèneglycol | | 5 g |
| Hydroxyéthylcellulose | | 2 g |
| 2-amino-2-méthyl-1-propanol | q.s. p.H. | 9,5 |
| Eau déminéralisée | q.s.p. | 100 g |

On a appliqué la composition ci-dessus sur des mèches de cheveux gris naturels ou permanentés à 90% de blancs et on a laissé poser pendant 30 minutes. Après rinçage à l'eau courante et séchage, les cheveux ont été teints dans une nuance verte.

### Exemple 2

On a préparé la composition de teinture suivante pour nuance gris noir violacé :

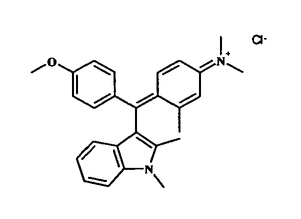

| | | | |
|---|---|---|---|
| {4-[(1,2-Diméthyl-1H-indol-3-yl)-(4-méthoxy-phényl)-methylène]- cyclohexa-2,5-diénylidène}-diméthylammonium chlorure | | | 0,569 g |
| Alcool benzylique | | | 4,0 g |
| Polyéthylèneglycol 6OE | | | 6,0 g |
| Hydroxyéthylcellulose | | | 0,7 g |
| Alkylpolyglucoside en solution aqueuse à 60% M.A* | | | 4,5 g M.A |
| Tampon phosphate | | q.s. pH | 7 |
| Eau déminéralisée | q.s.p. | | 100, g |

| | | | |
|---|---|---|---|
| * Matière Active | | | |

### Exemples 3 à 5

On a préparé les trois compositions de teinture directe réunies dans le tableau suivant :
(toutes teneurs exprimées en grammes)

| Exemple 3 | Exemple 4 | Exemple 5 |
|---|---|---|
| 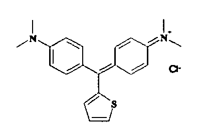 | 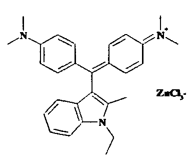 | 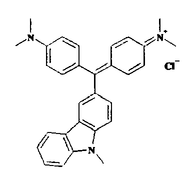 |
| Méthanaminium, N-[4-[[4-(diméthyl amino)phényl]-2-thiénylméthylène]-2,5-cyclo hexadièn-1-ylidène]-N-méthyl chlorure | {4-[(4-Diméthylamino-phényl)-(1-éthyl-2-méthyl-3a,7a-dihydro-1H-indol-3-yl)-méthylène] cyclohexa-2,5-diénylidène] diméthyl ammonium trichlorozincate | [4- [p- (Diméthyl amino)-α-(9-méthylcarbazol-3-yl) benzylidène]-2,5-cyclohexadièn-1-ylidène]diméthyl ammonium chlorure |

| | Exemple 3 | Exemple 4 | Exemple 5 |
|---|---|---|---|
| Colorant direct de formule (3) | 0,2 | | |
| Colorant direct de formule (4) | | 0,2 | |
| Colorant direct de formule (5) | | | 0,2 |
| Acide polyacrylique réticulé vendu sous la dénomination Carbopol 2984 par la société Goodrich | 1,0 MA* | | |
| Copolymère acrylate d'ammonium/acrylamide vendu sous la dénomination Bozepol C Nouveau par la société Hoechst | | 1,0 MA* | |
| Copolymère acide méthacrylique/acrylate d'éthyle réticulé vendu en dispersion aqueuse à 38% de matière active sous la dénomination Viscoatex 538C par la société Coatex | | | 1,0 MA* |
| Copolymère acide acrylique/acrylate d'éthyle réticulé vendu en dispersion aqueuse à 28% de matière active sous la dénomination Aculyn 33 par la société Rohm & Haas | | | |
| Ethanol | 10 | 10 | 10 |
| 2-amino-2-méthyl-1-propanol qs | pH 9 | pH 9 | pH 9 |
| Eau déminéralisée qsp | 100 | 100 | 100 |

| | | | |
|---|---|---|---|
| MA* désigne Matière Active | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| Exemples | Nuances obtenues |
|---|---|
| 3 | Vert puissant |
| 4 | Violet puissant |
| 5 | Bleu puissant |

### Exemples 6 à 9

On a préparé les compositions 6 (A) à 9 (A), conformes à l'invention, suivantes (teneurs en grammes) :

| COMPOSITION | 6 (A) | 7 (A) | 8 (A) | 9 (A) |
|---|---|---|---|---|
| Paratoluylènediamine | 0,25 | - | - | - |
| Para-aminophénol | 0,30 | 0,50 | 0,15 | |
| Paraphénylènediamine | | 0,20 | - | 0,30 |
| 5-N-β-hydroxyéthyl)amino 2-méthyl phénol | 0,5 | 0,8 | 0,17 | - |
| 5-amino 2-méthyl phénol | - | - | - | 0,30 |
| Colorant de structure (3) | 0,15 | - | - | |
| Colorant de structure (4) | - | 0,20 | 0,05 | - |
| Colorant de structure (5) | - | - | - | 0,1 |
| Support de teinture commun (*) | (*) | (*) | (*) | (*) |
| Eau q.s.p. | 100 g | 100 g | 100 g | 100 g |

| | | | | |
|---|---|---|---|---|
| (*) support de teinture commun : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN O12 par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | | | | |

Au moment de l'emploi, on a mélangé chacune de ces compositions 6 (A) à 9 (A) avec une quantité égale d'une composition (B) constituée par une solution de peroxyde d'hydrogène à 20 volumes (6 % en poids).

Chaque composition résultante (composition prête à l'emploi conforme à l'invention) a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| EXEMPLE [COMPOSITION] | NUANCE OBTENUE |
|---|---|
| 6 [6 (A)] | Blond foncé à reflet vert |
| 7 [7 (A)] | Blond à reflet violet intense |
| 8 [8 (A)] | Blond clair à reflet violet |
| 9 [9 (A)] | Blond à reflet bleu |

Les nuances obtenues ont présenté une très bonne ténacité aux shampooings ultérieurs.

Selon une variante de l'invention, les colorants directs peuvent être incorporés dans les compositions colorantes au moment de l'emploi.

### Exemple 10

On a préparé la composition 10 (A) suivante :
- 1,4-diaminobenzène 0,40 g
- 5-amino 2-méthyl phénol 0,45 g
- Support de teinture commun
   tel que décrit précédemment
   pour les exemples 1 à 4 (*)
- Eau déminéralisée q.s.p. 100 g

On a préparé la composition 10 (A') suivante :
- Colorant de l'exemple 4 4 g
- Polyammonium quaternaire vendu
   sous la dénomination commerciale
   CELQUAT SC-240 par la société
   National Starch 10 g
- Sciure de bois q.s.p. 100 g

Au moment de l'emploi, on a mélangé une partie en poids de la composition 10 (A) ci-dessus avec 0,1 partie en poids de la composition 10 (A') et avec une partie en poids d'une composition (B) constituée par une solution de peroxyde d'hydrogène à 20 volumes (6 % en poids).

La composition résultante a été appliquée pendant 30 minutes sur des mèches de cheveux gris naturels à 90 % de blancs. Les cheveux ont ensuite été rincées, lavées avec un shampooing standard puis séchées.

Les cheveux ont été teints dans une nuance châtain clair à reflet violet intense résistant très bien aux shampooings ultérieurs.

## Revendications

1. Composition pour la teinture des fibres kératiniques comprenant dans un milieu cosmétique approprié pour la teinture, au moins un colorant choisi parmi les composés répondant à la formule (I), (Ibis), (II) ou (IIbis) suivante ou leurs formes tautomères, et leurs sels d'addition : 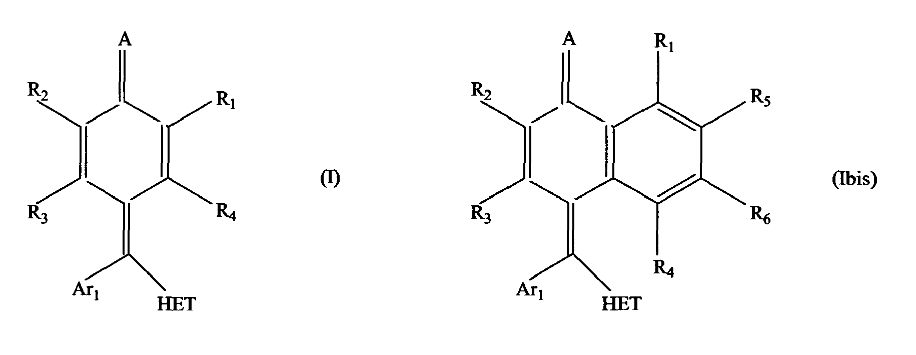 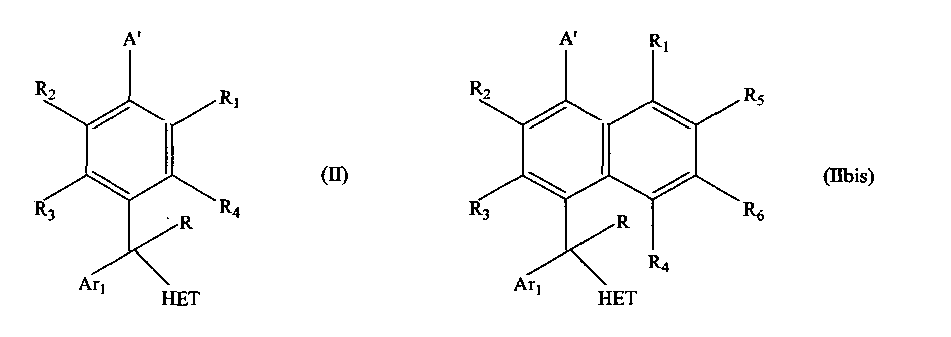 dans lesquelles :
- Ar₁ représente un radical aryle, tel que phényle ou naphtyle, éventuellement substitué par un ou plusieurs groupements Z ;
- HET représente un hétérocycle éventuellement substitué par un ou plusieurs groupements Z' ;
- A représente O ; NH ; N-alkyle ; N-hydroxyalkyle ; ammonium, N-alkylammonium, N-hydroxyalkylammonium, N,N-dialkylammonium, N,N-di(hydroxyalkyl)ammonium ou N-(hydroxyalkyl) N-(alkyl)ammonium, dans lesquels les deux groupements alkyle peuvent former avec l'atome d'azote auquel ils sont liés un cycle pouvant être interrompu par un ou plusieurs atomes d' azote d'oxygène ou de soufre ;
- R₁ à R₆ et Z, Z' désignent indépendamment l'un de l'autre un groupement choisi parmi l'atome d'hydrogène, les atomes d'halogène, tels que F, Cl, Br et I, les radicaux -NHSO₃H ; hydroxyle ; alkyle ; alcoxy ; alkylthio ; monoalkylamino ; dialkylamino, dans lesquels les deux groupements alkyle peuvent conjointement, avec l'atome d'azote, auquel ils sont liés, former un cycle pouvant être interrompu par un ou plusieurs atomes d'azote, d'oxygène ou de soufre ; les hétérocycles et les radicaux nitro ; aryle ; acyle ; alcoxycarbonyle; carboxamido; cyano ; -CO₂H ; -SO₃H ; -PO₃H₂ ;-PO₄H₂ ;
dans lesquelles :
- A' représente un atome d'hydrogène ; un groupe hydroxyle ; amino ; (hydroxyalkyl)amino ; monoalkylamino ; di(hydroxyalkyl)amino ; (hydroxyalkyl)(alkyl)amino ; (dialkyl)amino, dans lesquels les deux groupements alkyle peuvent former avec l'atome d'azote auquel ils sont liés un cycle pouvant être interrompu par un ou plusieurs atomes d'azote, d'oxygène ou de soufre ;
- R représente un atome d'hydrogène ou d'halogène ; un radical hydroxyle ; un radical alcoxy ; ou un radical alkylthio à l'exception du chlorhydrate de bis-(4-hydroxyphényl-1-(quinolyl-2)méthane et du bis-(4-hydroxyphényl)-(quinolyl-2) méthane.

2. Composition selon la revendication 1, dans laquelle HET représente un hétérocycle insaturé, éventuellement substitué par un ou plusieurs groupements Z'.

3. Composition selon la revendication 2, dans laquelle HET représente un hétérocycle aromatique, éventuellement substitué par un ou plusieurs groupements Z'.

4. Composition selon l'une quelconque des revendications 1 à 3, dans laquelle les hétérocycles, notamment ceux de HET, sont choisis parmi les hétérocycles suivants : thiophène, benzothiophène, furane, benzofurane, indole, indoline, carbazole, pyridine, déhydroquinoléine, chromone, julodinine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

5. Composition de teinture selon la revendication 1, **caractérisé en ce qu'**elle comprend un composé de formule (I), (Ibis), (II) ou (IIbis) choisi parmi les composés suivants pour lesquels les contre ions sont ou non spécifiés :
Méthanaminium, N-[4-[[4-(diméthylamino)phényl](2,2-diméthyl-2H-1-benzopyran-3-yl)méthylène] -2,5-cyclohexadièn-1-ylidène]-N-méthyl-, acétate Méthanaminium, N-[4-[[4-(diméthylamino)phényl](2,2-diméthyl-2H-1-benzopyran-3-yl)méthylène]-2,5-cyclohexadièn-1-ylidène]-N-méthyl-Méthanaminium, N-[4-[[4-(diméthylamino)phényl](2,2-diméthyl-2H-1-benzopyran-5-yl)méthylène]-2,5-cyclohexadièn-1-ylidéne]-N-méthyl-, acétate Méthanaminium, N-[4-[[4-(diméthylamino)phényl](2,2-diméthyl-2H-1-benzopyran-5-yl)méthylène]-2,5-cyclohexadièn-1-ylidène]-N-méthyl-Méthanaminium, N-[4-[[4-(diméthylamino)phényl](2,2-diméthyl-2H-1-benzopyran-4-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-4-yl)méthylène]-2,5-cyclohexadièn-1-ylidène]-N-méthyl-Méthanaminium, N-[4-[[4-(diméthylamino)phényl](2,2-diméthyl-2H-1-benzopyran-8-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-8-yl)méthylène]-2,5-cyclohexadièn-1-ylidène]-N-méthyl-Méthanaminium, N-[4-[[4-(diméthylamino)phényl](2,2-diméthyl-2H-1-benzopyran-7-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-7-yl)méthylène]-2,5-cyclohexadièn-1-ylidène]-N-méthyl-Méthanaminium, N-[4-[[4-(diméthylamino)phényl](2,2-diméthyl-2H-1-benzopyran-6-yl)méthylène]-2,5-cyclohexadièn-1-ylidène]-N-méthyl-, acétate Méthanaminium, N-[4-[[4-(diméthylamino)phényl](2,2-diméthyl-2H-1-benzopyran-6-yl)méthylène]-2,5-cyclohexadièn-1-ylidène]-N-méthyl-2,5-Cyclohexadièn-1-one, 4-[[4-amino-3,5-bis(1-méthyléthyl)phényl](4-éthyl-3,4-dihydro-2H-1,4-benzoxazin-7-yl)méthylène]-2,6-diméthoxy 2,5-Cyclohexadièn-1-one, 4-[[3,4-dihydro-4-(2-pyridinylméthyl)-2H-1,4-benzoxazin-7-yl](4-éthyl-3,4-dihydro-2H-1,4-benzoxazin-7-yl)méthylène]-2,6-diméthoxy-2,5-Cyclohexadièn-1-one, 4-[[4-(diméthylamino)phényl](4-éthyl-3,4-dihydro-2H-1,4-benzoxazin-7-yl)méthylène]-2,6-diméthoxy-2,5-Cyclohexadièn-1-one, 2,6-bis(1,1-diméthyléthyl)-4-[(5-éthyl-2-thiènyl)phénylméthylène]-Éthanaminium, N-[3-carboxy-4-[[2-carboxy-4-[éthyl(2-méthoxyéthyl)amino]phényl](7-carboxy-4-oxo-4H-1-benzothiopyran-3-yl)méthylène]-2,5-cyclohexadièn-1-ylidène]-N-éthyl-2-méthoxy Éthanaminium, N-[4-[[4-[bis(2-méthoxyéthyl)amino]-2-carboxyphényl](5-carboxy-2-pyridinyl)méthylène]-3-carboxy-2,5-cyclohexadièn-1-ylidène]-2-méthoxy-N-(2-méthoxyéthyl)-, Éthanaminium, N-[4-[3-benzofuranyl[2-carboxy-4-[éthyl[2-[(méthylsulfonyl)amino]éthyl]amino]phényl]méthylène]-3-carboxy-2,5-cyclohexadièn-1-ylidène]-N-éthyl-2-[(méthylsulfonyl)amino]-, perchlorate Éthanaminium, N-[4-[3-benzofuranyl[2-carboxy-4-[éthyl[2-[(méthylsulfonyl)amino]éthyl]amino]phényl]méthylène]-3-carboxy-2,5-cyclohexadièn-1-ylidène]-N-éthyl-2-[(méthylsulfonyl)amino]-Éthanaminium, N-[4-[[4-[bis(2-méthoxyéthyl)amino]-2-carboxyphényl](4-oxo-4H-1-benzothiopyran-3-yl)méthylène]-3-carboxy-2,5-cyclohexadièn-1-ylidène]-2-méthoxy-N-(2-méthoxyéthyl)-, acétate Éthanaminium, N-[4-[[4-[bis(2-méthoxyéthyl)amino]-2-carboxyphényl](4-oxo-4H-1-benzothiopyran-3-yl)méthylène]-3-carboxy-2,5-cyclohexadièn-1-ylidène]-2-méthoxy-N-(2-méthoxyéthyl)-Sel d'acide Benzoique et de, 5-[(3-carboxy-4-oxo-2,5-cyclohexadièn-1-ylidène)-3-pyridinylméthyl]-2-hydroxy-Méthanaminium, N-[2-carboxy-4-[[3-carboxy-4-(diméthylamino)phényl](4-oxo-4H-1-benzothiopyran-3-yl)méthylène]-2,5-cyclohexadièn-1-ylidène]-N-méthyl-, perchlorate Méthanaminium, N-[2-carboxy-4-[[3-carboxy-4-(diméthylamino)phényl](4-oxo-4H-1-benzothiopyran-3-yl)méthylène]-2,5-cyclohexadièn-1-ylidène]-N-méthyl-Sel d'acide benzoique et de 5-[(3-carboxy-4-oxo-2,5-cyclohexadièn-1-ylidène)[3,5-diméthyl-1-[2-[(méthylsulfonyl)amino]éthyl]-1H-pyrazol-4-yl]méthyl]-2-hydroxy-Sel d'acide benzoique et de 5-[[2-[bis(2-méthoxyéthyl)amino]-5-thiazolyl](3-carboxy-4-oxo-2,5-cyclohexadièn-1-ylidène)méthyl]-2-hydroxy-Éthanaminium, N-[4-[(7-carboxy-2-oxo-2H-1-benzopyran-4-yl)[4-(diéthylamino)phényl]méthylène]-2,5-cyclohexadièn-1-ylidène]-N-éthyl-, perchlorate Éthanaminium, N-[4-[(7-carboxy-2-oxo-2H-1-benzopyran-4-yl)[4-(diéthylamino)phényl]méthylène]-2,5-cyclohexadièn-1-ylidène]-N-éthyl-Éthanaminium, N-[4-[2-benzofuranyl[4-[éthyl[2-[(méthylsulfonyl)amino] éthyl]amino]phényl]méthylène]-2,5-cyclohexadièn-1-ylidène]-N-éthyl-2-[(méthylsulfonyl)amino]-, perchlorate Éthanaminium, N-[4-[2-benzofuranyl[4-[éthyl[2-[(méthylsulfonyl)amino]éthyl]amino]phényl]méthylène]-2,5-cyclohexadièn-1-ylidène]-N-éthyl-2-[(méthylsulfonyl)amino]-Éthanaminium, N-[4-[(6-carboxybenzo[b]thièn-2-yl)[4-(diéthylamino)phényl]méthylène]-2,5-cyclohexadièn-1-ylidène]-N-éthyl-, tétrafluoroborate(1-) Éthanaminium, N-[4-[(6-carboxybenzo[b]thièn-2-yl)[4-(diéthylamino)phényl]méthylène]-2,5-cyclohexadièn-1-ylidène]-N-éthyl-Éthanaminium, N-[4-[[5-[bis(2-méthoxyéthyl)amino]-2-furanyl][4- [éthyl [2-[(méthylsulfonyl)amino] éthyl]amino]phényl]méthylène]-2,5-cyclohexadièn-1-ylidène] -N-éthyl-2-[(méthylsulfonyl)amino]-, perchlorate Éthanaminium, N-[4-[[5-[bis(2-méthoxyéthyl)amino]-2-furanyl][4-[éthyl[2-[(méthylsulfonyl)amino]éthyl]amino]phényl]méthylène]-2,5-cyclohexadièn-1-ylidène]-N-éthyl-2-[(méthylsulfonyl)amino]-2,5-Cyclohexadièn-1-one, 4-[(4-hydroxyphényl)-4-quinolinylméthylène]-Éthanaminium,N-[4-[[4-[bis(2-méthoxyéthyl)amino]phényl](7-carboxy-4-oxo-4H-1-benzopyran-3-yl)méthylène]-2,5-cyclohexadièn-1-ylidène]-2-méthoxy-N-(2-méthoxyéthyl)-, sulfate (1:1) Éthanaminium, N-[4-[[4-[bis(2-méthoxyéthyl)amino]phényl](7-carboxy-4-oxo-4H-1-benzopyran-3-yl)méthylène]-2,5-cyclohexadièn-1-ylidène]-2-méthoxy-N-(2-méthoxyéthyl)-Méthanaminium, N-[4-[(5-carboxy-2-pyridinyl)[4-(diméthylamino)phényl]méthylène] -2,5-cyclohexadièn-1-ylidène]N-méthyl-, perchlorate Méthanaminium, N-[4-[(5-carboxy-2-pyridinyl)[4- (diméthylamino)phényl]méthylène] 2,5-cyclohexadièn-1-ylidène]-N-méthyl-Acide 2-Thiophénecarboxylique, 5-[(4-hydroxyphényl)(4-oxo-2,5-cyclohexadièn-1-ylidène)méthyl]-2,5-Cyclohexadièn-1-one, 4-[2-furanyl(4-hydroxyphényl)méthylène]-2,5-Cyclohexadièn-1-one, 4-[(4-hydroxyphényl)-2-pyridinylméthylène]-Méthanaminium, N-[3-carboxy-4-[[2-carboxy-4-(diméthylamino)phényl](4-oxo-4H-1-benzopyran-3-yl)méthylène]-2,5-cyclohexadièn-1-ylidène]-N-méthyl, perchlorate Méthanaminium, N-[3-carboxy-4-[[2-carboxy-4-(diméthylamino)phényl](4-oxo-4H-1-benzopyran-3-yl)méthylène]-2,5-cyclohexadièn-1-ylidène]-N-méthyl-Éthanaminium, N-[4-[(5-carboxy-2-thiènyl)[4-(diéthylamino)phényl]méthylène]-2,5-cyclohexadièn-1-ylidène]-N-éthyl-, perchlorate Éthanaminium, N-[4-[(5-carboxy-2-thiènyl)[4-(diéthylamino)phényl]méthylène]-2,5-cyclohexadièn-1-ylidène]-N-éthyl-2,5-Cyclohexadièn-1-one, 4-[(4-méthoxyphényl)-3,6,9,12,15-pentaoxabicyclo[15.3.1]héneicosa-1(21),17,19-trièn-21-ylméthylène]-3,6,9,12,15-Pentaoxabicyclo[15.3.1]héneicosane, 2,5-cyclohexadièn-1-one Méthanaminium, N-[4-[(2,3-dihydro-3-méthyl-2-benzothiazolyl)[4-(diméthylamino)phényl]méthylène]-2,5-cyclohexadièn-1-ylidène]-N-méthyl-, chlorure [4-(α-Benzo[b]thièn-2-yl-p-diméthylaminobenzylidène)-2,5-cyclohexadièn-1-ylidène] diméthylammonium chlorure [4-(α-Benzo[b]thièn-3-yl-p-diméthylaminobenzylidène)-2,5-cyclohexadièn-1-ylidène] diméthylammonium chlorure Méthanaminium, N-[4-[[9-(diméthylamino)benzo[a]phénoxazin-7-ium-5-yl][4-(diméthylamino)phényl]méthylène]-2,5-cyclohexadièn-1-ylidène]-N-méthyl-, bis[tétraphénylborate(1-)] Méthanaminium Benzo[a]phénoxazin-7-ium, Méthanaminium, N-[4-[[9-(diméthylamino)benzo[a]phénoxazin-7-ium-5-yl][4-(diméthylamino) phényl]méthylène]-2,5-cyclohexadèn-1-ylidène]-N-méthyl-[4-(α-3-Bromobenzo[b]thièn-2-yl-p-diméthylaminobenzylidène)-2,5-cyclohexadièn-1-ylidène]diméthylammonium chlorure [4-(α-3-Chlorobenzo[b]thièn-2-yl-p-diméthylaminobenzylidène)-2,5-cyclohexadièn-1-ylidène]diméthylammonium chlorure [4-(p-Diméthylamino-α-3-méthylbenzo[b]thièn-2-ylbenzylidène)-2,5-cyclohexadièn-1-ylidène]diméthylammonium chlorure [4-[2-Bromo-α-(p-diméthylaminophényl)-3-thénylidène]-2,5-cyclohexadièn-1-ylidène]diméthylammonium chlorure [4-[2-Chloro-α-(p-diméthylaminophényl)-3-thénylidène]-2,5-cyclohexadièn-1-ylidène]diméthylammonium chlorure [4-[α-(p-Diméthylaminophényl)-3-thénylidène]-2,5-cyclohexadièn-1-ylidène]diméthylammonium chlorure [4-[α-(p-Diméthylaminophényl)-2,5-diméthyl-3-thénylidène]-2,5-cyclohexadièn-1-ylidène]diméthylammonium chlorure Pyridinium, 1-[[4-(diméthylamino)phényl][4-(diméthyliminio)-2,5-cyclohexadièn-1-ylidène]méthyl]-, bis[tétrafluoroborate(1-)] 2,5-Cyclohexadièn-1-one, 2,3,5,6-tétrachloro-4-[(pentachlorophényl)(2,3,5,6-tétrachloro-4-pyridinyl)méthylène]-Éthanaminium, N-[4-[[4-(diéthylamino)phényl](4-oxo-4H-1-benzothiopyran-3-yl)méthylène]-2,5-cyclohexadièn-1-ylidène]-N-éthyl-, perchlorate Éthanaminium, N-[4-[[4-(diéthylamino)phényl](4-oxo-4H-1-benzothiopyran-3-yl)méthylène]-2,5-cyclohexadièn-1-ylidène]-N-éthyl-4H-1-Benzothiopyran, éthanaminium deriv. Benzènaminium, N-méthyl-N-[4-[[4-(méthylphénylamino)phényl](4-oxo-4H-1-benzothiopyran-3-yl)méthylène]-2,5-cyclohexadièn-1-ylidène]-, perchlorate Benzènaminium, N-méthyl-N-[4-[[4-(méthylphénylamino)phényl](4-oxo-4H-1-benzothiopyran-3-yl)méthylène]-2,5-cyclohexadièn-1-ylidène]-Éthanaminium, N-[4-[[4-(diéthylamino)-2-méthylphényl] (4-oxo-4H-1-benzothiopyran-3-yl)méthylène]-3-méthyl-2,5-cyclohexadièn-1-ylidéne]-N-éthyl-, perchlorate Éthanaminium, N-[4-[[4-(diéthylamino)-2-méthylphényl](4-oxo-4H-1-benzothiopyran-3-yl)méthylène]-3-méthyl-2,5-cyclohexadièn-1-ylidène]-N-éthyl-Ethanaminium 4H-1-Benzothiopyran, Méthanaminium, N-[4-[[4-(diméthylamino)phényl][5-(4-iodophényl)-2-furanyl]méthylène]-2,5-cyclohexadièn-1-ylidène]-N-méthyl-, chlorure Méthanaminium, N-[4-[[5-(4-bromophényl)-2-furanyl][4-(diméthylamino)phényl]méthylène]-2,5-cyclohexadièn-1-ylidène]-N-méthyl-, chlorure Méthanaminium, N-[4-[[4-(diméthylamino)phényl][5-(4-nitrophényl)-2-furanyl]méthylène]-2,5-cyclohexadièn-1-ylidène]-N-méthyl-, chlorure [Oxybis[méthylène-5,2-furandiyl[p-(N-méthylanilino)benzylidyne]-2,5-cyclohexadiène-4,1-diylidène]]bis[méthylphénylammonium chlorure] [Oxybis[méthylène-5,2-furandiyl[p-(N-méthylanilino)benzylidyne]-2,5-cyclohexadiène-4,1-diylidène]]bis[méthylphénylammonium hydrogènosulfate] Benzènaminium, N,N'-[oxybis[méthylène-5,2-furandiyl[[4-(méthylphénylamino)phényl]méthylidyne]-2,5-cyclohexadiène-4,1-diylidène]]bis[N-méthyl-Sel d'acide 1,4-Cyclohexadiène-1-sulfonique et de, 3-[4-anilino-α-(1-méthyl-2-phénylindol-3-yl)-3-sulfobenzylidène]-6-phénylimino-Sel d'acide trifluorométhane sulfonique et de Pyridinium, 1-[[4-(diméthylamino)phényl]4-(diméthyliminio)-2,5-cyclohexadièn-1-ylidène]méthyl Sel d'acide trifluorométhane sulfonique et de 1-[[4-(diméthylamino)phényl][4-(diméthyliminio)-2,5-cyclohexadièn-1-ylidène]méthyl]pyridinium Pyridinium, 1-[[4-(diméthylamino)phényl][4-(diméthyliminio)-2,5-cyclohexadièn-1-ylidène]méthyl]-[4-[p-(Diméthylamino)-α-(9-méthylcarbazol-3-yl)benzylidène]-2,5-cyclohexadièn-1-ylidène]diméthylammonium chlorure [4-[α-[p-(Diméthylamino)phényl]furfurylidène]-2,5-cyclohexadièn-1-ylidène]diméthylammonium chlorure Méthanaminium, N-[4-[bis (1,2,3,4,10,14b-hexahydro-2-méthyldibenzo[c,f]pyrazino [1,2-a]azépin-8-yl)méthylène]-2,5-cyclohexadièn-1-ylidène]-N-méthyl-, perchlorate, diperchlorate Méthanaminium, N-[4-[bis(1,2,3,4,10,14b-hexahydro-2-méthyldibenzo[c,f]pyrazino[1,2-a]azépin-8-yl)méthylène]-2,5-cyclohexadièn-1-ylidène]-N-méthyl-, perchlorate Méthanaminium, N-[4-[bis(1,2,3,4,10,14b-hexahydro-2-méthyldibenzo[c,f]pyrazino [1,2-a]azépin-8-yl)méthylène]-2,5-cyclohexadièn-1-ylidène]-N-méthyl-1H-Indolizinium, 3-[[4-(diméthyliminio)-2,5-cyclohexadièn-1-ylidène](4-méthoxyphényl)méthyl]-2-(2-hydroxy-5-méthylphényl)-1-méthyl-, 1H-Indolizinium, 3-[[4-(diméthyliminio)-2,5-cyclohexadièn-1-ylidène]phénylméthyl]-2-(2-hydroxy-5-méthylphényl)-1-méthyl-, 1H-Indolizinium, 3-[[4-(diméthylamino)phényl][4-(diméthyliminio)-2, 5-cyclohexadièn-1-ylidène]méthyl]-2-(2-hydroxy-5-méthylphényl)-1-méthyl-, 1H-Indolizinium, 3-[[4-(diméthylamino)phényl][4-(diméthyliminio)-2, 5-cyclohexadièn-1-ylidène]méthyl]-2-(2-hydroxy-5-méthylphényl)-1-méthyl-1-Propanaminium, N-[4-[2-benzofuranyl[3-chloro-4-[éthyl(3-sulfopropyl)amino] phényl]méthylène]-2-chloro-2,5-cyclohexadièn-1-ylidène]-N-éthyl-3-sulfo-, sel interne, sel de sodium 1-Propanaminimn, N-[4-[2-benzofuranyl[4-[éthyl(3-sulfopropyl)amino]-3-méthylphényl]méthylène]-2-méthyl-2,5-cyclohexadèn-1-ylidène]-N-éthyl-3-1-Butanaminium, N-[4-[2-benzofuranyl[4-[éthyl(4-sulfobutyl)amino]phényl]méthylène]-2,5-cyclohexadièn-1-ylidène]-N-éthyl-4-1-Propanaminium, N-[4-[(5-chlorobenzo[b]thièn-2-yl)[4-[méthyl(3-sulfopropyl)amino]phényl]méthylène]-2,5-cyclohexadièn-1-ylidène]-N-méthyl-3-sulfo-, 1-Propanaminium, N-[4-[(3-chlorobenzo[b]thièn-2-yl)[4-[méthyl(3-sulfopropyl)amino]phényl]méthylène]-2,5-cyclohexadièn-1-ylidène]-N-méthyl-3-sulfo-, sel de sodium 1-Propanaminium, N-[4-[benzo[b]thièn-2-yl[4-[méthyl(3-sulfopropyl)amino]phényl]méthylène]-2,5-cyclohexadièn-1-ylidène]-N-méthyl-3-sulfo-, sel de sodium 1-Propanaminium, N-méthyl-N-[4-[(3-méthylbenzo[b]thièn-2-yl)[4-[méthyl(3-sulfopropyl)amino]phényl]méthylène]-2,5-cyclohexadièn-1-ylidène]-3-sulfo-, sel de sodium 1-Propanaminium, N-[4-[(3-bromobenzo[b]thièn-2-yl)[4-[méthyl(3-sulfopropyl)amino]phényl]méthylène]-2,5-cyclohexadièn-1-ylidène]-N-méthyl-3-sulfo-, sel de sodium 1-Propanaminium, N-[4-[(6-chlorobenzo[b]thièn-2-yl)[4-[méthyl(3-sulfopropyl)amino]phényl]méthylène]-2,5-cyclohexadièn-1-ylidène]-N-méthyl-3-sulfo-, sel de sodium 1-Propanaminium, N-méthyl-N-[4-[[4-[méthyl(3-sulfopropyl)amino]phényl](5-nitro-2-benzofuranyl)méthylène]-2,5-cyclohexadièn-1-ylidène]-3-sulfo-, sel de sodium 1-Propanaminium, N-méthyl-N-[4-[(5-méthyl-2-benzofuranyl)[4-[méthyl (3-sulfopropyl)amino]phényl]méthylène]-2,5-cyclohexadièn-1-ylidène]-3-sulfo-, sel de sodium 1-Propanaminium, N-[4-[(5-chloro-2-benzofuranyl)[4-[méthyl(3-sulfopropyl)amino]phényl]méthylène]-2,5-cyclohexadièn-1-ylidène]-N-méthyl-3-sulfo-, sel de sodium 1-Propanaminium, N-[4-[2-benzofuranyl[4-[méthyl(3-sulfopropyl)amino]phényl]méthylène]-2,5-cyclohexadièn-1-ylidène]-N-méthyl-3-sulfo-, sel interne, sel de sodium Éthanaminium, N-[4-[(2-chlorophényl)(1-méthyl-2-phényl-1H-indol-3-yl)méthylène]-2,5-cyclohexadièn-1-ylidène] -N-éthyl-, trichlorozincate(1-) Zincate(1-), trichloro-, N-[4-[(2-chlorophényl)(1-méthyl-2-phényl-1H-indol-3-yl)méthylène]-2,5-cyclohexadièn-1-ylidène]-N-éthyléthanaminium Benzènesulfonic acid, 5-[(1,2-diméthyl-1H-indol-3-yl)[4-[(2-méthylphényl)imino]-3-sulfo-2,5-cyclohexadièn-1-ylidène]méthyl]-2-[(2-méthylphényl)amino]-, monosel de sodium Éthanaminium, N-[4-[(2-chlorophényl)(1-méthyl-2-phényl-1H-indol-3-yl)méthylène]-2,5-cyclohexadièn-1-ylidène]-N-éthyl-, méthyl sulfate 3H-Pyrazol-3-one, 1,2-dihydro-4-[(4-hydroxy-3-méthoxyphényl)(3-méthoxy-4-oxo-2,5-cyclohexadièn-1-ylidène)méthyl]-1,5-diméthyl-2-phényl-, ion(1-) Éthanaminium, N-[4-[(2-chlorophényl)(1-méthyl-2-phényl-1H-indol-3-yl)méthylène]-2,5-cyclohexadièn-1-ylidène]-N-éthyl-, acétate Éthanaminium, N-[4-[(2-chlorophényl)(1-méthyl-2-phényl-1H-indol-3-yl)méthylène]-2,5-cyclohexadièn-1-ylidène]-N-éthyl-, phosphate (1:1) Éthanaminium, N-[4-[(2-chlorophényl)(1-méthyl-2-phényl-1H-indol-3-yl)méthylène]-2,5-cyclohexadièn-1-ylidène]-N-éthyl-Éthanaminium, N-[4-[(2-chlorophényl)(1-méthyl-2-phényl-1H-indol-3-yl)méthylène]-2,5-cyclohexadièn-1-ylidène]-N-éthyl-, chlorure, composé avec le chlorure de zinc (ZnCl₂) N-[4-[(2-chlorophényl)(1-méthyl-2-phényl-1H-indol-3-yl)méthylène]-2,5-cyclohexadièn-1-ylidène]-N-éthyléthanaminium chlorure 1-Pipéridinyloxy, 4-[[3,5-bis(1,1-diméthyléthyl)-4-oxo-2,5-cyclohexadièn-1-ylidène][3,5-bis(1,1-diméthyléthyl)-4-oxyphényl]méthyl]-2,2,6,6-tétraméthyl-1H-Pyrrol-1-yloxy, 3-[[3,5-bis(1,1-diméthyléthyl)-4-oxo-2,5-cyclohexadièn-1-ylidène][3,5-bis(1,1-diméthyléthyl)-4-oxyphényl]méthyl]-2,5-dihydro-2,2,5,5-tétraméthyl-1H-Pyrrol-1-yloxy, 3-[[3,5-bis(1,1-diméthyléthyl)-4-hydroxyphényl][3,5-bis(1,1-diméthyléthyl)-4-oxo-2,5-cyclohexadièn-1-ylidène]méthyl]-2,5-dihydro-2,2,5,5-tétraméthyl-1-Pipéridinyloxy, 4-[[3,5-bis(1,1-diméthyléthyl)-4-hydroxyphényl][3,5-bis(1,1-diméthyléthyl)-4-oxo-2,5-cyclohexadièn-1-ylidène]méthyl]-2,2,6,6-tétraméthyl-Acétamide, N-méthyl-N-[[2-[[4-oxo-3-[2,2,2-trifluoro-1-hydroxy-1-(trifluorométhyl)éthyl]-1(4H)-naphthalènylidène](2,3,6,7-tétrahydro-1H,5H-benzo [ij]quinolizin-9-yl)méthyl]phényl]sulfonyl]-Ester d'acide carbamique et de [[2-[(3,5-diméthoxy-4-oxo-2,5-cyclohexadièn-1-ylidène)(2,3,6,7-tétrahydro-1H,5H-benzo[ij]quinolizin-9-yl)méthyl]phényl]sulfonyl]méthyl-, 2-(méthylsulfonyl)éthyl Ester d'acide carbamique et de, méthyl[[2-[[4-oxo-3-[2,2,2-trifluoro-1-hydroxy-1-(trifluorométhyl)éthyl]-1(4H)-naphthalènylidène](2,3,6,7-tétrahydro-1H,5H-benzo[ij]quinolizin-9-yl)méthyl]phényl]sulfonyl]-, 2-cyanoéthyl Ester d'acide carbamique et de méthyl[[2-[[4-oxo-3-[2,2,2-trifluoro-1-hydroxy-1-(trifluorométhyl)éthyl]-1(4H)-naphthalènylidène]](2,3,6,7-tétrahydro-1H,5H-benzo[ij]quinolizin-9-yl)méthyl]phényl]sulfonyl]-, 2-(méthylsulfonyl)éthylester Ester d'acide Carbamique et de méthyl[[2-[[4-oxo-3-[2,2,2-trifluoro-1-hydroxy-1-(trifluorométhyl)éthyl]-1(4H)-naphthalènylidène](2,3,6,7-tétrahydro-1H,5H-benzo[ij]quinolizin-9-yl)méthyl]phényl]sulfonyl]-, 2-(phénylsulfonyl)éthyl Acétamide, N-[[2-[(3,5-diméthyl-4-oxo-2,5-cyclohexadièn-1-ylidène)(2,3,6,7-tétrahydro-1H,5H-benzo[ij]quinolizin-9-yl)méthyl]phényl]sulfonyl]-N-méthyl-2,5-Cyclohexadièn-1-one, 4-[(4-hydroxy-3,5-diméthylphényl)(2,3,5,6,8,9,11,12-octahydro-1,4,7,10,13-benzopentaoxacyclopentadecin-15-yl)méthylène]-2,6-diméthyl-Phénoxy, 4-[[3,5-bis(1,1-diméthyléthyl)-4-oxo-2,5-cyclohexadièn-1-ylidène](2,3,5,6,8,9,11,12-octahydro-1,4,7,10,13 benzopentaoxacyclopentadecin-15-yl)méthyl]-2,6-bis(1,1-diméthyléthyl)-Éthanaminium, N-[4-[(2-chlorophényl)(1-méthyl-2-phényl-1H-indol-3-yl)méthylène]-2,5-cyclohexadièn-1-ylidène]-N-éthyl-, chlorure Phénoxy, 4-[[3,5-bis(1,1-diméthyléthyl)-4-oxo-2,5-cyclohexadièn-1-ylidène]-3-pyridinylméthyl]-2,6-bis(1,1-diméthyléthyl)-2,5-Cyclohexadièn-1-one, 4-[[3,5-bis(1,1-diméthyléthyl)-4-hydroxyphényl]-3-pyridinylméthylène]-2,6-bis(1,1-diméthyléthyl)-, ion(1-) 2,5-Cyclohexadièn-1-one, 4-[[3,5-bis(1,1-diméthyléthyl)-4-hydroxyphényl]-3-pyridinylméthylène]-2,6-bis(1,1-diméthyléthyl)-Phénoxy, 4,4'-[3,5-pyridinediylbis[[3,5-bis(1,1-diméthyléthyl)-4-oxo-2,5-cyclohexadièn-1-ylidène]méthylène]]bis[2,6-bis(1,1-diméthyléthyl)-Phénoxy, 4,4'-[2,4-pyridinediylbis[[3,5-bis(1,1-diméthyléthyl)-4-oxo-2,5-cyclohexadièn-1-ylidène]méthylène]]bis[2,6-bis(1,1-diméthyléthyl)-Phénoxy, 4,4'-[2,5-pyridinediylbis[[3,5-bis(1,1-diméthyléthyl)-4-oxo-2,5-cyclohexadièn-1-ylidène]méthylène]]bis[2,6-bis(1,1-diméthyléthyl)-Méthanaminium, N-[4-[9H-carbazol-3-yl[4-(diméthylamino)phényl]méthylène]-2,5-cyclohexadièn-1-ylidène]-N-méthyl-, chlorure [4-[α-Carbazol-3-yl-p-(diméthylamino)benzylidène]-2,5-cyclohexadièn-1-ylidène]diméthylammonium chlorure Benzènamine, 4-éthoxy-N-[4-[[4-[(4-éthoxyphényl)amino]phényl](1-méthyl-2-phényl-1H-indol-3-yl)méthylène]-2,5-cyclohexadièn-1-ylidène]-, acétate Benzènamine, 4-éthoxy-N-[4-[[4-[(4-éthoxyphényl)amino]phényl](1-méthyl-2-phényl-1H-indol-3-yl)méthylène]-2,5-cyclohexadièn-1-ylidène]-(2,5-Cyclohexadièn-1-one, 4,4'-[3,5-pyridinediylbis[[3,5-bis(1,1-diméthyléthyl)-4-hydroxyphényl]méthylidyne]]bis[2,6-bis(1,1-diméthyléthyl)-, ion (2-)-2,5-Cyclohexadièn-1-one, 4,4'-[2,6-pyridinediylbis[[3,5-bis(1,1-diméthyléthyl)-4-hydroxyphényl]méthylidyne]]bis[2,6-bis(1,1-diméthyléthyl)-, ion(2-)-2,5-Cyclohexadièn-1-one, 4,4'-[2,5-pyridinediylbis[[3,5-bis(1,1-diméthyléthyl)-4-hydroxyphényl]méthylidyne]]bis[2,6-bis(1,1-diméthyléthyl)-, ion(2-)-2,5-Cyclohexadièn-1-one, 4,4'-[2,4-pyridinediylbis[[3,5-bis(1,1-diméthyléthyl)-4-hydroxyphényl]méthylidyne]]bis[2,6-bis(1,1-diméthyléthyl)-, ion(2-)-2,5-Cyclohexadièn-1-one, 4,4'-[3,5-pyridinediylbis[[3,5-bis(1,1-diméthyléthyl)-4-hydroxyphényl]méthylidyne]]bis[2,6-bis(1,1-diméthyléthyl)-2,5-Cyclohexadièn-1-one, 4,4'-[2,5-pyridinediylbis[[3,5-bis(1,1-diméthyléthyl)-4-hydroxyphényl]méthylidyne]]bis[2,6-bis(1,1-diméthyléthyl)-2,5-Cyclohexadièn-1-one, 4,4'-[2,4-pyridinediylbis[[3,5-bis(1,1-diméthyléthyl)-4-hydroxyphényl]méthylidyne]]bis[2,6-bis(1,1-diméthyléthyl)-Cyclohexanaminium, N-méthyl-N-[4-[(2-méthyl-1H-indol-3-yl)phénylméthylène]-2,5-cyclohexadièn-1-ylidène]-, trichlorozincate(1-) Zincate(1-), trichloro-, N-méthyl-N-[4-[(2-méthyl-1H-indol-3-yl)phénylméthylène]-2,5-cyclohexadièn-1-ylidène]cyclohexanaminium Cyclohexanaminium, N-méthyl-N-[4-[(2-méthyl-1H-indol-3-yl)phénylméthylène]-2,5-cyclohexadièn-1-ylidène]-Cyclohexanaminium, N-[4-[(2-chlorophényl)(1-méthyl-2-phényl-1H-indol-3-yl)méthylène]-2,5-cyclohexadièn-1-ylidène]-N-méthyl-, trichlorozincate(1-) Zincate(1-), trichloro-, N-[4-[(2-chlorophényl)(1-méthyl-2-phényl-1H-indol-3-yl)méthylène]-2,5-cyclohexadièn-1-ylidène]-N-méthylcyclohexanaminium Cyclohexanaminium, N-[4-[(2-chlorophényl)(1-méthyl-2-phényl-1H-indol-3-yl)méthylène]-2,5-cyclohexadièn-1-ylidène]-N-méthyl-Cyclohexanaminium, N-[4-[(2-chlorophényl)[2-(4-chlorophényl)-1-méthyl-1H-indol-3-yl]méthylène]-2,5-cyclohexadièn-1-ylidène]-N-méthyl-,trichlorozincate(1-) Trichlorozincate de-, N-[4-[(2-chlorophényl)[2-(4-chlorophényl)-1-méthyl-1H-indol-3-yl]méthylène]-2,5-cyclohexadièn-1-ylidène]-N-méthylcyclohexanaminium Cyclohexanaminium, N-[4-[(2-chlorophényl)[2-(4-chlorophényl)-1-méthyl-1H-indol-3-yl]méthylène]-2,5-cyclohexadièn-1-ylidène]-N-méthyl-Benzèneméthanaminium, N-cyclohexyl-N-[4-[[1-(2-éthoxyéthyl)-2-phényl-1H-indol-3-yl](4-méthylphényl)méthylène]-2,5-cyclohexadièn-1-ylidène]-, trichlorozincate(1-) Trichlorozincate de N-cyclohexyl-N-[4-[[1-(2-éthoxyéthyl)-2-phényl-1H-indol-3-yl](4-méthylphényl)méthylène]-2,5-cyclohexadièn-1-ylidène]benzèneméthanaminium Benzèneméthanaminium, N-cyclohexyl-N-[4-[[1-(2-éthoxyéthyl)-2-phényl-1H-indol-3-yl](4-méthylphényl)méthylène]-2,5-cyclohexadièn-1-ylidènel-Cyclohexanaminium, N-méthyl-N-[4-[(1-méthyl-2-phényl-1H-indol-3-yl)phénylméthylène]-2,5-cyclohexadièn-1-ylidène]-, trichlorozincate(1-) Trichlorozincate de N-méthyl-N-[4-[(1-méthyl-2-phényl-1H-indol-3-yl)phénylméthylène]-2,5-cyclohexadièn-1-ylidène]cyclohexanaminium Cyclohexanaminium, N-méthyl-N-[4-[(1-méthyl-2-phényl-1H-indol-3-yl)phénylméthylène]-2,5-cyclohexadièn-1-ylidène]-Cyclohexanaminium, N-[4-[(2-bromophényl)(1,2-diméthyl-1H-indol-3-yl)méthylène]-2,5-cyclohexadièn-1-ylidène]-N-éthyl-, trichlorozincate(1-) Trichlorozincate de N-[4-[(2-bromophényl)(1,2-diméthyl-1H-indol-3-yl)méthylène]-2,5-cyclohexadièn-1-ylidène]-N-éthylcyclohexanaminium Cyclohexanaminium, N-[4-[(2-bromophényl)(1,2-diméthyl-1H-indol-3-yl)méthylène]-2,5-cyclohexadièn-1-ylidène]-N-éthyl-Éthanaminium, N-[3-chloro-4-[(1-méthyl-2-phényl-1H-indol-3-yl)phénylméthylène]-2,5-cyclohexadièn-1-ylidène]-N-éthyl-, sulfate (1:1) Éthanaminium, N-[3-chloro-4-[(1-méthyl-2-phényl-1H-indol-3-yl)phénylméthylène]-2,5-cyclohexadièn-1-ylidène]-N-éthyl-Éthanaminium, N-éthyl-N-[4-[(2-méthyl-1H-indol-3-yl)(2-nitrophényl)méthylène]-2,5-cyclohexadièn-1-ylidène]-, sulfate (1:1) Éthanaminium, N-éthyl-N-[4-[(2-méthyl-1H-indol-3-yl)(2-nitrophényl)méthylène]-2,5-cyclohexadièn-1-ylidène]-Méthanamine, N-[4-[(1-méthyl-2-phényl-1H-indol-3-yl)phénylméthylène]-2,5-cyclohexadièn-1-ylidène]-, sulfate (1:1) Méthanamine, N-[4-[(1-méthyl-2-phényl-1H-indol-3-yl)phénylméthylène]-2,5-cyclohexadièn-1-ylidène]-Méthanaminium, N-[4-[[4-(diméthylamino)phényl]-2-furanylméthylène]-2,5-cyclohexadièn-1-ylidène]-N-méthyl-, (T-4)-tétrabromothallate(1-) Thallate (1-), tétrabromo-, (T-4)-, N-[4-[[4-(diméthylamino)phényl]-2-furanylméthylène]-2,5-cyclohexadièn-1-ylidène]-N-méthylméthanaminium Phénoxy, 4-[[3,5-bis(1,1-diméthyléthyl)-4-oxo-2,5-cyclohexadièn-1-ylidène][6-[[3,5-bis(1,1-diméthyléthyl)-4-oxo-2,5-cyclohexadièn-1-ylidène][3,5-bis(1,1-diméthyléthyl)-4-oxyphényl]méthyl]-2-pyridinyl]méthyl]-2,6-bis(1,1-diméthyléthyl)-2,5-Cyclohexadièn-1-one, 4,4'-[2,6-pyridinediylbis[[3,5-bis(1,1-diméthyléthyl)-4-hydroxyphényl]méthylidyne]]bis[2,6-bis(1,1-diméthyléthyl)-Méthanaminium, N-[4-[(2,3-dihydro-1,5-diméthyl-3-oxo-2-phényl-1H-pyrazol-4-yl) (3-nitrophényl)méthylène]-2,5-cyclohexadièn-1-ylidène]-N-méthyl-,chlorure Méthanaminium, N-[4-[(3-bromphényl)(2,3-dihydro-1,5-diméthyl-3-oxo-2-phényl-1H-pyrazol-4-yl)méthylène]-2,5-cyclohexadièn-1-ylidène]-N-méthyl-,chlorure Méthanaminium, N-[4-[(2,3-dihydro-1,5-diméthyl-3-oxo-2-phényl-1H-pyrazol-4-yl) (3-méthoxyphényl)méthylène]-2,5-cyclohexadièn-1-ylidène]-N-méthyl-,chlorure Méthanaminium, N-[4-[(2,3-dihydro-1,5-diméthyl-3-oxo-2-phényl-1H-pyrazol-4-yl)phénylméthylène]-2,5-cyclohexadièn-1-ylidène]-N-méthyl-, chlorure [[4-[(1-méthyl-2-phényl-1H-indol-3-yl)[4-[(sulfophényl)amino]phényl]méthylène] -2,5-cyclohexadièn-1-ylidène]amino]-Méthanaminium, N-[4-[(2,3-dihydro-1,5-diméthyl-3-oxo-2-phényl-1H-pyrazol-4-yl)[4-(diméthylamino)phényl]méthylène]-2,5-cyclohexadièn-1-ylidène]-N-méthyl-, chlorure Benzènamine, 4-[(2-[1,1'-biphényl]-4-yl-1-butyl-1H-indol-3-yl)[4-[(4-éthoxyphényl)imino]-2,5-cyclohexadièn-1-ylidèneméthyl]-N-(4-éthoxyphényl)-Benzènaminium, N-méthyl-N-[4-[[4-(méthylphénylamino)phényl] (4-oxo-4H-1-benzopyran-3-yl)méthylène]-2,5-cyclohexadièn-1-ylidène]-Éthanaminium, N-[4-[[4-(diéthylamino)phényl](4-oxo-4H-1-benzopyran-3-yl)méthylène]-2,5-cyclohexadièn-1-ylidène]-N-éthyl-Benzènaminium, 4-éthoxy-N-méthyl-N-[4-[(1-méthyl-2-phényl-1H-indol-3-yl)phénylméthylène]-2,5-cyclohexadièn-1-ylidène]-, chlorure Méthanaminium, N-[4-[12H-benzo[a]phénoxazin-5-yl[4-(diméthylamino)phényl]méthylène]-2,5-cyclohexadièn-1-ylidène]-N-méthyl-, chlorure Benzènaminium, N-méthyl-N-[4-[(1-méthyl-2-phényl-1H-indol-3-yl)phénylméthylène]-2,5-cyclohexadièn-1-ylidène]-Éthanaminium, N-[4-[[4-(diéthylamino)phényl](7-méthoxy-2,1,3-benzothiadiazol-4-yl)méthylène]-2,5-cyclohexadièn-1-ylidène]-N-éthyl-Éthanaminium, N-[4-[[4-(diéthylamino)phényl](5-méthoxy-2,1,3-benzothiadiazol-4-yl)méthylène]-2,5-cyclohexadièn-1-ylidène]-N-éthyl-Éthanaminium, N-[4-[[4-(diéthylamino)phényl](7-méthyl-2,1,3-benzothiadiazol-4-yl)méthylène]-2,5-cyclohexadièn-1-ylidène]-N-éthyl-Éthanaminium, N-[4-[2,1,3-benzothiadiazol-5-yl[4-(diéthylamino)phényl]méthylène]-2,5-cyclohexadièn-1-ylidène]-N-éthyl-Éthanaminium, N-[4-[2,1,3-benzothiadiazol-4-yl[4-(diéthylamino)phényl]méthylène]-2,5-cyclohexadièn-1-ylidène]-N-éthyl-Méthanaminium, N-méthyl-N-[4-[(1-méthyl-2-phényl-1H-indol-3-yl)phénylméthylène]-2,5-cyclohexadièn-1-ylidène]-, chlorure Méthanaminium, N-méthyl-N-[4-[(1-méthyl-2-phényl-1H-indol-3-yl)phénylméthylène]-2,5-cyclohexadièn-1-ylidène]-Méthanaminium, N-[4-[[4-(diméthylamino)phényl](7-méthoxy-2,1,3-benzothiadiazol-4-yl)méthylène]-2,5-cyclohexadièn-1-ylidène]-N-méthyl-Méthanaminium, N-[4-[[4-(diméthylamino)phényl](5-méthoxy-2,1,3-benzothiadiazol-4-yl)méthylène]-2,5-cyclohexadièn-1-ylidène]-N-méthyl-Méthanaminium, N-[4-[[4-(diméthylamino)phényl](7-méthyl-2,1,3-benzothiadiazol-4-yl)méthylène]-2,5-cyclohexadièn-1-ylidène]-N-méthyl-Méthanaminium, N-[4-[2,1,3-benzothiadiazol-5-yl [4-(diméthylamino)phényl]méthylène]-2,5-cyclohexadièn-1-ylidène]-N-méthyl-Méthanaminium, N-[4-[2,1,3-benzothiadiazol-4-yl[4-(diméthylamino)phényl]méthylène]-2,5-cyclohexadièn-1-ylidène]-N-méthyl-Méthanaminium, N-[4-[[4-(diméthylamino)phényl]-3-pyridinylméthylène]-2,5-cyclohexadièn-1-ylidène]-N-méthyl-Méthanaminium, N-[4-[[4-(diméthylamino)phényl]-4-pyridinylméthylène]-2,5-cyclohexadièn-1-ylidène]-N-méthyl-Méthanaminium, N-[4-[[4-(diméthylamino)phényl]-2-thiènylméthylène]-2,5-cyclohexadièn-1-ylidène]-N-méthyl-Méthanaminium, N-[4-[[4-(diméthylamino)phényl]-2-furanylméthylène]-2,5-cyclohexadièn-1-ylidène]-N-méthyl-Méthanaminium, N-[4-[[4-(diméthylamino)phényl]-3-thiènylméthylène]-2,5-cyclohexadièn-1-ylidène]-N-méthyl-[[4-[(1-méthyl-2-phényl-1H-indol-3-yl)[4-[(sulfophényl)amino]phényl]méthylène]-2,5-cyclohexadièn-1-ylidène]amino]-, Ethoxy[[4-[[4-[(éthoxysulfophényl)amino]phényl](1-méthyl-2-phényl-1H-indol-3-yl)méthylène]-2,5-cyclohexadièn-1-ylidène]amino]-Benzènamine, N-méthyl-4-[[4- (méthylimino)-2,5-cyclohexadièn-1-ylidène](2-méthyl-1H-indol-3-yl)méthyl]-, Méthanaminium, N-[4-[4-(diméthylamino)phényl](2-méthyl-1H-indol-3-yl)méthylène]-2,5-cyclohexadièn-1-ylidène]-N-méthyl-Benzènamine, N-méthyl-4-[[4-(méthylimino)-2,5-cyclohexadièn-1-ylidène](1-méthyl-2-phényl-1H-indol-3-yl)méthyl]-, monoacide conjugué Méthanaminium, N-[4-[[4-(diméthylamino)phényl](1-méthyl-2-phényl-1H-indol-3-yl)méthylène]-2,5-cyclohexadièn-1-ylidène]-N-méthyl-Benzènamine, 4-éthoxy-N-[4-[[4-[(4-éthoxyphényl)amino]phényl](2-phényl-1H-indol-3-yl)méthylène]-2,5-cyclohexadièn-1-ylidène]-, Benzènamine, 4-éthoxy-N-[4-[[4-[(4-éthoxyphényl)amino]phényl](1-méthyl-1H-indol-3-yl)méthylène]-2,5-cyclohexadièn-1-ylidène]-, Benzènamine, N-[4-[(1,2-diméthyl-1H-indol-3-yl)[4-[(4-éthoxyphényl)amino]phényl]méthylène]-2,5-cyclohexadièn-1-ylidène]-4-éthoxy-Benzènamine, 4-[(1-méthyl-2-phényl-1H-indol-3-yl)[4-(phénylimino)-2,5-cyclohexadièn-1-ylidène]méthyl]-N-phényl-, monoacide conjugué Benzènamine, 4-éthoxy-N-[4-[[4-[(4-éthoxyphényl)amino]phényl](1-méthyl-2-phényl-1H-indol-3-yl)méthylène]-2,5-cyclohexadièn-1-ylidène]-, Méthanaminium, N-[4-[[4-(diméthylamino)phényl](2-phényl-1H-indol-3-yl)méthylène]-2,5-cyclohexadièn-1-ylidène]-N-méthyl-Benzènaminium, N-méthyl-N-[4-[[4-(méthylphénylamino)phényl](4-oxo-4H-1-benzopyran-3-yl)méthylène]-2,5-cyclohexadièn-1-ylidène]-, chlorure Benzènaminium, N-méthyl-N-[4-[[4-(méthylphénylamino)phényl](4-oxo-4H-1-benzopyran-3-yl)méthylène]-2,5-cyclohexadièn-1-ylidène]-, perchlorate Éthanaminium, N-[4-[[4-(diéthylamino)phényl](4-oxo-4H-1-benzopyran-3-yl)méthylène]-2,5-cyclohexadièn-1-ylidène]-N-éthyl-, perchlorate Éthanaminium, N-[4-[[4-(diéthylamino)phényl](4-oxo-4H-1-benzopyran-3-yl)méthylène]-2,5-cyclohexadièn-1-ylidène]-N-éthyl-, chlorure Benzènaminium, N-méthyl-N-[4-[[4-(méthylphénylamino)phényl(4-oxo-4H-1-benzopyran-3-yl)méthylène]-2,5-cyclohexadièn-1-ylidène]-, acétate Éthanaminium, N-[4-[[4-(diéthylamino)phényl](4-oxo-4H-1-benzopyran-3-yl)méthylène]-2,5-cyclohexadièn-1-ylidène]-N-éthyl-, acétate Méthanaminium, N-[4-[(2,3-dihydro-1,5-diméthyl-3-oxo-2-phényl-1H-pyrazol-4-yl)(3-nitrophényl)méthylène]-2,5-cyclohexadièn-1-ylidène]-N-méthyl-Méthanaminium, N-[4-[(3-bromophényl)(2,3-dihydro-1,5-diméthyl-3-oxo-2-phényl-1H-pyrazol-4-yl)méthylène]-2,5-cyclohexadièn-1-ylidène]-N-méthyl-Méthanaminium, N-[4-[(2,3-dihydro-1,5-diméthyl-3-oxo-2-phényl-1H-pyrazol-4-yl)(3-méthoxyphényl)méthylène]-2,5-cyclohexadièn-1-ylidène]-N-méthyl-Méthanaminium, N-[4-[(2,3-dihydro-1,5-diméthyl-3-oxo-2-phényl-1H-pyrazol-4-yl)phénylméthylène]-2,5-cyclohexadièn-1-ylidène]-N-méthyl-Méthanaminium, N-[4-[(2,3-dihydro-1,5-diméthyl-3-oxo-2-phényl-1H-pyrazol-4-yl)(3-méthylphényl)méthylène]-2,5-cyclohexadièn-1-ylidène]-N-méthyl-Benzènamine, N-[4-[(1-éthyl-2-phényl-1H-indol-3-yl)[4-[(2-méthoxyphényl)amino]phényl]méthylène]-2,5-cyclohexadièn-1-ylidène]-2-méthoxy-, monochlorhydrate 2-hydroxy-5-[[4-[[4-[(4-hydroxy-3-sulfophényl)amino]phényl](1-méthyl-2-phényl-1H-indol-3-yl)méthylène]-2,5-cyclohexadièn-1-ylidène]amino]-Benzènamine, 2-éthoxy-N-[4-[[4-[(2-éthoxyphényl)amino]phényl](1-méthyl-2-phényl-1H-indol-3-yl)méthylène]-2,5-cyclohexadièn-1-ylidène]-, monochlorhydrate Méthanaminium, N-[4-[[4-(diméthylamino)phényl]-2-furanylméthylène]-2,5-cyclohexadièn-1-ylidène]-N-méthyl-, perchlorate 2-éthoxy-5-[[4-[[4-[(4-éthoxy-3-sulfophényl)amino]phényl](1-méthyl-2-phényl-1H-indol-3-yl)méthyl]-2,5-cyclohexadièn-1-ylidène]amino]-Ammonium, [4-[α-[p-(diméthylamino)phényl]furfurylidène]-2,5-cyclohexadièn-1-ylidène]diméthyl-, tétrachlorogallate(1-) Méthanaminium, N-[4-[[4-(diméthylamino)phényl]-2-thiènylméthyl]-2,5-cyclohexadièn-1-ylidène]-N-méthyl-, perchlorate Ammonium, [4-[p-(diméthylamino)-α-2-thiènylbenzylidène]-2,5-cyclohexadièn-1-ylidène]diméthyl-, perchlorate Ammonium, (2-cyanoéthyl)[4-[α-[4-[(2-cyanoéthyl)éthylamino]-o-tolyl]pipéronylidène]-3-méthyl-2,5-cyclohexadièn-1-ylidène]éthyl-Ammonium, diméthyl[4-[α-(1-méthyl-2-phénylindol-3-yl)benzylidène]-2,5-cyclohexadièn-1-ylidène]-, chlorure, Ammonium, (p-éthoxyphényl)méthyl[4-[α-(1-méthyl-2-phénylindol-3-yl)benzylidène]-2,5-cyclohexadièn-1-ylidène]-, chlorure, (p-éthoxyphényl)méthyl[4-[α-(1-méthyl-2-phénylindol-3-yl)benzylidène]-2,5-cyclohexadièn-1-ylidène]ammonium chlorure Ammonium, [4-[α-2,1,3-benzothiadiazol-4-yl-p- (diméthylamino)benzylidène]-2,5-cyclohexadièn-1-ylidène]diméthyl-,perchlorate Ammonium, [4-[p-(diméthylamino)-α-(5-méthoxy-2,1,3-benzothiadiazol-4-yl)benzylidène]-2,5-cyclohexadièn-1-ylidène]diméthyl-, perchlorate Ammonium, [4-[p-(diéthylamino)-α-(5-méthoxy-2,1,3-benzothiadiazol-4-yl)benzylidène]-2,5-cyclohexadièn-1-ylidène]diéthyl-, perchlorate Ammonium, [4-[p-(diéthylamino)-α-(7-méthoxy-2,1,3-benzothiadiazol-4-yl)benzylidène]-2,5-cyclohexadièn-1-ylidène]diéthyl-, perchlorate Ammonium, [4-[p-(diméthylamino)-α-(7-méthoxy-2,1,3-benzothiadiazol-4-yl)benzylidène]-2,5-cyclohexadièn-1-ylidène]diméthyl-, perchlorate Ammonium,[4-[p-(diéthylamino)-α-(7-méthyl-2,1,3-benzothiadiazol-4-yl)benzylidène]-2,5-cyclohexadièn-1-ylidène]diéthyl-, perchlorate Ammonium, [4-[p-(diméthylamino)-α-(7-méthyl-2,1,3-benzothiadiazol-4-yl)benzylidéne]-2,5-cyclohexadièn-1-ylidène]diméthyl-, perchlorate Ammonium, [4-[α-2,1,3-benzothiadiazol-4-yl-p-(diéthylamino)benzylidène]-2,5-cyclohexadièn-1-ylidène]diéthyl-,perchlorate Ammonium, [4-[α-2,1,3-benzothiadiazol-5-yl-p-(diéthylamino)benzylidéne]-2,5-cyclohexadièn-1-ylidène]diéthyl-,perchlorate Ammonium, [4-[α-2,1,3-benzothiadiazol-5-yl-p-(diméthylamino)benzylidène]-2,5-cyclohexadièn-1-ylidène]diméthyl-,perchlorate Ammonium, méthyl[4-[α-(1-méthyl-2-phénylindol-3-yl)benzylidène]-2,5-cyclohexadièn-1-ylidène]phényl-, trichlorozincate(1-) Trichlorozincate de-, méthyl[4-[α-(1-méthyl-2-phénylindol-3-yl)benzylidène]-2,5-cyclohexadièn-1-ylidène]phénylammonium Trichlorozincate d'Ammonium, diméthyl[4-[α-(1-méthyl-2-phénylindol-3-yl)benzylidène]- 2,5-cyclohexadièn-1-ylidène]-, Trichlorozincate de diméthyl[4-[α-(1-méthyl-2-phénylindol-3-yl)benzylidène]-2,5-cyclohexadièn-1-ylidène]ammonium Indole, 3-[α-(4-imino-2,5-cyclohexadièn-1-ylidène)benzyl]-1,2-diméthyl-, monochlorhydrate Indole, 3-[α-[4-[(p-éthoxyphényl)imino]-2,5-cyclohexadièn-1-ylidène]benzyl]-1-méthyl-2-phényl-, monochlorhydrate Ammonium, [4-[α-[9-(2-cyanoéthyl)carbazol-3-yl]-p- (diméthylamino)benzylidène]-2,5-cyclohexadièn-1-ylidène]diméthylAmmonium, [4-[α-carbazol-3-yl-p-(diméthylamino)benzylidène]-2,5-cyclohexadièn-1-ylidène]diméthylAmmonium, [4-[α-[9-(2-cyanoéthyl)carbazol-3-yl]benzylidène]-2,5-cyclohexadièn-1-ylidène]diméthylAmmonium, [4-(α-carbazol-3-ylbenzylidène)-2,5-cyclohexadièn-1-ylidène]diméthyl-2,5-Cyclohexadièn-1-one, 2,6-di-tert-butyl-4-(α-morpholinobenzylidène)-2,5-Cyclohexadièn-1-one, 2,6-di-tert-butyl-4-[α-(2,2-diméthyl-1-aziridinyl)benzylidène]-2,5-Cyclohexadièn-1-one, 4-(α-1-aziridinylbenzylidène)-2,6-di-tert-butyl-2,5-Cyclohexadièn-1-one, 2,6-bis(1,1-diméthyléthyl)-4-(phényl-1-pipéridinylméthylène)-2,5-Cyclohexadièn-1-one, 2,6-di-tert-butyl-4-(α-pipéridinobenzylidène)-Ammonium, [4-[α-[4-(diéthylamino)-o-tolyl]pipéronyl]-3-méthyl-2,5-cyclohexadièn-1-ylidène]diéthyl-, chlorure Ammonium, [4-[α-[4-(diéthylamino)-o-tolyl]-2-thénylidène]-3-méthyl-2,5-cyclohexadièn-1-ylidène]diéthyl-, chlorure Ammonium, [4-[α-[p-(diméthylamino)phényl]-5-nitrofurfurylidène]-2,5-cyclohexadièn-1-ylidène]diméthyl-, chlorure Ammonium, [4-[5-bromo-α-[p-(diéthylamino)phényl]furfurylidène]-2,5-cyclohexadièn-1-ylidène]diéthyl-, chlorure Ammonium, [4-[5-bromo-α-[p-(diméthylamino)phényl]furfurylidène]-2,5-cyclohexadièn-1-ylidène]diméthyl-, chlorure Ammonium, [4-[α-[p-(diméthylamino)phényl]-2-furfurylidène]-2,5-cyclohexadièn-1-ylidène]diéthyl-, chlorure 2-hydroxy-5-[[4-[[4-[(4-hydroxy-3-sulfophényl)amino]phényl](1-méthyl-2-phényl-1H-indol-3-yl)méthylène]-2,5-cyclohexadièn-1-ylidène]amino]-, Ammonium, [4-[α-12H-benzo[a]phénoxazin-5-yl-p- (diméthylamino)benzylidène]-2,5-cyclohexadièn-1-ylidène]diméthyl-,chlorure, composé avec le chlorure de zinc [4-[α-12H-benzo[a]phénoxazin-5-yl-p-(diméthylamino)benzylidène]-2,5-cyclohexadièn-1-ylidène]diméthylammonium chlorure Benzènamine, N,2-diméthyl-4-[(2-méthyl-1H-indol-3-yl)[3-méthyl-4-(méthylimino)-2,5-cyclohexadièn-1-ylidène]méthyl]-, monochlorhydrate Indole, 2-méthyl-3-[3-méthyl-4-(méthylamino)-α-[3-méthyl-4-(méthylimino)-2,5-cyclohexadièn-1-ylidène]benzyl]-, monochlorhydrate Méthanaminium, N-[4-[[4-(diméthylamino)phényl]-2-furanylméthylène]-2,5-cyclohexadièn-1-ylidène]-N-méthyl-, chlorure Ammonium, [4-[α-[p-(diméthylamino)phényl]furfurylidène]-2,5-cyclohexadièn-1-ylidène]diméthyl-, chlorure Benzènaminium, N-[4-[[2-(4-chlorophényl)-4,6-diméthyl-1-(2-méthylpropyl)-1H-indol-3-yl](2,4-disulfophényl)méthylène]-2,5-cyclohexadièn-1-ylidène]-4-méthoxy-N-méthyl-, Ammonium, [4-[α-[2-(p-chlorophényl)-1-isobutyl-4,6-diméthylindol-3-yl]-2,4-disulfobenzylidène]-2,5-cyclohexadièn-1-ylidène](p-méthoxyphényl)méthyl-, hydroxyde, 2-éthoxy-5-[[4-[[4-[(4-éthoxy-3-sulfophényl)amino]phényl](1-méthyl-2-phényl-1H-indol-3-yl)méthylène]-2,5-cyclohexadièn-1-ylidène]amino]-, 5-[(3-carboxy-5-méthyl-4-oxo-2,5-cyclohexadièn-1-ylidène)(4,5-dihydro-3-méthyl-5-oxo-1-phényl-1H-pyrazol-4-yl)méthyl]-2-hydroxy-3-méthyl-5-[[4-[[2-(4-chlorophényl)-4,6-diméthyl-1-(2-méthylpropyl)-1H-indol-3-yl][4-[(4-éthoxy-3-sulfophényl)amino]phényl]méthylène]-2,5-cyclohexadièn-1-ylidène]amino]-2-éthoxy-, N-[α-[2-(p-chlorophényl)-1-isobutyl-4,6-diméthylindol-3-yl]-α-[4-[[(4-éthoxy-3-sulfophényl)imino]-2,5-cyclohexadièn-1-ylidène]-p-tolyl]-6-éthoxy-, 6-(4-éthoxyphényl)imino]-3-[(1-méthyl-2-phényl-1H-indol-3-yl)[4-[(2-sulfoéthyl)amino]phényl]méthylène]-, 6-[(p-éthoxyphényl)imino]-3-[α-(1-méthyl-2-phénylindol-3-yl)-p-[(2-sulfoéthyl)amino]benzylidène]-, Méthanaminium, N-[4-[[4-(diméthylamino)phényl]-3-thiènylméthylène]-2,5-cyclohexadièn-1-ylidène]-N-méthyl-, perchlorate [4-[α[p-(Diméthylamino)phényl]-3-thénylidène]-2,5-cyclohexadièn-1-ylidène]diméthylammonium perchlorate Ammonium, 4-[α-[p-(diméthylamino)phényl]furfurylidène]-2,5-cyclohexadièn-1-ylidène]diméthyl-, bromure [4-[α-[p-(Diméthylamino)phényl]furfurylidène]-2,5-cyclohexadièn-1-ylidène]diméthylammonium bromure Méthanaminium, N-[4-[[4-(diméthylamino)phényl]-3-pyridinylméthylène]-2,5-cyclohexadièn-1-ylidène]-N-méthyl-, perchlorate Ammonium, [4-[p-(diméthylamino)-α-3-pyridylbenzylidène]-2,5-cyclohexadièn-1-ylidène]diméthyl-, perchlorate [4-[p-(Diméthylamino)-α-3-pyridylbenzylidène]-2,5-cyclohexadièn-1-ylidène]diméthylammonium perchlorate Ammonium, [4-[p-(diméthylamino)-α-4-pyridylbenzylidène]-2,5-cyclohexadièn-1-ylidène]diméthyl-, perchlorate [4-[p-(Diméthylamino)-α-4-pyridylbenzylidène]-2,5-cyclohexadièn-1-ylidène]diméthylammonium perchlorate Ammonium, [4-[p-(diméthylamino)-α-2-pyridylbenzylidène]-2,5-cyclohexadièn-1-ylidène]diméthyl-, chlorure [4-[p-(Diméthylamino)-α-2-pyridylbenzylidène]-2,5-cyclohexadièn-1-ylidène]diméthylammonium chlorure,
et leurs sels d'addition.

6. Composition selon l'une quelconque des revendications précédentes, **caractérisée en ce que** le ou les composé(s) de formule (I), (Ibis), (II) ou (IIbis) et/ou le ou les sel(s) d'addition représentent de 0,0001 à 10 % en poids du poids total de la composition de teinture, de préférence de 0,005 à 10 % en poids et, de préférence encore, de 0,01 à 6 % en poids du poids total de la composition de teinture.

7. Composition selon l'une quelconque des revendications 1 à 6, **caractérisée en ce qu'**elle contient, en outre, un ou plusieurs colorant(s) direct(s) différent(s) des composés de formules (I), (Ibis), (II) et (IIbis).

8. Composition selon la revendication 7, **caractérisée en ce que** le ou les colorant(s) direct(s) différent(s) des composés de formule (I), (Ibis), (II) ou (IIbis) sont choisis parmi les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

9. Composition selon l'une quelconque des revendications 7 et 8, **caractérisée en ce que** le ou les colorant(s) direct(s) différents des composés de formules (I), (Ibis), (II) ou (IIbis) représentent de 0,001 à 20 % en poids, de préférence de 0,005 à 10 % en poids du poids total de la composition de teinture.

10. Composition selon l'une quelconque des revendications précédentes, **caractérisée en ce qu'**elle contient, en outre, une ou plusieurs base(s) d'oxydation et éventuellement un ou plusieurs coupleur(s).

11. Composition selon la revendication 10, **caractérisé en ce que** la ou les base(s) d'oxydation sont choisies parmi les paraphénylènediamines, les bis-phénylalkylènediamines, les para-aminophénols, les ortho-aminophénols, les bases hétérocycliques et leurs sels d'addition.

12. Composition selon la revendication 10, **caractérisée en ce que** le ou les coupleur(s) sont choisi(s) parmi les coupleurs métaphénylènediamines, les coupleurs méta-aminophénols, les coupleurs métadiphénols, les coupleurs naphtaléniques, les coupleurs hétérocycliques et leurs sels d'addition.

13. Composition selon l'une quelconque des revendications 10 à 12, **caractérisée en ce que** le ou les base(s) d'oxydation et/ou le ou les coupleur(s) sont présents chacun en une quantité de 0,001 à 10 % en poids, de préférence de 0,005 à 6 % en poids du poids total de la composition de teinture.

14. Composition selon l'une quelconque des revendications précédentes, **caractérisée en ce qu'**elle a un pH de 3 à 12, de préférence de 5 à 11, de préférence encore de 6 à 8,5.

15. Procédé de teinture directe des fibres kératiniques et en particulier des fibres kératiniques humaines, telles que les cheveux, comprenant l'application d'au moins une composition de teinture selon l'une quelconque des revendications 1 à 14 contenant au moins un composé de formule (I), (Ibis), (II) ou (IIbis) sur les fibres kératiniques puis l'observation d'un temps de pause suivi du rinçage des fibres.

16. Procédé selon la revendication 15, **caractérisé en ce que** le temps de pause est de 3 à 50 minutes, de préférence de 5 à 30 minutes.

17. Procédé de teinture des fibres kératiniques et, en particulier, des fibres kératiniques humaines, telles que les cheveux, dans lequel on applique sur lesdites fibres au moins une composition de teinture comprenant soit au moins un composé de formule (II) ou (IIbis), soit au moins un composé de formule (I) ou (Ibis) et au moins une base d'oxydation et éventuellement un ou plusieurs coupleur(s), la couleur étant révélée à pH acide, neutre ou alcalin, à l'aide d'un agent oxydant.

18. Procédé selon la revendication 17, **caractérisé en ce que** l'agent oxydant est choisi parmi le peroxyde d'hydrogène, le peroxyde d'urée, les bromates de métaux alcalins, les persels tels que les perborates et persulfates, les peracides et les enzymes oxydases, tel que les peroxydases, les oxydo-réducteurs à 2 électrons, comme les unicases et les oxydénases à 4 électrons, comme les laccases.

19. Dispositif à plusieurs compartiments comprenant un premier compartiment renfermant une composition tinctoriale selon l'une quelconque des revendications 1 à 14, comprenant soit au moins un composé de formule (II) ou (IIbis), soit au moins un composé de formule (I) ou (Ibis) et au moins une base d'oxydation et éventuellement un ou plusieurs coupleurs, et un deuxième compartiment renfermant une composition oxydante.

## Claims

1. A composition for dyeing keratinous fibres comprising in a cosmetic medium suitable for dyeing, at least one dye selected from the compounds of the following formulae (I), (Ibis), (II) or (IIbis) or their tautomeric forms, and addition salts thereof: 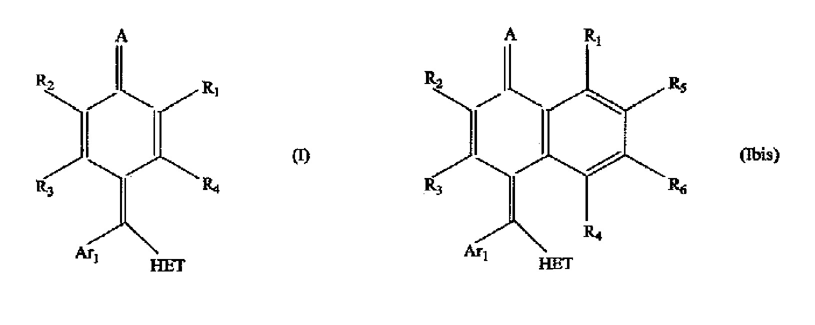 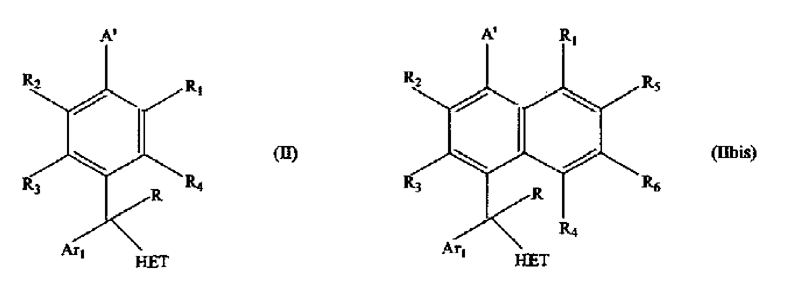 wherein:
- Ar₁ is an aryl radical, such as phenyl or naphthyl, possibly substituted by one or more Z groups;
- HET is an heterocycle possibly substituted by one or more Z' groups;
- A is 0; NH; N-alkyl; N-hydroxalkyl; ammonium, N-alkylammonium, N-hydroxyalkylammonium, N,N-dialkylammonium, N,N-di(hydroxyalkyl)ammonium or N-(hydroxyalkyl)N-(alkyl)ammonium, wherein both alkyl groups may form together with the nitrogen atom to which they are attached a ring which can be interrupted by one or more nitrogen, oxygen or sulphur atoms;
- R₁ to R₆ and Z, Z' are independently a group selected from hydrogen atom, halogen atoms, such as F, Cl, Br and I, -NHSO₃H; hydroxyl; alkyl; alkoxy; alkylthio; monoalkylamino; dialkylamino radicals, wherein both alkyl groups may, taken together with the nitrogen atom to which they are attached, form a ring which can be interrupted by one or more nitrogen, oxygen or sulphur atoms; heterocycles and nitro; aryl; acyl; alkoxycarbonyl; carboxamido; cyano; -CO₂H; -SO₃H; -PO₃H₂; -PO₄H₂ radicals;
wherein:
- A' is a hydrogen atom; a hydroxyl; amino; (hydroxyalkyl)amino; monoalkylamino; di(hydroxyalkyl)amino; (hydroxyalkyl)(alkyl)amino; (dialkyl)amino group, wherein both alkyl groups may form, together with the nitrogen atom to which they are attached, a ring which can be interrupted by one or more nitrogen, oxygen or sulphur atoms;
- R is a hydrogen or halogen atom; a hydroxyl radical; an alkoxy radical; or an alkylthio radical, except for bis-(4-hydroxyphenyl)-(quinolyl-2)methane hydrochloride and bis-(4-hydroxyphenyl)-(quinolyl-2)methane.

2. The composition according to claim 1, wherein HET is an unsaturated heterocycle, possibly substituted by one or more Z' groups.

3. The composition according to claim 2, wherein HET is an aromatic heterocycle, possibly substituted by one or more Z' groups.

4. The composition according to any one of claims 1 to 3, wherein the heterocycles, particularly those of HET, are selected from the following heterocycles: thiophene, benzothiophene, furan, benzofuran, indole, indoline, carbazole, pyridine, dehydroquinoline, chromone, julodinine, thiadiazole, triazole, isoxazole, oxazole, thiazole, isothiazole, imidazole, pyrazole, triazine, thiazine, pyrazine, pyridazine, pyrimidine, pyridine, diazepine, oxazepine, benzotriazole, benzoxazole, benzimidazole, benzothiazole, morpholine, piperidine, piperazine, azetidine, pyrrolidine and aziridine.

5. The dyeing composition according to claim 1, **characterised in that** the composition includes a compound of formula (I), (Ibis), (II) or (IIbis) selected from the following compounds for which the counter-ions are specified or not:
Methanaminium, N-[4-[[4-(dimethylamino)phenyl](2,2-dimethyl-2H-1-benzopyran-3-yl)methylene]-2,5-cyclohexadien-1-ylidene]-N-methyl-, acetate Methanaminium, N-[4-[[4-(dimethylamino)phenyl](2,2-dimethyl-2H-1-benzopyran-3-yl)methylene]-2,5-cyclohexadien-1-ylidene]-N-methyl-Methanaminium, N-[4-[[4-(dimethylamino)phenyl](2,2-dimethyl-2H-1-benzopyran-5-yl)methylene]-2,5-cyclohexadien-1-ylidene]-N-methyl-, acetate Methanaminium, N-[4-[[4-(dimethylamino)phenyl](2,2-dimethyl-2H-1-benzopyran-5-yl)methylene]-2,5-cyclohexadien-1-ylidene]-N-methyl-Methananinium, N-[4-[[4-(dimethylamino)phenyl](2,2-dimethyl-2H-1-benzopyran-4-yl)methylene]-2,5-cyclohexadien-1-ylidene]-N-methyl-, acetate Methanaminium, N-[4-[[4-(dimethylamino)phenyl](2,2-dimethyl-2H-1-benzopyran-4-yl)methylene]-2,5-cyclohexadien-1-ylidene]-N-methyl-Methanaminium, N-[4-[[4-(dimethylamino)phenyl](2,2-dimethyl-2H-1-benzopyran-8-yl)methylene]-2,5-cyclohexadien-1-ylidene]-N-methyl-, acetate Methanaminium, N-[4-[[4-(dimethylamino)phenyl](2,2-dimethyl-2H-1-benzopyran-8-yl)methylene]-2,5-cyclohexadien-1-ylidene]-N-methyl-Methanaminium, N-[4-[[4-(dimethylamino)phenyl](2,2-dimethyl-2H-1-benzopyran-7-yl)methylene]-2,5-cyclohexadien-1-ylidene]-N-methyl-, acetate Methanaminium, N-[4-[[4-(dimethylamino)phenyl](2,2-dimethyl-2H-1-benzopyran-7-yl)methylene]-2,5-cyclohexadien-1-ylidene]-N-methyl-Methanaminium, N-[4-[[4-(dimethylamino)phenyl](2,2-dimethyl-2H-1-benzopyran-6-yl)methylene]-2,5-cyclohexadien-1-ylidene]-N-methyl-, acetate Methanaminium, N-[4-[[4-(dimethylamino)phenyl](2,2-dimethyl-2H-1-benzopyran-6-yl)methylene]-2,5-cyclohexadien-1-ylidene]-N-methyl-2,5-Cyclohexadien-1-one, 4-[[4-amino-3,5-bis(1-methylethyl)phenyl](4-ethyl-3,4-dihydro-2H-1,4-benzoxazin-7-yl)methylene]-2,6-dimethoxy 2,5-Cyclohexadien-1-one, 4-[[3,4-dihydro-4-(2-pyridinylmethyl)-2H-1,4-benzoxazin-7-yl](4-ethyl-3,4-dihydro-2H-1,4-benzoxazin-7-yl)methylene]-2,6-dimethoxy-2,5-Cyclohexadien-1-one, 4-[[4-(dimethylamino)phenyl](4-ethyl-3,4-dihydro-2H-1,4-benzoxazin-7-yl)methylene]-2,6-dimethoxy-2,5-Cyclohexadien-1-one, 2,6-bis(1,1-dimethylethyl)-4-[(5-ethyl-2-thienyl)phenylmethylene]-Ethanaminium, N-[3-carboxy-4-[[2-carboxy-4-[ethyl(2-methoxyethyl)amino]phenyl](7-carboxy-4-oxo-4H-1-benzothiopyran-3-yl)methylene]-2,5-cyclohexadien-1-ylidene]-N-ethyl-2-methoxy Ethanaminium, N-[4-[[4-[bis(2-methoxyethyl)amino]-2-carboxyphenyl](5-carboxy-2-pyridinyl)methylene]-3-carboxy-2,5-cyclohexadien-1-ylidene]-2-methoxy-N-(2-methoxyethyl)-, Ethanaminium, N-[4-[3-benzofuranyl[2-carboxy-4-[ethyl[2-[(methylsulphonyl)amino]ethyl]amino]phenyl]-methylene]-3-carboxy-2,5-cyclohexadien-1-ylidene]-N-ethyl-2-[(methylsulphonyl)amino]-, perchlorate Ethanaminium, N-[4-[3-benzofuranyl[2-carboxy-4-[ethyl[2-((methylsulphonyl)amino]ethyl]amino]phenyl]-methylene]-3-carboxy-2,5-cyclohexadien-1-ylidene]-N-ethyl-2-[(methylsulphonyl)amino]-Ethanaminium, N-[4-[[4-[bis(2-methoxyethyl)amino]-2-carboxyphenyl](4-oxo-4H-1-benzothiopyran-3-yl)-methylene]-3-carboxy-2,5-cyclohexadien-1-ylidene]-2-methoxy-N-(2 methoxyethyl)-, acetate Ethanaminium, N-[4-[[4-[bis(2-methoxyethyl)amino]-2-carboxyphenyl](4-oxo-4H-1-benzothiopyran-3-yl)-methylene]-3-carboxy-2,5-cyclohexadien-1-ylidene]-2-methoxy-N-(2-methoxyethyl)-Benzoic acid and 5-[(3-carboxy-4-oxo-2,5-cyclohexadien-1-ylidene)-3-pyridinylmethyl]-2-hydroxy-salt Methanaminium, N-[2-carboxy-4-[[3-carboxy-4-(dimethylamino)phenyl](4-oxo-4H-1-benzothiopyran-3-yl)methylene]-2,5-cyclohexadien-1-ylidene]-N-methyl-, perchlorate Methanaminium, N-[2-carboxy-4-[[3-carboxy-4-(dimethylamino)phenyl](4-oxo-4H-1-benzothiopyran-3-yl)methylene]-2,5-cyclohexadien-1-ylidene]-N-methyl-, Benzoic acid and 5-[(3-carboxy-4-oxo-2,5-cyclohexadien-1-ylidene)[3,5-dimethyl-1-[2-[(methylsulphonyl)amino]ethyl]-1H-pyrazol-4-yl]methyl]-2-hydroxy- salt Benzoic acid and 5-[[2-[bis(2-methoxyethyl)amino]-5-thiazolyl](3-carboxy-4-oxo-2,5-cyclohexadien-1-ylidene)methyl]-2-hydroxy- salt Ethanaminium, N-[4-[(7-carboxy-2-oxo-2H-1-benzopyran-4-yl)[4-(diethylamino)phenyl]methylene]-2,5-cyclohexadien-1-ylidene]-N-ethyl-, perchlorate Ethanaminium, N-[4-[(7-carboxy-2-oxo-2H-1-benzapyran-4-yl)[4-(diethylamino)phenyl]methylene]-2,5-cyclohexadien-1-ylidene]-N-ethylEthanaminium, N-[4-[2-benzofuranyl[4-[ethyl-2-[(methylsulphonyl)amino]ethyl]amino]phenyl]methylene]-2,5-cyclohexadien-1-ylidene]-N-ethyl-2-[(methylsulphonyl)amino]-, perchlorate Ethanaminium, N-[4-[2-benzofuranyl[4-[ethyl[2-[(methylsulphonyl)amino]ethyl]amino]phenyl]methylene]-2,5-cyclohexadien-1-ylidene]-N-ethyl-2-[(methylsulphonyl)amino]-Ethanaminium, N-[4-[(6-carboxybenzo[b]thien-2-yl)[4-(diethylamino)phenyl]methylene]-2,5-cyclohexadien-1-ylidene]-N-ethyl-, tetrafluoroborate (1-) Ethanaminium, N-[4-[(6-carboxybenzo[b]thien-2-yl)[4-(diethylamino)phenyl]methylene]-2,5-cyclohexadien-1-ylidene]-N-ethylEthanaminium, N-[4-[[5-[bis(2-methoxyethyl)amino]-2-furanyl][4-[ethyl[2-[(methylsulphonyl)amino]ethyl]amino]phenyl]methylene]-2,5-cyclohexadien-1-ylidene]-N-ethyl-2-[(methylsulphonyl)amino]-, perchlorate Ethanaminium, N-[4-[[5-[bis(2-methoxyethyl)amino]-2-furanyl][4-[ethyl[2-[(methylsulphonyl)amino]ethyl]amino]phenyl]methylene]-2,5-cyclohexadien-1-ylidene]-N-ethyl-2-[(methylsulphonyl)amino]-2,5-Cyclohexadien-1-one, 4-[(4-hydroxyphenyl)-4-quinolinylmethylene]-Ethanaminium, N-[4-[[4-[bis(2-methoxyethyl)amino]phenyl](7-carboxy-4-oxo-4H-1-benzopyran-3-yl)methylene]-2,5-cyclohexadien-1-ylidene]-2-methoxy-N-(2-methoxyethyl)-, sulphate (1:1) Ethanaminium, N-[4-[[4-[bis(2-methoxyethyl)amino]phenyl](7-carboxy-4-oxo-4H-1-benzopyran-3-yl)methylene]-2,5-cyclohexadien-1-ylidene]-2-methoxy N-(2-methoxyethyl)-Methanaminium, N-[4-[(5-carboxy-2-pyridinyl)[4-(dimethylamino)phenyl]methylene]-2,5-cyclohexadien-1-ylidene]-N-methyl-, perchlorate Methanaminium, N-[4-[(5-carboxy-2-pyridinyl)[4-(dimethylamino)phenyl]methylene]-2,5-cyclohexadien-1-ylidene]-N-methyl-2-Thiophenecarboxylic acid, 5-[(4-hydroxyphenyl)(4-oxo-2,5-cyclohexadien-1-ylidene)methyl]-2,5-Cyclohexadien-1-one, 4-[2-furanyl(4-hydroxyphenyl)methylene]-2,5-Cyclohexadien-1-one, 4-[(4-hydroxyphenyl)-2-pyridinylmethylene]-Methanaminium, N-[3-carboxy-4-[[2-carboxy-4-(dimethylamino)phenyl](4-oxo-4H-1-benzopyran-3-yl)methylene]-2,5-cyclohexadien-1-ylidene]-N-methyl, perchlorate Methanaminium, N-[3-carboxy-4-[[2-carboxy-4-(dimethylamino)phenyl](4-oxo-4H-1-benzopyran-3-yl)methylene]-2,5-cyclohexadien-1-ylidene]-N-methyl-Ethanaminium, N-[4-[(5-carboxy-2-thienyl)[4-(diethylamino)phenyl]methylene]-2,5-cyclohexadien-1-ylidene]-N-ethyl-, perchlorate Ethanaminium, N-[4-[(5-carboxy-2-thienyl)[4-(diethylamino)phenyl]methylene]-2,5-cyclohexadien-1-ylidene]-N-ethyl-2,5-Cyclohexadien-1-one, 4-[(4-methoxyphenyl)-3,6,9,12,15-pentaoxabicyclo[15.3.1]heneicosa-1-(21),17,19-trien-21-ylmethylene]-3,6,9,12,15-Pentaoxabicyclo[15.3.1]heneicosane, 2,5-cyclohexadien-1-one Methanaminium, N-[4-[(2,3-dihydro-3-methyl-2-benzothiazolyl)[4-(dimethylamino)phenyl]methylene]-2,5-cyclohexadien-1-ylidene]-N-methyl-, chloride [4-(α-Benzo[b]thien-2-yl-p-dimethylaminobenzylidene)-2,5-cyclohexadien-1-ylidene] dimethylammonium chloride [4-(α-Benzo[b]thien-3-yl-p-dimethylaminobenzylidene)-2,5-cyclohexadien-1-ylidene]dimethylammonium chloride Methanaminium, N-[4-[[9-(dimethylamino)benzo[a]phenoxazin-7-ium-5-yl][4-(dimethylamino)phenyl]methylene]-2,5-cyclohexadien-1-ylidene]-N-methyl-, bis [tetraphenylborate (1-)] Methanaminium Benzo[a]phenoxazin-7-ium, Methanaminium, N-[4-[[9-(diethylamino)benzo[a]phenoxazin-7-ium-5-yl][4-(dimethylamino)phenyl]methylene]-2,5-cyclohexadien-1-ylidene]-N-methyl-[4-(α-3-Bromobenzo[b]thien-2-yl-p-dimethylaminobenzylidene)-2,5-cyclohexadien-1-ylidene]dimethylammonium chloride [4-(α-3-Chlorobenzo[b]thien-2-yl-p-dimethylaminobenzylidene)-2,5-cyclohexadien-1-ylidene]dimethylammonium chloride [4-(p-Dimethylamino-α-3-methylbenzo[b]thien-2-ylbenzylidene)-2,5-cyclohexadien-1-ylidene]dimethylammonium chloride [4-[2-Bromo-α-(p-dimethylaminophenyl)-3-thenylidene]-2,5-cyclohexadien-1-ylidene]dimethylammonium chloride [4-[2-Chloro-α-(p-dimethylaminophenyl)-3-thenylidene]-2,5-cyclohexadien-1-ylidene]dimethylammonium chloride [4-[α-(p-Dimethylaminophenyl)-3-thenylidene]-2,5-cyclohexadien-1-ylidene]dimethylammonium chloride [4-[α-(p-Dimethylaminophenyl)-2,5-dimethyl-3-thenylidene]-2,5-cyclohexadien-1-ylidene]dimethylammonium chloride Pyridinium, 1-[[4-(dimethylamino)phenyl][4-(dimethylamino)-2,5-cyclohexadien-1-ylidene]methyl]-, bis[tetrafluoroborate (1-)] 2,5-Cyclohexadien-1-one, 2,3,5,6-tetrachloro-4-[(pentachlorophenyl)(2,3,5,6-tetrachloro-4-pyridinyl)methylene]-Ethananinium, N-[4-[[4-(diethylamino)phenyl](4-oxo-4H-1-benzothiopyran-3-yl)methylene]-2,5-cyclohexadien-1-ylidene]-N-ethyl-, perchlorate Ethanaminium, N-[4-[[4-(diethylamino)phenyl](4-oxo-4H-1-benzothiopyran-3-yl)methylene]-2,5-cyclohexadien-1-ylidene]-N-ethyl-4H-1-Benzothiopyran, ethanaminium deriv. Benzenaminium, N-methyl-N-[4-[[4-(methylphenylamino) phenyl)(4-oxo-4H-1-benzothiopyran-3-yl)methylene]-2,5-cyclohexadien-1-ylidene]-, perchlorate Benzenaminium, N-methyl-N-[4-[[4-(methylphenylamino)phenyl](4-oxo-4H-1-benzothiopyran-3-yl)methylene]-2,5-CyClohexadien-1-ylidene]-Ethanaminium, N-[4-[[4-(diethylamino)-2-methylphenyl](4-oxo-4H-1-benzothiopyran-3-yl)methylene]-3-methyl-2,5-cyclohexadien-1-ylidene]-N-ethyl-, perchlorate Ethanaminium, N-[4-[[4-(diethylamino)-2-methylphenyl](4-oxo-4H-1-benzothiopyran-3-yl)methylene]-3-methyl-2,5-cyclohexadien-1-ylidene]-N-ethylEthanaminium 4H-1-Benzothiopyran, Methanaminium, N-[4-[[4-(dimethylamino)phenyl][5-(4-iodophenyl)-2-furanyl]methylene]-2,5-cyclohexadien-1-ylidene]-N-methyl-, chloride Methanaminium, N-[4-[[5-(4-bromophenyl)-2-furanyl][4-(dimethylamino)phenyl]methylene]-2,5-cyclohexadien-1-ylidene]-N-ethyl-, chloride Methanaminium, N-[4-[[4-(dimethylamino)phenyl][5-(4-nitrophenyl)-2-furanyl]methylene]-2,5-cyclohexadien-1-ylidene]-methyl-, chloride [Oxybis[methylene-5,2-furandiyl[p-(N-methylanilino)benzylidyne]-2,5-cyclohexadiene-4,1-diylidene]]bis[methylphenylammonium chloride] [Oxybis[methylene-5,2-furandiyl[p-(N-methylanilino)benzylidyne]-2,5-cyclohexadiene-4,1-diylidene]]bis[methylphenylammonium hydrogensulphate] Benzenaminium, N,N'-[oxybis[methylene-5,2-furandiyl[[4-(methylphenylamino)phenyl]methylidyne]-2,5-cyclohexadiene-4,1-diylidene]]bis[N-methyl-1,4-Cyclohexadiene-1-sulphonic acid and 3-[4-anilino-α-(1-methyl-2-phenylindol-3-yl)-3-sulphobenzylidene]-6-phenylimino- salt Trifluoromethane sulphonic acid, 1-[[4-(dimethylamino)phenyl][4-(dimethyliminio)-2,5-cyclohexadien-1-ylidene]methyl, and pyridinium salt Trifluoromethane sulphonic acid and 1-[[4-(dimethylamino)phenyl][4-(dimethyliminio)-2,5-yclohexadien-1-ylidene]methyl]pyridinium salt Pyridinium, 1-[[4-(dimethylamino)phenyl][4-(dimethylimino)-2,5-cyclohexadien-1-ylidene]methyl]-[4-[p-(Dimethylamino)-α-(9-methylcarbazol-3-yl)benzylidene]-2,5-cyclohexadien-1-ylidene]dimethylammonium chloride [4-[α-[p-(Dimethylamino)phenyl]furfurylidene]-2,5-cyclohexadien-1-ylidene]dimethylammonium chloride Methanaminium N-[4-[bis(1,2,3,4,10,14b-hexahydro-2-methyldibenzo[c,f]pyrazino[1,2-a]azepin-8-yl)methylene]-2,5-cyclohexadien-1-ylidene]-N-methyl-, perchlorate, diperchlorate Methanaminium, N-[4-[bis(1,2,3,4,10,14b-hexahydro-2-methyldibenzo[c,f]pyrazino[1,2-a]azepin-8-yl)methylene]-2,5-cyclohexadien-1-ylidene]-N-methyl-, perchlorate Methanaminium, N-[4-[bis(1,2,3,4,10,14b-hexahydro-2-methyldibenzo[c,f]pyrazino[1,2-a]azepin-8-yl)methylene]-2,5-cyclohexadien-1-ylidene]-N-methyl-1H-Indolizinium, 3-[[4-(dimethyliminio)-2,5-cyclohexadien-1-ylidene](4-methoxyphenyl)methyl]-2-(2-hydroxy-5-methylphenyl)-1-methyl-, 1H-Indolizinium, 3-[[4-(dimethyliminio)-2,5-cyclohexadien-1-ylidene]phenylmethyl]-2-(2-hydroxy-5-methylphenyl)-1-methyl-, 1H-Indolizinium, 3-[[4-(dimethylamino)phenyl][4-(dimethyliminio)-2,5-cyclohexadien-1-ylidene]methyl]-2-(2-hydroxy-5-methylphenyl)-1-methyl-, 1H-Indolizinium, 3-[[4-(dimethylamino)phenyl][4-(dimethyliminio)-2,5-cyclohexadien-1-ylidene]methyl]-2-(2-hydroxy-5-methylphenyl)-1-methyl-1-Propanaminium, N-[4-[2-benzofuranyl[3-chloro-4-[ethyl(3-sulphopropyl)amino]phenyl]methylene]-2-chloro-2,5-cyclohexadien-1-ylidene]-N-ethyl-3-sulpho-, inner salt, sodium salt 1-Propanaminium, N-[4-[2-benzofuranyl[4-[ethyl(3-sulphopropyl)amino]-3-methylphenyl]methylene]-2-methyl-2,5-cyclohexadien-1-ylidene]-N-ethyl-3-1-Butanaminium, N-[4-[2-benzofuranyl[4-[ethyl(4-sulphobutyl)amino]phenyl]methylene]-2,5-cyclohexadien-1-ylidene]-N-ethyl-4-1-Propanaminium, N-[4-[(5-chlorobenzo[b]thien-2-yl)[4-[methyl(3-sulphopropyl)amino]phenyl]methylene]-2,5-cyclohexadien-1-ylidene]-N-methyl-3-sulpho-, 1-Propanaminium, N-[4-[(3-chlorobenzo[b]thien-2-yl)[4-[methyl(3-sulphopropyl)amino]phenyl]methylene]-2,5-cyclohexadien-1-ylidene]-N-methyl-3-sulpho-, sodium salt 1-Propanaminium, N-[4-[benze[b]thien-2-yl[4-[methyl(3-sulphopropyl)amino]phenyl]methylene]-2,5-cyclohexadien-1-ylidene]-N-methyl-3-sulpho-, sodium salt 1-Propanaminium, N-methyl-N-[4-[(3-methylbenzo[b]thien-2-yl)[4-[methyl(3-sulphopropyl)amino]phenyl]methylene]-2,5-cyclohexadien-1-ylidene]-3-sulpho-, sodium salt 1-Propanaminium, N-[4-[(3-bromobenzo[b]thien-2-yl)[4-[methyl(3-sulphopropyl)amino]phenyl]methylene]-2,5-cyclohexadien-1-ylidene]-N-methyl-3-sulpho-, sodium salt 1-Propanaminium, N-[4-[(6-chlorobenzo[b]thien-2-yl)[4-[methyl(3-sulphopropyl)amino]phenyl]methylene]-2,5-cyclohexadien-1-ylidene]-N-methyl-3-sulpho-, sodium salt 1-Propanaminium, N-methyl-N-[4-[[4-[methyl(3-sulphopropyl)amino]phenyl](5-nitro-2-benzofuranyl)methylene]-2,5-cyclohexadien-1-ylidene]-3-sulpho-, sodium salt 1-Propanaminium, N-methyl-N-[4-[(5-methyl-2 benzofuranyl)[4-[methyl(3-sulphopropyl)amino]phenyl]methylene]-2,5-cyclohexadien-1-ylidene]-3-sulpho-, sodium salt 1-Propanaminium, N-[4-[(5-chloro-2-benzofuranyl)[4-[methyl (3-sulphopropyl)amino]phenyl]methylene]-2,5-cyclohexadien-1-ylidene]-N-methyl-3-sulpho-, sodium salt 1-Propanaminium, N-[4-[2-benzofuranyl[4-[methyl(3-sulphopropyl)amino]phenyl]methylene]-2,5-cyclohexadien-1-ylidene]-N-methyl-3-sulpho-, inner salt, sodium salt Ethanaminium, N-[4-[(2-chlorophenyl)(1-methyl-2-phenyl-1H-indol-3-yl)methylene]-2,5-cyclohexadien-1-ylidene]-N-ethyl-, trichlorozincate (1-) Zincate (1-), trichloro-, N-[4-[(2-chlorophenyl)(1-methyl-2-phenyl-1H-indol-3-yl)methylene]-2,5-cyclohexadien-1-ylidene]-N-ethylethanaminium Benzenesulphonic acid, 5-[(1,2-dimethyl-1-H-indol-3-yl)[4-[(2-methylphenyl)imino]-3-sulpho-2,5-cyclohexadien-1-ylidene]methyl]-2-[(2-methylphenyl)amino]-, monosodium salt Ethanaminium, N-[4-[(2-chlorophenyl)(1-methyl-2-phenyl-1H-indol-3-yl)methylene]-2,5-cyclohexadien-1-ylidene]-N-ethyl-, methyl sulphate 3H-Pyrazol-3-one, 1,2-dihydro-4-[(4-hydroxy-3-methoxyphenyl)(3-methoxy-4-oxo-2,5-cyclohexadien-1-ylidene)methyl]-1,5-dimethyl-2-phenyl-, ion (1-) Ethanaminium, N-[4-[(2-chlorophenyl)(1-methyl-2-phenyl-1H-indol-3-yl)methylene]-2,5-cyclohexadien-1-ylidene]-N-ethyl-, acetate Ethanaminium, N-[4-[(2-chlorophenyl)(1-methyl-2-phenyl-1H-indol-3-yl)methylene]-2,5-cyclohexadien-1-ylidene]-N-ethyl-, phosphate (1:1) Ethanaminium, N-[4-[(2-chlorophenyl)(1-methyl-2-phenyl-1H-indol-3-yl)methylene]-2,5-cyclohexadien-1-ylidene]-N-ethylEthanaminium, N-[4-[(2-chlorophenyl)(1-methyl-2-phenyl-1H-indol-3-yl)methylene]-2,5-cyclohexadien-1-ylidene]-N-ethyl-, chloride, compounded with zinc chloride (ZnCl₂) N-[4-[(2-chlorophenyl)(1-methyl-2-phenyl-1H-indol-3-yl)methylene]-2,5-cyclohexadien-1-ylidene]-N-ethylethanaminium chloride 1-Piperidinyloxy, 4-[[3,5-bis(1,1-dimethylethyl)-4-oxo-2,5-cyclohexadien-1-ylidene][3,5-bis(1,1-dimethylethyl)-4-oxyphenyl]methyl]-2,2,6,6-tetramethyl-1H-Pyrrol-1-yloxy, 3-[[3,5-bis(1,1-dimethylethyl)-4-oxo-2,5-cyclohexadien-1-ylidene][3,5-bis(1,1-dimethylethyl)-4-oxyphenyl]methyl]-2,5-dihydro-2,2,5,5-tetramethyl-1H-Pyrrol-1-yloxy, 3-[[3,5-bis(1,1-dimethylethyl)-4-hydroxyphenyl][3,5-bis(1,1-dimethylethyl)-4-oxo-2,5-cyclohexadien-1-ylidene]methyl]-2,5-dihydro-2,2,5,5-tetramethyl-1-Piperidinyloxy, 4-[[3,5-bis(1,1-dimethylethyl)-4-hydroxyphenyl][3,5-bis(1,1-dimethylethyl)-4-oxo-2,5-cyclohexadien-1-ylidene]methyl]-2,2,6,6-tetramethyl-Acetamide, N-methyl-N-[[2-[[4-oxo-3-[2,2,2-trifluoro-1-hydroxy-1-(trifluoromethyl)ethyl]-1(4H-naphthalenylidene](2,3,6,7-tetrahydro-1H,5H-benzo[ij]quinolizin-9-yl)methyl]phenyl]sulphonyl]-Carbamic acid and [[2-[(3,5-dimethoxy-4-oxo-2,5-cyclohexadien-1-ylidene)(2,3,6,7-tetrahydro-1H,5H-benzo[ij]quinolizin-9-yl)methyl]phenyl]sulphonyl]methyl-, 2-(methylsulphonyl)ethyl ester Carbamic acid and methyl[[2-[[4-oxo-3-[2,2,2-trifluoro-1-hydroxy-1-(trifluoromethyl)ethyl]-1(4H)-naphthalenylidene](2,3,6,7-tetrahydro-1H,5H-benzo[ij]quinolizin-9-yl)methyl]phenyl]sulphonyl]-, 2-cyanoethyl ester Carbamic acid and methyl[[2-[[4-oxo-3-[2,2,2-trifluoro-1-hydroxy-1-(trifluoromethyl)ethyl]-1(4H)-naphthalenylidene](2,3,6,7-tetrahydro-1H,5H-benzo[ij]quinolizin-9-yl)methyl]phenyl]sulphonyl]-ester, 2-(methylsulphonyl)ethyl ester Carbamic acid and methyl[[2-[[4-oxo-3-[2,2,2-trifluoro-1-hydroxy-1-(trifluoromethyl)ethyl]-1(4H)-naphthalenylidene](2,3,6,7-tetrahydro-1H,5H-benzo[ij]quinolizin-9-yl)methyl]phenyl]sulphonyl]-, 2-(phenylsulphonyl)ethyl ester, Acetamide, N-[[2-[(3,5-dimethyl-4-oxo-2,5-cyclohexadien-1-ylidene)(2,3,6,7-tetrahydro-1H,5H-benzo[ij]quinolizin-9-yl)methyl]phenyl]sulphonyl]-N-methyl-2,5-Cyclohexadien-1-one, 4-[(4-hydroxy-3,5-dimethylphenyl)(2,3,5,6,8,9,11,12-octahydro-1,4,7,10,13-benzopentaoxacyclopentadecin-15-yl)methylene]-2,6-dimethyl-Phenoxy, 4-[[3,5-bis(1,1-dimethylethyl)-4-oxo-2,5-cyclohexadien-1-ylidene](2,3,5,6,8,9,11,12-octahydro-1,4,7,10,13-benzopentaoxacyclopentadecin-15-yl)methyl]-2,6-bis (1,1-dimethylethyl)-Ethanaminiumn, N-[4-[(2-chlorophenyl)(1-methyl-2-phenyl-1H-indol-3-yl)methylene]-2,5-cyclohexadien-1-ylidene]-N-ethyl-, chloride Phenoxy, 4-[[3,5-bis(1,1-dimethylethyl)-4-oxo-2,5-cyclohexadien-1-ylidene]-3-pyridinylmethyl]-2,6-bis(1,1-dimethylethyl)-2,5-Cyclohexadien-1-one, 4-[[3,5-bis(1,1-dimethylethyl)-4-hydroxyphenyl]-3-pyridinylmethylene]-2,6-bis(1,1-dimethylethyl)-, ion (1-) 2,5-Cyclohexadien-1-one, 4-[[3,5-bis(1,1-dimethylethyl)-4-hydroxyphenyl]-3-pyridinylmethylene]-2,6-bis(1,1-dimethylethyl)-Phenoxy, 4,4'-[3,5-pyridinediylbis[[3,5-bis(1,1-dimethylethyl)-4-oxo-2,5-cyclohexadien-1-ylidene]methylene]]bis[2,6-bis(1,1-dimethylethyl)-Phenoxy, 4,4'-[2,4-pyridinediylbis[[3,5-bis(1,1-dimethylethyl)-4-oxo-2,5-cyclohexadien-1-ylidene]methylene]]bis[2,6-bis(1,1-dimethylethyl)-Phenoxy, 4,4'-[2,5-pyridinediylbis[[3,5-bis(1,1-dimethylethyl)-4-oxo-2,5-cyclohexadien-1-ylidene]methylene]]bis[2,6-bis(1,1-dimethylethyl)-Methanaminium, N-[4-[9H-carbazol-3-yl[4-(dimethylamino) phenyl]methylene]-2,5-cyclohexadien-1-ylidene]-methyl-, chloride [4-[α-Carbazol-3-yl-p-(dimethylamino)benzylidene]-2,5-cyclohexadien-1-ylidene]dimethylammonium chloride Benzenamine, 4-ethoxy-N-[4-[[4-[(4-ethoxyphenyl)amino]phenyl](1-methyl-2-phenyl-1H-indol-3-yl)methylene]-2,5-cyclohexadien-1-ylidene]-, acetate Benzenamine, 4-ethoxy-N-[4-[[4-[(4-ethoxyphenyl)amino]phenyl](1-methyl-2-phenyl-1H-indol-3-yl)methylene]-2,5-cyclohexadien-1-ylidene]-(2,5-Cyclohexadien-1-one, 4,4'-[3,5-pyridinediylbis[[3,5-bis(1,1-dimethylethyl)-4-hydroxyphenyl]methylidyne]]bis[2,6-bis(1,1-dimethylethyl)-, ion (2-)-2,5-Cyclohexadien-1-one, 4,4'-[2,6-pyridinediylbis[[3,5-bis (1,1-dimethylethyl)-4-hydroxyphenyl]methylidyne]]bis[2,6-bis(1,1-dimethylethyl)-, ion (2-)-2,5-Cyclohexadien-1-one, 4,4'-[2,5-pyridinediylbis[[3,5-bis(1,1-dimethylethyl)-4-hydroxyphenyl]methylidyne]]bis[2,6-bis(1,1-dimethylethyl)-, ion (2-)-2,5-Cyclohexadien-1-one, 4,4'-[2,4-pyridinediylbis[[3,5-bis (1,1-dimethylethyl)-4-hydroxyphenyl]methylidyne]]bis[2,6-bis(1,1-dimethylethyl)-, ion (2-)-2,5-Cyclohexadien-1-one, 4,4'-[3,5-pyridinediylbis[[3,5-bis(1,1-dimethylethyl)-4-hydroxyphenyl]methylidyne]]bis[2,6-bis(1,1-dimethylethyl)-2,5-Cyclohexadien-1-one, 4,4'-[2,5-pyridinediylbis[[3,5-bis (1,1-dimethylethyl)-4-hydroxyphenyl]methylidyne]]bis[2,6-bis(1,1-dimethylethyl)-2,5-Cyclohexadien-1-one, 4,4'-[2,4-pyridinediylbis[[3,5-bis (1,1-dimethylethyl)-4-hydroxyphenyl]methylidyne]]bis[2,6-bis(1,1-dimethylethyl)-Cyclohexanaminium, N-methyl-N-[4-[(2-methyl-1H-indol-3-yl)phenylmethylene]-2,5-cyclohexadien-1-ylidene]-, trichlorozincate (1-) Zincate (1-), trichloro-, N-methyl-N-[4-[(2-methyl-1H-indol-3-yl)phenylmethylene)-2,5-cyclohexadien-1-ylidene]cyclohexanaminium Cyclohexanaminium, N-methyl-N-[4-[(2-methyl-1H-indol-3-yl)phenylmethylene]-2,5-cyclohexadien-1-ylidene]-Cyclohexanaminium, N-[4-[(2-chlorophenyl)(1-methyl-2-phenyl-1H-indol-3-yl)methylene]-2,5-cyclohexadien-1-ylidene]-N-methyl-, trichlorozincate(1-) Zincate (1-), trichloro-, N-[4-[(2-chlorophenyl)(1-methyl-2-phenyl-1H-indol-3-yl)methylene]-2,5-cyclohexadien-1-ylidene]-N-methylcyclohexanaminium Cyclohexanaminium, N-[4-[(2-chlorophenyl)(1-methyl-2-phenyl-1H-indol-3-yl)methylene]-2,5-cyclohexadien-1-ylidene]-N-methyl-Cyclohexanaminium, N-[4-[(2-chlorophenyl)(2-(4-chlorophenyl)-1-methyl-1H-indol-3-yl]methylene]-2,5-cyclohexadien-1-ylidene]-N-methyl-, trichlorozincate(1-) N-[4-[(2-chlorophenyl)[2-(4-chlorophenyl)-1-methyl-1H-indol-3-yl]methylene]-2,5-cyclohexadien-1-ylidene]-N-methylcyclohexanaminium trichlorozincate Cyclohexanaminium, N-[4-[(2-chlorophenyl)[2-(4-chlorophenyl)-1-methyl-1H-indol-3-yl]methylene]-2,5-cyclohexadien-1-ylidene]-N-methyl-Benzenemethanaminium, N-cyclohexyl-N-[4-[[1-(2-ethoxyethyl)-2-phenyl-1H-indol-3-yl](4-methylphenyl)methylene]-2,5-cyclohexadien-1-ylidene]-, trichlorozincate(1-) N-cyclohexyl-N-[4-[[1-(2-ethoxyethyl)-2-phenyl-1H-indol-3-yl](4-methylphenyl)methylene]-2,5-cyclohexadien-1-ylidene]benzenemethanaminium trichlorozincate Benzenemethanaminium, N-cyclohexyl-N-[4-[[1-(2-ethoxyethyl)-2-phenyl-1H-indol-3-yl](4-methylphenyl)methylene]-2,5-cyclohexadien-1-ylidene]-Cyclohexanaminium, N-methyl-N-[4-[(1-methyl-2-phenyl-1H-indol-3-yl)phenylmethylene]-2,5-cyclohexadien-1-ylidene]-, trichlorozincate(1-) N-methyl-N-[4-[(1-methyl-2-phenyl-1H-indol-3-yl)phenylmethylene]-2,5-cyclohexadien-1-ylidene]cyclohexanaminium trichlorozincate Cyclohexanaminium, N-methyl-N-[4-[(1-methyl-2-phenyl-1H-indol-3-yl)phenylmethylene]-2,5-cyclohexadien-1-ylidene]-Cyclohexanaminium, N-[4-[(2-bromophenyl)(1,2-dimethyl-1H-indol-3-yl)methylene]-2,5-cyclohexadien-1-ylidene]-N-ethyl-, trichlorozincate(1-) N-[4-[(2-bromophenyl)(1,2-dimethyl-1H-indol-3-yl)methylene]-2,5-cyclohexadien-1-ylidene]-N-ethylcyclohexanaminium, trichlorozincate Cyclohexanaminium, N-[4-[(2-bromophenyl)(1,2-dimethyl-1H-indol-3-yl)methylene]-2,5-cyclohexadien-1-ylidene]-N-ethylEthanaminium, N-[3-chloro-4-[(1-methyl-2-phenyl-1H-indol-3-yl)phenylmethylene]-2,5-cyclohexadien-1-ylidene]-N-ethyl-, sulphate (1:1) Ethanaminium, N-[3-chloro-4-[(1-methyl-2-phenyl-1H-indol-3-yl)phenylmethylene]-2,5-cyclohexadien-1-ylidene]-N-ethylEthanaminium, N-ethyl-N-[4-[(2-methyl-1H-indol-3-yl)(2-nitrophenyl)methylene]-2,5-cyclohexadien-1-ylidene]-, sulphate (1:1) Ethanaminium, N-ethyl-N-[4-[(2-methyl-1H-indol-3-yl)(2-nitrophenyl)methylene]-2,5-cyclohexadien-1-ylidene]-Methanamine, N-[4-[(1-methyl-2-phenyl-1H-indol-3-yl)phenylmethylene]-2,5-cyclohexadien-1-ylidene]-, sulphate (1:1) Methanamine, N-[4-[(1-methyl-2-phenyl-1H-indol-3-yl) phenylmethylene]-2,5-cyclohexadien-1-yldene]-Methanaminium, N-[4-[[4-(dimethylamino)phenyl]-2-furanylmethylene]-2,5-cyclohexadien-1-ylidene]-N-methyl-, (T-4)-tetrabrothallate(1-) Thallate(1-), tetrabromo-, (T-4)-, N-[4-[[4-(dimethylamino)phenyl]-2-furanylmethylene]-2,5-cyclohexadien-1-ylidene]-N-methylmethanaminium Phenoxy, 4-[[3,5-bis(1,1-dimethylethyl)-4-oxo-2,5-cyclohexadien-1-ylidene][6-[[3,5-bis(1,1-dimethylethyl)-4-oxo-2,5-cyclohexadien-1-ylidene]]3,5-bis(1,1-dimethylethyl)-4-oxyphenyl]methyl]-2-pyridinyl]methyl]-2,6-bis(1,1-dimethylethyl)-2,5-Cyclohexadien-1-one, 4,4'-[2,6-pyridinediylbis[[3,5-bis(1,1-dimethylethyl)-4-hydroxyphenyl]methylidyne]]bis[2,6-bis(1,1-dimethylethyl)-Methanaminium, N-[4-[(2,3-dihydro-1,5-dimethyl-3-oxo-2-phenyl-1H-pyrazol-4-yl)(3-nitrophenyl)methylene]-2,5-cyclohexadien-1-ylidene]-N-methyl-, chloride Methanaminium, N-[4-[(3-bromophenyl)(2,3-dihydro-1,5-dimethyl-3-oxo-2-phenyl-1H-pyrazol-4-yl)methylene]-2,5-cyclohexadien-1-ylidene]-N-methyl-, chloride Methanaminium, N-[4-[(2,3-dihydro-1,5-dimethyl-3-oxo-2-phenyl-1H-1-pyrazol-4-yl)(3-methoxyphenyl)methylene]-2,5-cyclohexadien-1-ylidene]-N-methyl-, chloride Methanaminium, N-[4-[(2,3-dihydro-1,5-dimethyl-3-oxo-2-phenyl-1H-pyrazol-4-yl)phenylmethylene]-2,5-cyclohexadien-1-ylidene]-N-methyl-, chloride [[4-[(1-methyl-2-phenyl-1H-indol-3-yl)[4-[(sulphophenyl)amino]phenyl]methylene]-2,5-cyclohexadien-1-ylidene]amino]-Methanaminium, N-[4-[(2,3-dihydro-1,5-dimethyl-3-oxo-2-phenyl-1H-pyrazol-4-yl)[4-(dimethylamino)phenyl]methylene]-2,5-cyclohexadien-1-ylidene]-N-methyl-, chloride Benzenamine, 4-[(2-[1,1'-biphenyl]-4-yl-1-butyl-1H-indol-3-yl)[4-[(4-ethoxyphenyl)imino]-2,5-cyclohexadien-1-ylidenemethyl]-N-(4-ethoxyphenyl)-Benzenaminium, N-methyl-N-[4-[[4-(methylphenylamino)phenyl](4-oxo-4H-1-benzopyran-3-yl)methylene]-2,5-cyclohexadien-1-ylidene]-Ethanaminium, N-[4-[[4-(diethylamino)phenyl](4-oxo-4H-1-benzopyran-3-yl)methylene]-2,5-cyclohexadien-1-ylidene]-N-ethyl-Benzenaminium, 4-ethoxy-N-methyl-N-[4-[(1-methyl-2-phenyl-1H-indol-3-yl)phenylmethylene]-2,5-cyclohexadien-1-ylidene]-, chloride Methanaminium, N-[4-[12H-benzo[a]phenoxazin-5-yl[4-(dimethylamino)phenyl]methylene]-2,5-cyclohexadien-1-ylidenel N-methyl-, chloride Benzenaminium, N-methyl-N-[4-[(1-methyl-2-phenyl-1H-indol-3-yl)phenylmethylenel-2,5-cyclohexadien-1-ylidene]-Ethanaminium, N-[4-[[4-(diethylamino)phenyl](7-methoxy-2,1,3-benzothiadiazol-4-yl)methylene]-2,5-cyclohexadien-1-ylidene]-N-ethylEthanaminium, N-[4-[[4-(diethylamino)phenyl)(5-methoxy-2,1,3-benzothiadiazol-4-yl)methylene]-2,5-cyclohexadien-1-ylidene]-N-ethylEthanaminium, N-[4-[[4-(diethylamino)phenyl](7-methyl-2,1,3-benzothiadiazol-4-yl)methylene]-2,5-cyclohexadien-1-ylidene]-N-ethylEthanaminium, N-[4-[2,1,3-benzothiadiazol-5-yl[4-(diethylamino)phenyl]methylene]-2,5-cyclohexadien-1-ylidene]N-ethylEthanaminium, N-[4-[2,1,3-benzothiadiazol-4-yl[4-(diethylamino)phenyl]methylene]-2,5-cyclohexadien-1-ylidene] N-ethyl-Methanaminium, N-methyl-N-[4-[(1-methyl-2-phenyl-1H-indol-3-yl)phenylmethylene]-2,5-cyclohexadien-1-ylidene]-, chloride Methanaminium, N-methyl-N-[4-[(1-methyl-2-phenyl-1H-indol-3-yl)phenylmethylene]-2,5-cyclohexadien-1-ylidene]-Methanaminium, N-[4-[[4-(dimethylamino)phenyl](7-methoxy-2,1,3-benzothiadiazol-4-yl)methylene]-2,5-cyclohexadien-1-ylidene]-N-methyl-Methanaminium, N-[4-[[4-(dimethylamino)phenyl](5-methoxy-2,1,3-benzothiadiazol-4-yl)methylene]-2,5-cyclohexadien-1-ylidene]-N-methyl-Methanaminium, N-[4-[[4-(dimethylamino)phenyl](7-methyl-2,1,3-benzothiadiazol-4-yl)methylene]-2,5-cyclohexadien-1-ylidene]-N-methyl-Methanaminium, N-[4-[2,1,3-benzothiadiazol-5-yl[4-(dimethylamino)phenyl]methylene]-2,5-cyclohexadien-1-ylidene]-N-methyl-Methanaminium, N-[4-[2,1,3-benzothiadiazol-4-yl[4-(dimethylamino)phenyl]methylene]-2,5-cyclohexadien-1-ylidene]-N-methyl-Methanaminium, N-[4-[[4-(dimethylamino)phenyl]-3-pyridinylmethylene]-2,5-cyclohexadien-1-ylidene]-N-methyl-Methanaminium, N-[4-[[4-(dimethylamino)phenyl]-4-pyridinylmethylene]-2,5-cyclohexadien-1-ylidene]-N-methyl-Methanaminium, N-[4-[[4-(dimethylamino)phenyl]-2-thienylmethylene]-2,5-cyclohexadien-1-ylidene]-N-methyl-Methanaminium, N-[4-[[4-(dimethylamino)phenyl]-2-furanylmethylene]-2,5-cyclohexadien-1-ylidene]-N-methyl-Methanaminium, N-[4-[[4-dimethylamino)phenyl]-3-thienylmethylene]-2,5-cyclohexadien-1-ylidene]-N-methyl-[[4-[(1-methyl-2-phenyl-1H-indol-3-yl)[4-[(sulphophenyl)amino]phenyl]methylene]-2,5-cyclohexadien-1-ylidene]amino]-, Ethoxy[[4-[[4-[(ethoxysulphophenyl)amino]phenyl](1-methyl-2-phenyl-1H-indol-3-yl)methylene]-2,5-cyclohexadien-1-ylidene] amino]-Benzenamine, N-methyl-4-[[4-(methylimino)-2,5-cyclohexadien-1-ylidene](2-methyl-1H-indol-3-yl)methyl]-, Methanaminium, N-[4-[[4-(dimethylamino)phenyl](2-methyl-1H-indol-3-yl)methylene]-2,5-cyclohexadien-1-ylidene]-N-methyl-Benzenamine, N-methyl-4-[[4-(methylimino)-2,5-cyclohexadien-1-ylidene](1-methyl-2-phenyl-1H-indol-3-yl)methyl]-, conjugate monoacid Methanaminium, N-[4-[[4-(dimethylamino)phenyl](1-methyl-2-phenyl-1H-indol-3-yl)methylene]-2,5-cyclohexadien-1-ylidene]-N-methyl-Benzenamine, 4-ethoxy-N-[4-[[4-[(4-ethoxyphenyl)amino]phenyl](2-phenyl-1H-indol-3-yl)methylene]-2,5-cyclohexadien-1-ylidene]-, Benzenamine, 4-ethoxy-N-[4-[[4-[(4-ethoxyphenyl)amino]phenyl](1-methyl-1H-indol-3-yl)methylene]-2,5-cyclohexadien-1-ylidene]-, Benzenamine, N-[4-[(1,2-dimethyl-1H-indol-3-yl)[4-[(4-ethoxyphenyl)amino]phenyl]methylene]-2,5-cyclohexadien-1-ylidene]-4-ethoxy-Benzenamine, 4-[(1-methyl-2-phenyl-1H-indol-3-yl)[4-(phenylimino)-2,5-cyclohexadien-1-ylidene]methyl]-N-phenyl-, conjugate monoacid Benzenamine, 4-ethoxy-N-[4-[[4-[(4-ethoxyphenyl)amino]phenyl](1-methyl-2-phenyl-1H-indol-3-yl)methylene]-2,5-cyclohexadien-1-ylidene]-, Methanaminium, N-[4-[[4-(dimethylamino)phenyl](2-phenyl-1H-indol-3-yl)methylene]-2,5-cyclohexadien-1-ylidene]-N-methyl-Benzenaminium, N-methyl-N-[4-[[4-(methylphenylamino)phenyl](4-oxo-4H-1-benzopyran-3-yl)methylene]-2,5-cyclohexadien-1-ylidene]-, chloride Benzenaminium, N-methyl-N-[4-[[4-(methylphenylamino)phenyl](4-oxo-4H-1-benzopyran-3-yl)methylene]-2,5-cyclohexadien-1-ylidene]-, perchlorate Ethanaminium, N-[4-[[4-(diethylamino)phenyl](4-oxo-4H-1-benzopyran-3-yl)methylene]-2,5-cyclohexadien-1-ylidene]-N-ethyl-, perchlorate Ethanaminium, N-[4-[[4-(diethylamino)phenyl](4-oxo-4H-1-benzopyran-3-yl)methylene]-2,5-cyclohexadien-1-ylidene]-N-ethyl-, chloride Benzenaminium, N-methyl-N-[4-[[4-(methylphenylamino)phenyl](4-oxo-4H-1-benzopyran-3-yl)methylene]-2,5-cyclohexadien-1-ylidene]-, acetate Ethanaminium, N-[4-[[4-(diethylamino)phenyl](4-oxo-4H-1-benzopyran-3-yl)methylene]-2,5-cyclohexadien-1-ylidene]-N-ethyl-, acetate Methanaminium, N-[4-[(2,3-dihydro-1,5-dimethyl-3-oxo-2-phenyl-1H-pyrazol-4-yl)(3-nitrophenyl)methylene]-2,5-cyclohexadien-1-ylidene]-N-methyl-Methanaminium, N-[4-[(3-bromophenyl)(2,3-dihydro-1,5-dimethyl-3-oxo-2-phenyl-1H-pyrazol-4-yl)methylene]-2,5-cyclohexadien-1-ylidene]-N-methyl Methanaminium, N-[4-[(2,3-dihydro-1,5-dimethyl-3-oxo-2-phenyl-1H-pyrazol-4-yl)(3-methoxyphenyl)methylene]-2,5-cyclohexadien-1-ylidene]-N-methyl-Methanaminium, N-[4-[(2,3-dihydro-1,5-dimethyl-3-oxo-2-phenyl-1H-pyrazol-4-yl)phenylmethylene]-2,5-cyclohexadien-1-ylidene]-N-methyl-Methanaminium, N-(4-[(2,3-dihydro-1,5-dimethyl-3-oxo-2-phenyl-1H-pyrazol-4-yl)(3-methylphenyl)methylene]-2,5-cyclohexadien-1-ylidene]-N-methyl-Benzenamine, N-[4-[(1-ethyl-2-phenyl-1H-indol-3-yl)[4-[(2-methoxyphenyl)amino]phenyl]methylene]-2,5-cyclohexadien-1-ylidene]-2-methoxy-, monohydrochloride 2-hydroxy-5-[[4-[[4-[(4-hydroxy-3-sulphophenyl)amino]phenyl](1-methyl-2-phenyl-1H-indol-3-yl)methylene]-2,5-cyclohexadien-1-ylidene]amino]-Benzenamine, 2-ethoxy-N-[4-[[4-[(2-ethoxyphenyl)amino]phenyl](1-methyl-2-phenyl-1H-indol-3-yl)methylene]-2,5-cyclohexadien-1-ylidene]-, monohydrochloride Methanaminium, N-[4-[[4-(dimethylamino)phenyl]-2-furanylmethylene]-2,5-cyclohexadien-1-ylidene]-N-methyl-, perchlorate 2-ethoxy-5-[[4-[[4-[(4-ethoxy-3-sulphophenyl)amino]phenyl](1-methyl-2-phenyl-1H-indol-3-yl)methyl]-2,5-cyclohexadien-1-ylidene]amino]-Ammonium, [4-[α-[p-(dimethylamino)phenyl]furfurylidene]-2,5-cyclohexadien-1-ylidene]dimethyl-, tetrachlorogallate (1-) Methanaminium, N-[4-[[4-(dimethylamino)phenyl]-2-thienylmethyl]-2,5-cyclohexadien-1-ylidene]-N-methyl-, perchlorate Ammonium, [4-[p-(dimethylamino)-α-2-thienylbenzylidene]-2,5-cyclohexadien-1-ylidene]dimethyl-, perchlorate Ammonium, (2-cyanoethyl)[4-[α-[4-[(2-cyanoethyl)ethylamino]-o-tolyl]piperonylidene]-3-methyl-2,5-cyclohexadien-1-ylidene]ethylAmmonium, dimethyl[4-[α-(1-methyl-2-phenylindol-3-yl)benzylidene]-2,5-cyclohexadien-1-ylidene]-, chloride, Ammonium, (p-ethoxyphenyl)methyl[4-[α-(1-methyl-2-phenylindol-3-yl)benzylidene]-2,5-cyclohexadien-1-ylidene]-, chloride, (p-ethoxyphenyl)methyl[4-[α-(1-methyl-2-phenylindol-3-yl)benzylidene]-2,5-cyclohexadien-1-ylidene]ammonium chloride Ammonium, [4-[α-2,1,3-benzothiadiazol-4-yl-p-(dimethylamino)benzylidene]-2,5-cyclohexadien-1-ylidene]dimethyl-, perchlorate Ammonium, [4-[p-(dimethylamino)-α-(5-methoxy-2,1,3-benzothiadiazol-4-yl)benzylidene]-2,5-cyclohexadien-1-ylideneldimethyl-, perchlorate Ammonium, [4-[p-(diethylamino)-α-(5-methoxy-2,1,3-benzothiadiazol-4-yl)benzylidene]-2,5-cyclohexadien-1-ylidene]diethyl-, perchlorate Ammonium, [4-[p-(diethylamino)-α-(7-methoxy-2,1,3-benzothiadiazol-4-yl)benzylidene]-2,5-cyclohexadien-1-ylidene]diethyl-, perchlorate Ammonium, [4-[p-(dimethylamino)-α-(7-methoxy-2,1,3-benzothiadiazol-4-yl)benzylidene]-2,5-cyclohexadien-1-ylideneldimethyl-, perchlorate Ammonium, [4-[p-(diethylamino)-α-(7-methyl-2,1,3-benzothiadiazol-4-yl)benzylidene]-2,5-cyclohexadien-1-ylidene]diethyl-, perchlorate Ammonium, [4-[p-(dimethylamino)-α-(7-methyl-2,1,3-benzothiadiazol-4-yl)benzylidene]-2,5-cyclohexadien-1-ylidene]dimethyl-, perchlorate Ammonium, [4-[α-2,1,3-benzothiadiazol-4-yl-p-(diethylamino)benzylidene]-2,5-cyclohexadien-1-ylidene]diethyl-, perchlorate Ammonium,[4-[α-2,1,3-benzothiadiazol-5-yl-p-(diethylamino)benzylidene]-2,5-cyclohexadien-1-ylidene]diethyl-, perchlorate Ammonium, [4-[α,-2,1,3-benzothiadiazol-5-yl-p-(dimethylamino)benzylidene]-2,5-cyclohexadien-1-ylidene]dimethyl-, perchlorate Ammonium, [4-[α-(1-methyl-2-phenylindol-3-yl)benzylidene]-2,5-cyclohexadien-1-ylidene]phenyl-, trichlorozincate(1-) Methyl[4-[α-(1-methyl-2-phenylindol-3-yl)benzylidene]-2,5-cyclohexadien-1-ylidene]phenylammonium trichlorozincate Dimethyl[4-[α-(1-methyl-2-phenylindol-3-yl)benzylidene]-2,5-cyclohexadien-1-ylidene]-ammonium trichlorozincate, Dimethyl[4-[α-(1-methyl-2-phenylindol-3-yl)benzylidene]-2,5-cyclohexadien-1-ylidene]ammonium trichlorozincate Indole, 3-[α-(4-imino-2,5-cyclohexadien-1-ylidene)benzyl]-1,2-dimethyl-, monohydrochloride Indole, 3-[α-[4-[(p-ethoxyphenyl)imino]-2,5-cyclohexadien-1-ylidene]benzyl]-1-methyl-2-phenyl-, monohydrochloride Ammonium, [4-[α-[9-(2-cyanoethyl)carbazol-3-yl]-p-(dimethylamino)benzylidene]-2,5-cyclohexadien-1-ylidene]dimethylAmmonium, [4-[α-carbazol-3-yl-p-(dimethylamino)benzylidene]-2,5-cyclohexadien-1-ylidene]dimethylAmmonium, [4-[α-[9-(2-cyanoethyl)carbazol-3-yl]benzylidene]-2,5-cyclohexadien-1-ylidene] dimethylAmmonium, [4-(α-carbazol-3-ylbenzylidene)-2,5-cyclohexadien-1-ylidene]dimethyl-2,5-Cyclohexadien-1-one, 2,6-di-tert-butyl-4-(α-morpholinobenzylidene)-2,5-Cyclohexadien-1-one, 2,6-di-tert-butyl-4-[α-(2,2-dimethyl-1-aziridinyl)benzylidene]-2,5-Cyclohexadien-1-one, 4-(α-1-aziridinylbenzylidene)-2,6-di-tert-butyl-2,5-Cyclohexadien-1-one, 2,6-bis(1,1-dimethylethyl)-4-(phenyl-1-piperidinylmethylene)-2,5-Cyclohexadien-1-one, 2,6-di-tert-butyl-4-(α-piperidinobenzylidene)-Ammonium, [4-[α-[4-(diethylamino)-o-tolyl]piperonyl]-3-methyl-2,5-cyclohexadien-1-ylidene]diethyl-, chloride Ammonium, [4-[α-[4-(diethylamino)-o-tolyl]-2-thenylidene]-3-methyl-2,5-cyclohexadien-1-ylidene]diethyl-, chloride Ammonium, [4-[α-[p-(dimethylamino)phenyl]-5-nitrofurfurylidene]-2,5-cyclohexadien-1-ylidene]dimethyl-, chloride Ammonium, [4-[5-bromo-α-[p-(diethylamino)phenyl]furfurylidene]-2,5-cyclohexadien-1-ylidene]diethyl-, chloride Ammonium, [4-[5-bromo-α-[p-(dimethylamino)phenyl]furfurylidene]-2,5-cyclohexadien-1-ylidene]dimethyl-, chloride Ammonium, [4-[α-[p-(dimethylamino)phenyl]-2-furfurylidene]-2,5-cyclohexadien-1-ylidene]diethyl-, chloride 2-hydroxy-5-[[4-[[4-[(4-hydroxy-3-sulphophenyl)amino]phenyl](1-methyl-2-phenyl-1H-indol-3-yl)methylene]-2,5-cyclohexadien-1-ylidene]amino]-, Ammonium, [4-[α-12H-benzo[a]phenoxazin-5-yl-p-(dimethylamino)benzylidene]-2,5-cyclohexadien-1-ylidene]dimethyl-, chloride, compound with zinc chloride [4-[α-12H-benzo[a]phenoxazin-5-yl-p-(dimethylamino)benzylidene]-2,5-cyclohexadien-1-ylidene]dimethylammonium chloride Benzenamine, N-2-dimethyl-4-[(2-methyl-1H-indol-3-yl)[3-methyl-4-(methylimino)-2,5-cyclohexadien-1-ylidene]methyl]-, monohydrochloride Indole, 2-methyl-3-[3-methyl-4-(methylamino)-α-[3-methyl-4-(methylimino)-2,5-cyclohexadien-1-ylidene]benzyl]-, monohydrochloride Methanaminium, N-[4-[[4-(dimethylamino)phenyl]-2-furanylmethylene]-2,5-cyclohexadien-1-ylidene]-N-methyl-, chloride Ammonium, [4-[α-[p-(dimethylamino)phenyl]furfurylidene]-2,5-cyclohexadien-1-ylidene]dimethyl-, chloride Benzenaminium, N-[4-[[2-(4-chlorophenyl)-4,6-dimethyl-1-(2-methylpropyl)-1H-indol-3-yl](2,4-disulphophenyl)methylene]-2,5-cyclohexadien-1-ylidene]-4-methoxy-N-methyl-, Ammonium, [4-[α-[2-(p-chlorophenyl)-1-isobutyl-4,6-dimethylindol-3-yl]-2,4-disulphobenzylidene]-2,5-cyclohexadien-1-ylidene](p-methoxyphenyl)methyl-, hydroxide, 2-ethoxy-5-[[4-[[4-[(4-ethoxy-3-sulphophenyl)amino]phenyl](1-methyl-2-phenyl-1H-indol-3-yl)methylene]-2,5-cyclohexadien-1-ylidene]amino]-, 5-[(3-carboxy-5-methyl-4-oxo-2,5-cyclohexadien-1-ylidene)(4,5-dihydro-3-methyl-5-oxo-1-phenyl-1H-pyrazol-4-yl)methyl]-2-hydroxy-3-methyl-5-[[4-[[2-(4-chlorophenyl)-4,6-dimethyl-1-(2-methylpropyl)-1H-indol-3-yl][4-[(4-ethoxy-3-sulphophenyl)amino]phenyl]methylene]-2,5-cyclohexadien-1-ylidene]amino]-2-ethoxy-, N-[α-[2-(p-chlorophenyl)-1-isobutyl-4,6-dimethylindol-3-yl]-α-[4-[(4-ethoxy-3-sulphophenyl)imino]-2,5-cyclohexadien-1-ylidene]-p-tolyl]-6-ethoxy-, 6-[(4-ethoxyphenyl)imino]-3-[(1-methyl-2-phenyl-1H-indol-3-yl)[4-[(2-sulphoethyl)amino]phenyl]methylene]-, 6-[(p-ethoxyphenyl)imino]-3-[α-(1-methyl-2-phenylindol-3-yl)-p-[(2-sulphoethyl)amino]benzylidene]-, Methanaminium, N-[4-[[4-(dimethylamino)phenyl]-3-thienylmethylene]-2,5-cyclohexadien-1-ylidene]-N-methyl-, perchlorate [4-[α-[p-(Dimethylamino)phenyl]-3-thenylidene]-2,5-cyclohexadien-1-ylidene]dimethylammonium perchlorate Ammonium, 4-[α-[p-(dimethylamino)phenyl]furfurylidene]-2,5-cyclohexadien-1-ylidene]dimethyl-, bromide [4-[α-[p-(Dimethylamino)phenyl]furfurylidene]-2,5-cyclohexadien-1-ylidene]dimethylammonium bromide Methanaminium, N-[4-[[4-(dimethylamino)phenyl]-3-pyridinylmethylene]-2,5-cyclohexadien-1-ylidene]-N-methyl-, perchlorate Ammonium, [4-[p-(dimethylamino)-α-3-pyridylbenzylidene]-2,5-cyclohexadien-1-ylidene]dimethyl-, perchlorate [4-[p-(Dimethylamino)-α-3-pyridylbenzylidene]-2,5-cyclohexadien-1-ylidene]dimethylammonium perchlorate Ammonium, [4-[p-(dimethylamino)-α-4-pyridylbenzylidene]-2,5-cyclohexadien-1-ylidene]dimethyl-, perchlorate [4-[p-(Dimethylamino)-α-4-pyridylbenzylidene]-2,5-cyclohexadien-1-ylidene]dimethylammonium perchlorate Ammonium, [4-[p-(dimethylamino)-α-2-pyridylbenzylidene]-2,5-cyclohexadien-1-ylidene]dimethyl-, chloride [4-[p-(Dimethylamino)-α-2-pyridylbenzylidene]-2,5-cyclohexadien-1-ylidene] dimethylammonium chloride,
and addition salts thereof.

6. The composition according to any one of the preceding claims, **characterised in that** the compound(s) of formula (I), (Ibis), (II) or (IIBis) and/or the addition salt(s) account(s) for 0.0001 to 10% by weight with respect to the total weight of the dyeing composition, preferably from 0.005 to 10% by weight and more preferably from 0.01 to 6% by weight with respect to the total weight of the dyeing composition.

7. The composition according to any one of claims 1 to 6, **characterised in that** it further contains one or more direct dye(s) different from the compounds of formulae (I), (Ibis), (II) and (IIbis).

8. The composition according to claim 7, **characterised in that** the direct dye(s) different from the compounds of formula (I), (Ibis), (II) or (IIbis) are selected from neutral, acidic or cationic direct, nitrated benzene dyes, neutral, acidic or cationic direct azo dyes, direct quinone dyes and particularly neutral, acidic or cationic anthraquinone dyes, direct azine dyes, direct triarylmethane dyes, direct indoamino dyes, and direct natural dyes.

9. The composition according to any one of claims 7 and 8, **characterised in that** the direct dye(s) different from the compounds of formulae (I), (Ibis), (II) or (IIbis) account for 0.001 to 20% by weight, preferably from 0.005 to 10% by weight with respect to the total weight of the dyeing composition.

10. The composition according to any one of the preceding claims, **characterised in that** the composition further contains one or more oxidation base(s) and possibly one or more coupling agent(s).

11. The composition according to claim 10, **characterised in that** the oxidation base(s) is (are) selected from paraphenylenediamines, bisphenylalkylenediamines, para-aminophenols, ortho-aminophenols, heterocyclic bases, and addition salts thereof.

12. The composition according to claim 10, **characterised in that** the coupling agent(s) are selected from metaphenylenediamine coupling agents, meta-aminophenol coupling agents, metadiphenol coupling agents, naphthalenic coupling agents, heterocyclic coupling agents and addition salts thereof.

13. The composition according to any one of claims 10 to 12, **characterised in that** the oxidation base(s) and/or the coupling agent(s) is (are) each present in an amount of 0.001 to 10% by weight, preferably from 0.005 to 6% by weight with respect to the total weight of the dyeing composition.

14. The composition according to any one of the preceding claims, **characterised in that** the composition has a pH from 3 to 12, preferably from 5 to 11, more preferably from 6 to 8.5.

15. A method for directly dyeing keratinous fibres, especially human keratinous fibres, such as hair, comprising applying at least one dyeing composition according to any one of claims 1 to 14 containing at least one compound of formula (I), (Ibis), (II) or (IIbis) onto the keratinous fibres, then observing a waiting time, and then rinsing the fibres.

16. The method according to claim 15, **characterised in that** the waiting time is from 3 to 50 minutes, preferably from 5 to 30 minutes.

17. A method for dyeing keratinous fibres and, particularly, human keratinous fibres, such as hair, wherein at least one dyeing composition comprising either at least one compound of formula (II) or (IIbis), or at least one compound of formula (I) or (Ibis), and at least one oxidation base and possibly one or more coupling agent(s) are applied on said fibres, the colour being developed at acidic, neutral or alkaline pH, using an oxidation agent.

18. A method according to claim 17, **characterised in that** the oxidation agent is selected from hydrogen peroxide, urea peroxide, alkali metal bromates, persalts such as perborates and persulphates, peracids and oxidase enzymes, such as peroxidases, 2 electron-redox agents, such as unicases, and 4 electron-oxigenases such as laccases.

19. A device having several compartments including a first compartment enclosing a dyeing composition according to any one of claims 1 to 14, comprising either at least one compound of formula (II) or (IIbis), or at least one compound of formula (I) or (Ibis) and at least one oxidation base, and possibly one or more coupling agents, and a second compartment enclosing an oxidising composition.

## Patentansprüche

1. Zusammensetzung zum Färben von Keratinfasern umfassend in einem zum Färben geeigneten kosmetischen Medium wenigstens ein Färbemittel, das aus den der folgenden Formel (I), (Ibis), (II) oder (IIbis) entsprechenden Verbindungen oder ihren Tautomeren und ihren Additionssalzen ausgewählt ist: 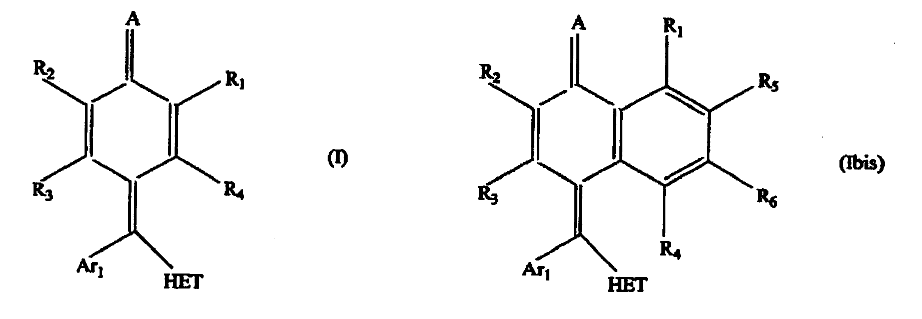 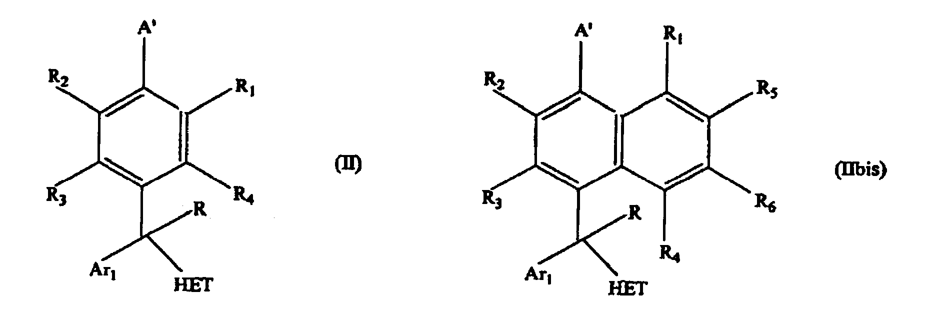 worin
- Ar₁ einen Arylrest wie Phenyl oder Naphthyl darstellt, der gegebenenfalls durch eine oder mehrere Gruppen Z substituiert ist;
- HET einen gegebenenfalls durch eine oder mehrere Gruppen Z' substituierten Heterocyclus darstellt;
- A O, NH, N-Alkyl, N-Hydroxyalkyl, Ammonium, N-Alkylammonium, N-Hydroxyalkylammonium, N,N-Dialkylammonium, N,N-Di(hydroxyalkyl)ammonium oder N-(Hydroxyalkyl)-N-(alkyl)-ammonium darstellt, wobei die beiden Alkylgruppen mit dem Stickstoffatom, an das sie gebunden sind, einen Ring bilden können, der durch ein oder mehrere Stickstoff-, Sauerstoff-oder Schwefelatome unterbrochen sein kann.
- R₁ bis R₆ und Z, Z' unabhängig voneinander eine Gruppe bezeichnen, die aus einem Wasserstoffatom, Halogenatomen wie F, Cl, Br und I, den Resten -NHSO₃H, Hydroxy, Alkyl, Alkoxy, Alkylthio, Monoalkylamino, Dialkylamino, wobei die beiden Alkylgruppen zusammen mit dem Stickstoffatom, an das sie gebunden sind, einen Ring bilden können, der durch ein oder mehrere Stickstoff-, Sauerstoff- oder Schwefelatome unterbrochen sein kann, Heterocyclen und den Resten Nitro, Aryl, Acyl, Alkoxycarbonyl, Acylamino(carboxamido), Cyano, -CO₂H, -SO₃H, -PO₃H₂ und -PO₄H₂ ausgewählt sind,
wobei
- A' ein Wasserstoffatom, eine Hydroxygruppe, Amino, (Hydroxyalkyl)amino, Monoalkylamino, Di(hydroxyalkyl)amino, (Hydroxyalkyl)(alkyl)amino oder (Dialkyl)amino darstellt, wobei die beiden Alkylgruppen mit dem Stickstoffatom, an das sie gebunden sind, einen Ring bilden können, der durch ein oder mehrere Stickstoff-, Sauerstoff- oder Schwefelatome unterbrochen sein kann,
- R ein Wasserstoff- oder Halogenatom, einen Hydroxyrest, einen Alkoxyrest oder einen Alkylthiorest mit Ausnahme von Bis(4-hydroxyphenyl)-(chinolyl-2)-methan-hydrochlorid und Bis(4-hydroxyphenyl)-(chinolyl-2)-methan darstellt.

2. Zusammensetzung gemäß Anspruch 1, wobei HET einen ungesättigten Heterocyclus darstellt, der gegebenenfalls durch eine oder mehrere Gruppen Z' substituiert ist.

3. Zusammensetzung gemäß Anspruch 2, wobei HET einen aromatischen Heterocyclus darstellt, der gegebenenfalls durch eine oder mehrere Gruppen Z' substituiert ist.

4. Zusammensetzung gemäß einem der Ansprüche 1 bis 3, wobei die Heterocyclen, insbesondere die von HET, aus den folgenden Heterocyclen ausgewählt sind: Thiophen, Benzothiophen, Furan, Benzofuran, Indol, Indolin, Carbazol, Pyridin, Dehydrochinolin, Chromon, Julodinin, Thiadiazol, Triazol, Isoxazol, Oxazol, Thiazol, Isothiazol, Imidazol, Pyrazol, Triazin, Thiazin, Pyrazin, Pyridazin, Pyrimidin, Pyridin, Diazepin, Oxazepin, Benzotriazol, Benzoxazol, Benzimidazol, Benzothiazol, Morpholin, Piperidin, Piperazin, Azetidin, Pyrrolidin, Aziridin.

5. Färbezusammensetzung gemäß Anspruch 1, **dadurch gekennzeichnet, dass** sie eine Verbindung der Formel (I), (Ibis), (II) oder (IIbis) umfasst, die aus den folgenden Verbindungen ausgewählt sind, für die die Gegenionen angegeben oder nicht angegeben sind:
N-[4-[[4-(Dimethylamino)phenyl](2,2-dimethyl-2H-1-benzopyran-3-yl)methylen]-2,5-cyclohexadien-1-yliden]-N-methyl-methanaminiumacetat,
N-[4-[[4-(Dimethylamino)phenyl](2,2-dimethyl-2H-1-benzopyran-3-yl)methylen]-2,5-cyclohexadien-1-yliden]-N-methyl-methanaminium,
N-[4-[[4-(Dimethylamino)phenyl](2,2-dimethyl-2H-1-benzopyran-5-yl)methylen]-2,5-cyclohexadien-1-yliden]-N-methyl-methanaminiumacetat,
N-[4-[[4-(Dimethylamino)phenyl](2,2-dimethyl-2H-1-benzopyran-5-yl)methylen]-2,5-cyclohexadien-1-yliden]-N-methyl-methanaminium,
N-[4-[[4-(Dimethylamino)phenyl](2,2-dimethyl-2H-1-benzopyran-4-yl)methylen]-2,5-cyclohexadien-1-yliden]-N-methyl-methanaminiumacetat,
N-[4-[[4-(Dimethylamino)phenyl](2,2-dimethyl-2H-1-benzopyran-4-yl)methylen]-2,5-cyclohexadien-1-yliden]-N-methyl-methanaminium,
N-[4-[[4-(Dimethylamino)phenyl](2,2-dimethyl-2H-1-benzopyran-8-yl)methylen]-2,5-cyclohexadien-1-yliden]-N-methyl-methanaminiumacetat,
N-[4-[[4-(Dimethylamino)phenyl](2,2-dimethyl-2H-1-benzopyran-8-yl)methylen]-2,5-cyclohexadien-1-yliden]-N-methyl-methanaminium,
N-[4-[[4-(Dimethylamino)phenyl](2,2-dimethyl-2H-1-benzopyran-7-yl)methylen]-2,5-cyclohexadien-1-yliden]-N-methyl-methanaminiumacetat,
N-[4-[[4-(Dimethylamino)phenyl](2,2-dimethyl-2H-1-benzopyran-7-yl)methylen]-2,5-cyclohexadien-1-yliden]-N-methyl-methanaminium,
N-[4-[[4-(Dimethylamino)phenyl](2,2-dimethyl-2H-1-benzopyran-6-yl)methylen]-2,5-cyclohexadien-1-yliden]-N-methyl-methanaminiumacetat,
N-[4-[[4-(Dimethylamino)phenyl](2,2-dimethyl-2H-1-benzopyran-6-yl)methylen]-2,5-cyclohexadien-1-yliden]-N-methyl-methanaminium,
4-[[4-Amino-3,5-bis(1-methylethyl)phenyl](4-ethyl-3,4-dihydro-2H-1,4-benzoxazin-7-yl)-methylen]-2,6-dimethoxy-2,5-cyclohexadien-1-on,
4-[[3,4-Dihydro-4-(2-pyridinylmethyl)-2H-1,4-benzoxazin-7-yl](4-ethyl-3,4-dihydro-2H-1,4-benzoxazin-7-yl)methylen]-2,6-dimethoxy-2,5-cyclohexadien-1-on,
4-[[4-(Dimethylamino)phenyl](4-ethyl-3,4-dihydro-2H-1,4-benzoxazin-7-yl)methylen]-2,6-dimethoxy-2,5-cyclohexadien-1-on,
2,6-Bis(1,1-dimethylethyl)-4-[(5-ethyl-2-thienyl)phenylmethylen]-2,5-cyclohexadien-1-on,
N-[3-Carboxy-4-[[2-carboxy-4-[ethyl(2-methoxyethyl)amino]phenyl](7-carboxy-4-oxo-4H-1-benzothiopyran-3-yl)methylen]-2,5-cyclohexadien-1-yliden]-N-ethyl-2-methoxyethanaminium,
N-[4-[[4-[Bis(2-methoxyethyl)amino]-2-carboxyphenyl](5-carboxy-2-pyridinyl)methylen]-3-carboxy-2,5-cyclohexadien-1-yliden]-2-methoxy-N-(2-methoxyethyl)-ethanaminium,
N-[4-[3-Benzofuranyl[2-carboxy-4-[ethyl[2-(methylsulfonyl)amino]ethyl]amino]phenyl]methylen]-3-carboxy-2,5-cyclohexadien-1-yliden]-N-ethyl-2-[(methylsulfonyl)amino]ethanaminium-perchlorat,
N-[4-[3-Benzofuranyl[2-carboxy-4-[ethyl[2-[(methylsulfonyl)amino]ethyl]amino]phenyl]methylen]-3-carboxy-2,5-cyclohexadien-1-yliden]-N-ethyl-2-[(methylsulfonyl)amino]ethanaminium,
N-[4-[[4-[Bis(2-methoxyethyl)amino]-2-carboxyphenyl](4-oxo-4H-1-benzothiopyran-3-yl)methylen]-3-carboxy-2,5-cyclohexadien-1-yliden]-2-methoxy-N-(2-methoxyethyl)-ethanaminium-acetat,
N-[4-[[4-[Bis(2-methoxyethyl)amino]-2-carboxyphenyl](4-oxo-4H-1-benzothiopyran-3-yl)methylen]-3-carboxy-2,5-cyclohexadien-1-yliden]-2-methoxy-N-(2-methoxyethyl)-ethanaminium, 5-[(3-Carboxy-4-oxo-2,5-cyclohexadien-1-yliden)-3-pyridinylmethyl]-2-hydroxybenzoesäure-salz,
N-[2-Carboxy-4-[[3-carboxy-4-(dimethylamino)phenyl](4-oxo-4H-1-benzothiopyran-3-yl)methylen]-2,5-cyclohexadien-1-yliden]-N-methyl-methanaminiumperchlorat,
N-[2-Carboxy-4-[[3-carboxy-4-(dimethylamino)phenyl](4-oxo-4H-1-benzothiopyran-3-yl)methylen]-2,5-cyclohexadien-1-yliden]-N-methyl-methanaminium,
5-[(3-Carboxy-4-oxo-2,5-cyclohexadien-1-yliden)[3,5-dimethyl-1-[2-[(methylsulfonyl)amino]-ethyl]-1H-pyrazol-4-yl]methyl]-2-hydroxybenzoesäuresalz,
5-[[2-[Bis(2-methoxyethyl)amino]-5-thiazolyl](3-carboxy-4-oxo-2,5-cyclohexadien-1-yliden)-methyl]-2-hydroxybenzoesäuresalz,
N-[4-[(7-Carboxy-2-oxo-2H-1-benzopyran-4-yl)[4-(diethylamino)phenyl]methylen]-2,5-cyclohexadien-1-yliden]-N-ethyl-ethanaminiumperchlorat,
N-[4-[(7-Carboxy-2-oxo-2H-1-benzopyran-4-yl)[4-(diethylamino)phenyl]methylen]-2,5-cyclohexadien-1-yliden]-N-ethyl-ethanaminium,
N-[4-[2-Benzofuranyl[4-[ethyl[2-[(methylsulfonyl)amino]ethyl]amino]phenyl]methylen]-2,5-cyclohexadien-1-yliden]-N-ethyl-2-[(methylsulfonyl)amino]ethanaminiumperchlorat,
N-[4-[2-Benzofuranyl[4-[ethyl[2-[(methylsulfonyl)amino]ethyl]amino]phenyl]methylen]-2,5-cyclohexadien-1-yliden]-N-ethyl-2-[(methylsulfonyl)amino]ethanaminium,
N-[4-[(6-Carboxybenzo[b]thien-2-yl)[4-(diethylamino)phenyl]methylen]-2,5-cyclohexadien-1-yliden]-N-ethyl-ethanaminiumtetrafluorborat(1-),
N-[4-[(6-Carboxybenzo[b]thien-2-yl)[4-(diethylamino)phenyl]methylen]-2,5-cyclohexadien-1-yliden]-N-ethyl-ethanaminium,
N-[4-[[5-[Bis(2-methoxyethyl)amino]-2-furanyl][4-[ethyl[2-(methylsulfonyl)amino]ethyl]amino]-phenyl]methylen]-2,5-cyclohexadien-1-yliden]-N-ethyl-2-[(methylsulfonyl)amino]ethan-aminiumperchlorat,
N-[4-[[5-[Bis(2-methoxyethyl)amino]-2-furanyl][4-[ethyl[2-[(methylsulfonyl)amino]ethyl]amino]-phenyl]methylen]-2,5-cyclohexadien-1-yliden]-N-ethyl-2-[(methylsulfonyl)amino]ethanaminium, 4-[(4-Hydroxyphenyl)-4-chinolinylmethylen]-2,5-cyclohexadien-1-on,
N-[4-[[4-[Bis(2-methoxyethyl)amino]phenyl](7-carboxy-4-oxo-4H-1-benzopyran-3-yl)methylen]-2,5-cyclohexadien-1-yliden]-2-methoxy-N-(2-methoxyethyl)ethanaminiumsulfat (1:1),
N-[4-[[4-[Bis(2-methoxyethyl)amino]phenyl](7-carboxy-4-oxo-4H-1-benzopyran-3-yl)methylen]-2,5-(cyclohexadien-1-yliden]-2-methoxy-N-(2-methoxyethyl)ethanaminium,
N-[4-[(5-Carboxy-2-pyridinyl)[4-(dimethylamino)phenyl]methylen]-2,5-cyclohexadien-1-yliden]-N-methyl-methanaminiumperchlorat,
N-[4-[(5-Carboxy-2-pyridinyl)[4-(dimethylamino)phenyl]methylen]-2,5-cyclohexadien-1-yliden]-N-methyl-methanaminium,
5-[(4-Hydroxyphenyl)(4-oxo-2,5-cyclohexadien-1-yliden)methyl]thiophen-2-carbonsäure, 4-[2-Furanyl(4-hydroxyphenyl)methylen]-2,5-cyclohexadien-1-on,
4-[(4-Hydroxyphenyl)-2-pyridinylmethylen]-2,5-cyclohexadien-1-on,
N-[3-Carboxy-4-[[2-carboxy-4-(dimethylamino)phenyl](4-oxo-4H-1-benzopyran-3-yl)methylen]-2,5-cyclohexadien-1-yliden]-N-methyl-methanaminiumperchlorat,
N-[3-Carboxy-4-[[2-carboxy-4-(dimethylamino)phenyl](4-oxo-4H-1-benzopyran-3-yl)methylen]-2,5-cyclohexadien-1-yliden]-N-methyl-methanaminium,
N-[4-[(5-Carboxy-2-thienyl)[4-(diethylamino)phenyl]methylen]-2,5-cyclohexadien-1-yliden]-N-ethyl-ethanaminiumperchlorat,
N-[4-[(5-Carboxy-2-thienyl)[4-(diethylamino)phenyl]methylen]-2,5-cyclohexadien-1-yliden]-N-ethyl-ethanaminium,
4-[(4-Methoxyphenyl)-3,6,9,12,15-pentaoxabicyclo[15.3.1]heneicosa-1(21),17,19-trien-21-ylmethylen]-2,5-cyclohexadien-1-on,
3,6,9,12,15-Pentaoxabicyclo[15.3.1]heneicosan, 2,5-Cyclohexadien-1-on,
N-[4-[(2,3-Dihydro-3-methyl-2-benzothiazolyl)[4-(dimethylamino)phenyl]methylen]-2,5-cyclohexadien-1-yliden]-N-methyl-methanaminiumchlorid,
[4-(α-Benzo[b]thien-2-yl-p-dimethylaminobenzyliden)-2,5-cyclohexadien-1-yliden]dimethyl-ammoniumchlorid,
[4-(α-Benzo[b]thien-3-yl-p-dimethylaminobenzyliden)-2,5-cyclohexadien-1-yliden]dimethyl-ammoniumchlorid,
N-[4-[[9-(Dimethylamino)benzo[a]phenoxazin-7-ium-5-yl][4-dimethylamino)phenyl]methylen]-2,5-cyclohexadien-1-yliden]-N-methyl-methanaminium-bis[tetraphenylborat(1-)], Benzo[a]phenoxazin-7-iummethanaminium,
N-[4-[[9-(Dimethylamino)benzo[a]phenoxazin-7-ium-5-yl][4-(dimethylamino)phenyl]methylen]-2,5-cyclohexadien-1-yliden]-N-methyl-methanaminium,
[4-(α-3-Brombenzo[b]thien-2-yl-p-dimethylamonobenzyliden)-2,5-cyclohexadien-1-yliden]-dimethylammoniumchlorid,
[4-(α-3-Chlorbenzo[b]thien-2-yl-p-dimethylaminobenzyliden)-2,5-cyclohexadien-1-yliden]-dimethylammoniumchlorid,
[4-(p-Dimethylamino-a-3-methylbenzo[b]thien-2-ylbenzyliden)-2,5-cyclohexadien-1-yliden]-dimethylammoniumchlorid,
[4-[2-Brom-α-(p-dimethylaminophenyl)-3-thenyliden]-2,5-cyclohexadien-1-yliden]dimethyl-ammoniumchlorid,
[4-[2-Chlor-α-(p-dimethylaminophenyl)-3-thenyliden]-2,5-cyclohexadien-1-yliden]dimethyl-ammoniumchlorid,
[4-[α-(p-Dimethylaminophenyl)-3-thenyliden]-2,5-cyclohexadien-1-yliden]dimethylammonium-chlorid,
[4-[α-(p-Dimethylaminophenyl)-2,5-dimethyl-3-thenyliden]-2,5-cyclohexadien-1-yliden]-dimethylammoniumchlorid,
1-[[4-(Dimethylamino)phenyl][4-(dimethyliminio)-2,5-cyclohexadien-1-yliden]methyl]pyridinium-bis[tetrafluorborat(1-)],
2,3,5,6-Tetrachlor-4-[(pentachlorphenyl)(2,3,5,6-tetrachlor-4-pyridinyl)methylen]-2,5-cyclohexadien-1-on,
N-[4-[[4-(Diethylamino)phenyl](4-oxo-4H-1-benzothiopyran-3-yl)methylen]-2,5-cyclohexadien-1-yliden]-N-ethyl-ethanaminiumperchlorat,
N-[4-[[4-(Diethylamino)phenyl](4-oxo-4H-1-benzothiopyran-3-yl)methylen]-2,5-cyclohexadien-1-yliden]-N-ethyl-ethanaminium,
4H-1-Benzothiopyranethanaminiumderiv.,
N-Methyl-N-[4-[[4-methylphenylamino)phenyl](4-oxo-4H-1-benzothiopyran-3-yl)methylen]-2,5-cyclohexadien-1-yliden]benzolaminiumperchlorat,
N-Methyl-N-[4-[[4-methylphenylamino)phenyl](4-oxo-4H-1-benzothiopyran-3-yl)methylen]-2,5-cyclohexadien-1-yliden]benzolaminium,
N-[4-[[4-(Diethylamino)-2-methylphenyl](4-oxo-4H-1-benzothiopyran-3-yl)methylen]-3-methyl-2,5-cyclohexadien-1-yliden]-N-ethyl-ethanaminiumperchlorat,
N-[4-[[4-(Diethylamino)-2-methylphenyl](4-oxo-4H-1-benzothiopyran-3-yl)methylen]-3-methyl-2,5-cyclohexadien-1-yliden]-N-ethyl-ethanaminium,
4H-1-Benzothiopyran-ethanaminium,
N-[4-[[4-(Dimethylamino)phenyl][5-(4-iodphenyl)-2-furanyl]methylen]-2,5-cyclohexadien-1-yliden]-N-methyl-methanaminiumchlorid,
N-[4-[[5-(4-Bromphenyl)-2-furanyl][4-(dimethylamino)phenyl]methylen]-2,5-cyclohexadien-1-yliden]-N-methyl-methanaminiumchlorid,
N-[4-[[4-(Dimethylamino)phenyl][5-(4-nitrophenyl)-2-furanyl]methylen]-2,5-cyclohexadien-1-yliden]-N-methyl-methanaminiumchlorid,
[Oxybis[methylen-5,2-furandiyl[p-(N-methylanilino)benzylidin]-2,5-cyclohexadien-4,1-diyliden]]-bis[methylphenylammoniumchlorid],
[Oxybis[methylen-5,2-furandiyl[p-(N-methylanilino)benzylidin]-2,5-cyclohexadien-4,1-diyliden]]-bis[methylphenylammoniumhydrogensulfat],
N,N'-[Oxybis[methylen-5,2-furandiyl[[4-(methylphenylamino)phenyl]methylidin]-2,5-cyclohexadien-4,1-diyliden]]bis[N-methyl]benzolaminium,
3-[4-Anilino-α-(1-methyl-2-phenylindol-3-yl)-3-sulfobenzyliden]-6-phenylimino-1,4-cyclohexadien-1-sulfonsäuresalz,
1-[[4-(Dimethylamino)phenyl][4-(dimethyliminio)-2,5-cyclohexadien-1-yliden]methyl]pyridinium-trifluormethansulfonsäuresalz,
1-[[4-(Dimethylamino)phenyl][4-(dimethyliminio)-2,5-cyclohexadien-1-yliden]methyl]pyridinium-trifluormethansulfonsäuresalz,
1-[[4-(Dimethylamino)phenyl][4-(dimethyliminio)-2,5-cyclohexadien-1-yliden]methyl]pyridinium,
[4-[p-(Dimethylamino)-α-(9-methylcarbazol-3-yl)benzyliden]-2,5-cyclohexadien-1-yliden]-dimethylammoniumchlorid,
[4-[α-[p-(Dimethylamino)phenyl]furfuryliden]-2,5-cyclohexadien-1-yliden]dimethylammonium-chlorid,
N-[4-[Bis(1,2,3,4,10,14b-hexahydro-2-methyldibenzo[c,f]pyrazino[1,2-a]azepin-8-yl)methylen]-2,5-cyclohexadien-1-yliden]-N-methyl-methanaminiumperchlorat-diperchlorat,
N-[4-[Bis(1,2,3,4,10,14b-hexahydro-2-methyldibenzo[c,f]pyrazino[1,2-a]azepin-8-yl)methylen]-2,5-cyclohexadien-1-yliden]-N-methyl-methanaminiumperchlorat,
N-[4-[Bis(1,2,3,4,10,14b-hexahydro-2-methyldibenzo[c,f]pyrazino[1,2-a]azepin-8-yl)methylen]-2,5-cyclohexadien-1-yliden]-N-methyl-1H-indolizinium-methanaminium,
3-[[4-(Dimethyliminio)-2,5-cyclohexadien-1-yliden](4-methoxyphenyl)methyl]-2-(2-hydroxy-5-methylphenyl)-1-methyl-1 H-indolizinium,
3-[[4-(Dimethyliminio)-2,5-cyclohexadien-1-yliden]phenylmethyl]-2-(2-hydroxy-5-methylphenyl)-1-methyl-1 H-indolizinium,
3-[[4-(Dimethylamino)phenyl][4-(dimethyliminio)-2,5-cyclohexadien-1-yliden]methyl]-2-(2-hydroxy-5-methylphenyl)-1-methyl-1 H-indolizinium,
3-[[4-(Dimethylamino)phenyl][4-(dimethyliminio)-2,5-cyclohexadien-1-yliden]methyl]-2-(2-hydroxy-5-methylphenyl)-1-methyl-1 H-indolizinium,
inneres Salz des N-[4-[2-Benzofuranyl[3-chlor-4-[ethyl(3-sulfopropyl)amino]phenyl]methylen]-2-chlor-2,5-cyclohexadien-1-yliden]-N-ethyl-3-sulfopropan-1-aminium-natriumsalzes,
N-[4-[2-Benzofuranyl[4-[ethyl(3-sulfopropyl)amino]-3-methylphenyl]methylen]-2-methyl-2,5-cyclohexadien-1-yliden]-N-ethyl-3-(propan-1-aminium),
N-[4-[2-Benzofuranyl[4-[ethyl(4-sulfobutyl)amino]phenyl]methylen]-2,5-cyclohexadien-1-yliden]-N-ethyl-4-(butan-1-aminium),
N-[4-[(5-Chlorbenzo[b]thien-2-yl)[4-[methyl(3-sulfopropyl)amino]phenyl]methylen]-2,5-cyclohexadien-1-yliden]-N-methyl-3-sulfo-propan-1-aminium,
N-[4-[(3-Chlorbenzo[b]thien-2-yl)[4-[methyl(3-sulfopropyl)amino]phenyl]methylen]-2,5-cyclohexadien-1-yliden]-N-methyl-3-sulfo-propan-1-aminium-natriumsalz,
N-[4-[Benzo[b]thien-2-yl[4-[methyl(3-sulfopropyl)amino]phenyl]methylen]-2,5-cyclohexadien-1-yliden]-N-methyl-3-sulfo-propan-1-aminium-natriumsalz,
N-Methyl-N-[4-[(3-methylbenzo[b]thien-2-yl)[4-[methyl(3-sulfopropyl)amino]phenyl]methylen]-2,5-cyclohexadien-1-yliden]-3-sulfo-propan-1-aminium-natriumsalz,
N-[4-[(3-Brombenzo[b]thien-2-yl)[4-[methyl(3-sulfopropyl)amino]phenyl]methylen]-2,5-cyclohexadien-1-yliden]-N-methyl-3-sulfo-propan-1-aminium-natriumsalz,
N-[4-[(6-Chlorbenzo[b]thien-2-yl)[4-[methyl(3-sulfopropyl)amino]phenyl]methylen]-2,5-cyclohexadien-1-yliden]-N-methyl-3-sulfo-propan-1-aminium-natriumsalz,
N-Methyl-N-[4-[[4-[methyl(3-sulfopropyl)amino]phenyl](5-nitro-2-benzofuranyl)methylen]-2,5-cyclohexadien-1-yliden]-3-sulfo-propan-1-aminium-natriumsalz,
N-Methyl-N-[4-[(5-methyl-2-benzofuranyl)[4-[methyl(3-sulfopropyl)amino]phenyl]methylen]-2,5-cyclohexadien-1-yliden]-3-sulfo-propan-1-aminium-natriumsalz,
N-[4-[(5-Chlor-2-benzofuranyl)[4-[methyl(3-sulfopropyl)amino]phenyl]methylen]-2,5-cyclohexadien-1-yliden]-N-methyl-3-sulfo-propan-1-aminium-natriumsalz,
inneres Salz des N-[4-[2-Benzofuranyl[4-[methyl(3-sulfopropyl)amino]phenyl]methylen]-2,5-cyclohexadien-1-yliden]-N-methyl-3-sulfo-propan-1-aminium-natriumsalzes,
N-[4-[(2-Chlorphenyl)(1-methyl-2-phenyl-1H-indol-3-yl)methylen]-2,5-cyclohexadien-1-yliden]-N-ethyl-ethanaminium-trichlorzinkat(1-),
N-[4-[(2-Chlorphenyl)(1-methyl-2-phenyl-1H-indol-3-yl)methylen]-2,5-cyclohexadien-1-yliden]-N-ethyl-ethanaminium-trichlorzinkat(1-),
5-[(1,2-Dimethyl-1 H-indol-3-yl)[4-[(2-methylphenyl)imino]-3-sulfo-2,5-cyclohexadien-1-yliden]-methyl]-2-[(2-methylphenyl)amino]benzolsulfonsäure-mononatriumsalz,
N-[4-[(2-Chlorphenyl)(1-methyl-2-phenyl-1H-indol-3-yl)methylen]-2,5-cyclohexadien-1-yliden]-N-ethyl-ethanaminium-methylsulfat,
1,2-Dihydro-4-[(4-hydroxy-3-methoxyphenyl)(3-methoxy-4-oxo-2,5-cyclohexadien-1-yliden)-methyl]-1,5-dimethyl-2-phenyl-3H-pyrazol-3-onion(1-),
N-[4-[(2-Chlorphenyl)(1-methyl-2-phenyl-1H-indol-3-yl)methylen]-2,5-cyclohexadien-1-yliden]-N-ethyl-ethanaminiumacetat,
N-[4-[(2-Chlorphenyl)(1-methyl-2-phenyl-1H-indol-3-yl)methylen]-2,5-cyclohexadien-1-yliden]-N-ethyl-ethanaminiumphosphat (1:1),
N-[4-[(2-Chlorphenyl)(1-methyl-2-phenyl-1H-indol-3-yl)methylen]-2,5-cyclohexadien-1-yliden]-N-ethyl-ethanaminium,
N-[4-[(2-Chlorphenyl)(1-methyl-2-phenyl-1H-indol-3-yl)methylen]-2,5-cyclohexadien-1-yliden]-N-ethyl-ethanaminiumchlorid, Verbindung mit Zinkchlorid (ZnCl₂),
N-[4-[(2-Chlorphenyl)(1-methyl-2-phenyl-1H-indol-3-yl)methylen]-2,5-cyclohexadien-1-yliden]-N-ethyl-ethanaminiumchlorid,
4-[[3,5-Bis(1,1-dimethylethyl)-4-oxo-2,5-cyclohexadien-1-yliden][3,5-bis(1,1-dimethylethyl)-4-oxyphenyl]methyl]-2,2,6,6-tetramethylpiperidin-1-yloxy,
3-[[3,5-Bis(1,1-dimethylethyl)-4-oxo-2,5-cyclohexadien-1-yliden][3,5-bis(1,1-dimethylethyl)-4-oxyphenyl]methyl]-2,5-dihydro-2,2,5,5-tetramethyl-1H-pyrrol-1-yloxy,
3-[[3,5-Bis(1,1-dimethylethyl)-4-hydroxyphenyl][3,5-bis(1,1-dimethylethyl)-4-oxo-2,5-cyclohexadien-1-yliden]methyl]-2,5-dihydro-2,2,5,5-tetramethyl-1H-pyrrol-1-yloxy,
4-[[3,5-Bis(1,1-dimethylethyl)-4-hydroxyphenyl][3,5-bis(1,1-dimethylethyl)-4-oxo-2,5-cyclohexadien-1-yliden]methyl]-2,2,6,6-tetramethylpiperidin-1-yloxy,
N-Nethyl-N-[[2-[[4-oxo-3-[2,2,2-trifluor-1-hydroxy-1-(trifluormethyl)ethyl]-1(4H)-naphthalinyliden](2,3,6,7-tetrahydro-1H,5H-benzo[ij]chinolizin-9-yl)methyl]phenyl]sulfonyl]acetamid,
[[2-[(3,5-Dimethoxy-4-oxo-2,5-cyclohexadien-1-yliden)(2,3,6,7-tetrahydro-1H,5H-benzo[ij]-chinolizin-9-yl)methyl]phenyl]sulfonyl]methylcarbaminsäure-[2-(methylsulfonyl)ethyl]ester,
Methyl[[2-[[4-oxo-3-[2,2,2-trifluor-1-hydroxy-1-(trifluormethyl)ethyl]-1(4H)-naphthalinyliden]-(2,3,6,7-tetrahydro-1H,5H-benzo[ij]chinolizin-9-yl)methyl]phenyl]sulfonyl]carbaminsäure-[2-cyanethyl]ester,
Methyl[[2-[[4-oxo-3-[2,2,2-trifluor-1-hydroxy-1-(trifluormethyl)ethyl]-1(4H)-naphthalinyliden]-(2,3,6,7-tetrahydro-1H,5H-benzo[ij]chinolizin-9-yl)methyl]phenyl]sulfonyl]carbaminsäure-[2-(methylsulfonyl)ethyl]ester,
Methyl[[2-[[4-oxo-3-[2,2,2-trifluor-1-hydroxy-1-(trifluormethyl)ethyl]-1(4H)-naphthalinyliden]-(2,3,6,7-tetrahydro-1H,5H-benzo[ij]chinolizin-9-yl)methyl]phenyl]sulfonyl]carbaminsäure-[2-(phenylsulfonyl)ethyl]ester,
N-[[2-[(3,5-Dimethyl-4-oxo-2,5-cyclohexadien-1-yliden)(2,3,6,7-tetrahydro-1H,5H-benzo[ij]-chinolizin-9-yl)methyl]phenyl]sulfonyl]-N-methyl-acetamid,
4-[(4-Hydroxy-3,5-dimethylphenyl)(2,3,5,6,8,9,11,12-octahydro-1,4,7,10,13-benzopentaoxacyclopentadecin-15-yl)methylen]-2,6-dimethyl-2,5-cyclohexadien-1-on,
4-[[3,5-Bis(1,1-dimethylethyl)-4-oxo-2,5-cyclohexadien-1-yliden](2,3,5,6,8,9,11,12-octahydro-1,4,7,10,13-benzopentaoxacyclopentadecin-15-yl)methyl]-2,6-bis(1,1-dimethylethyl)phenoxy,
N-[4-[(2-Chlorphenyl)(1-methyl-2-phenyl-1H-indol-3-yl)methylen]-2,5-cyclohexadien-1-yliden]-N-ethyl-ethanaminiumchlorid,
4-[[3,5-Bis(1,1-dimethylethyl)-4-oxo-2,5-cyclohexadien-1-yliden]-3-pyridinylmethyl]-2,6-bis(1,1-dimethylethyl)phenoxy,
4-[[3,5-Bis(1,1-dimethylethyl)-4-hydroxyphenyl]-3-pyridinylmethylen]-2,6-bis(1,1-dimethylethyl)-2,5-cyclohexadien-1-onion(1-),
4-[[3,5-Bis(1,1-dimethylethyl)-4-hydroxyphenyl]-3-pyridinylmethylen]-2,6-bis(1,1-dimethylethyl)-2,5-cyclohexadien-1-on,
4,4'-[3,5-Pyridindiylbis[[3,5-bis(1,1-dimethylethyl)-4-oxo-2,5-cyclohexadien-1-yliden]methylen]]-bis-2,6-bis(1,1-dimethylethyl)phenoxy,
4,4'-[2,4-Pyridindiylbis[[3,5-bis(1,1-dimethylethyl)-4-oxo-2,5-cyclohexadien-1-yliden]methylen]]-bis-2,6-bis(1,1-dimethylethyl)phenoxy,
4,4'-[2,5-Pyridindiylbis[[3,5-bis(1,1-dimethylethyl)-4-oxo-2,5-cyclohexadien-1-yliden]methylen]]-bis-2,6-bis(1,1-dimethylethyl)phenoxy,
N-[4-[9H-Carbazol-3-yl[4-(dimethylamino)phenyl]methylen]-2,5-cyclohexadien-1-yliden]-N-methyl-methanaminiumchlorid,
[4-[α-Carbazol-3-yl-p-(dimethylamino)benzyliden]-2,5-cyclohexadien-1-yliden]dimethyl-ammoniumchlorid,
4-Ethoxy-N-[4-[[4-[(4-ethoxyphenyl)amino]phenyl](1-methyl-2-phenyl-1H-indol-3-yl)methylen]-2,5-cyclohexadien-1-yliden]benzolaminacetat,
4-Ethoxy-N-[4-[[4-[(4-ethoxyphenyl)amino]phenyl](1-methyl-2-phenyl-1H-indol-3-yl)methylen]-2,5-cyclohexadien-1-yliden]benzolamin,
4,4'-[3,5-Pyridindiylbis[[3,5-bis(1,1-dimethylethyl)-4-hydroxyphenyl]methylidin]]bis[2,6-bis(1,1-dimethylethyl)-2,5-cyclohexadien-1-on]ion(2-),
4,4'-[2,6-Pyridindiylbis[[3,5-bis(1,1-dimethylethyl)-4-hydroxyphenyl]methylidin]]bis[2,6-bis(1,1-dimethylethyl)-2,5-cyclohexadien-1-on]ion(2-),
4,4'-[2,5-Pyridindiylbis[[3,5-bis(1,1-dimethylethyl)-4-hydroxyphenyl]methylidin]]bis[2,6-bis(1,1-dimethylethyl)-2,5-cyclohexadien-1-on]ion(2-),
4,4'-[2,4-Pyridindiylbis[[3,5-bis(1,1-dimethylethyl)-4-hydroxyphenyl]methylidin]]bis[2,6-bis(1,1-dimethylethyl)-2,5-cyclohexadien-1-on]ion(2-),
4,4'-[3,5-Pyridindiylbis[[3,5-bis(1,1-dimethylethyl)-4-hydroxyphenyl]methylidin]]bis[2,6-bis(1,1-dimethylethyl)-2,5-cyclohexadien-1-on],
4,4'-[2,5-Pyridindiylbis[[3,5-bis(1,1-dimethylethyl)-4-hydroxyphenyl]methylidin]]bis[2,6-bis(1,1-dimethylethyl)-2,5-cyclohexadien-1-on],
4,4'-[2,4-Pyridindiylbis[[3,5-bis(1,1-dimethylethyl)-4-hydroxyphenyl]methylidin]]bis[2,6-bis-1,1-dimethylethyl)-2,5-cyclohexadien-1-on],
N-Methyl-N-[4-[(2-methyl-1H-indol-3-yl)phenylmethylen]-2,5-cyclohexadien-1-yliden]cyclohexanaminium-trichlorzinkat(1-),
N-Methyl-N-[4-[(2-methyl-1H-indol-3-yl)phenylmethylen]-2,5-cyclohexadien-1-yliden]cyclohexanaminium-trichlorzinkat(1-),
N-Methyl-N-[4-[(2-methyl-1H-indol-3-yl)phenylmethylen]-2,5-cyclohexadien-1-yliden]-cyclohexanaminium,
N-[4-[(2-Chlorphenyl)(1-methyl-2-phenyl-1H-indol-3-yl)methylen]-2,5-cyclohexadien-1-yliden]-N-methyl-cyclohexanaminium-trichlorzinkat(1-),
N-[4-[(2-Chlorphenyl)-(1-methyl-2-phenyl-1H-indol-3-yl)methylen]-2,5-cyclohexadien-1-yliden]-N-methylcyclohexanaminium-trichlorzinkat(1-),
N-[4-[(2-Chlorphenyl)(1-methyl-2-phenyl-1H-indol-3yl)methylen]-2,5-cyclohexadien-1-yliden]-N-methyl-cyclohexanaminium,
N-[4-[(2-Chlorphenyl)[2-(4-chlorphenyl)-1-methyl-1H-indol-3-yl]methylen]-2,5-cyclohexadien-1-yliden]-N-methyl-cyclohexanaminium-trichlorzinkat(1-),
N-[4-[(2-Chlorphenyl)[2-(4-chlorphenyl)-1-methyl-1H-indol-3-yl]methylen]-2,5-cyclohexadien-1-yliden]-N-methylcyclohexanaminium-trichlorzinkat,
N-[4-[(2-Chlorphenyl)[2-(4-chlorphenyl)-1-methyl-1H-indol-3-yl]methylen]-2,5-cyclohexadien-1-yliden]-N-methyl-cyclohexanaminium,
N-Cyclohexyl-N-[4-[[1-(2-ethoxyethyl)-2-phenyl-1H-indol-3-yl](4-methylphenyl)methylen]-2,5-cyclohexadien-1-yliden]benzolmethanaminium-trichlorzinkat(1-),
N-Cyclohexyl-N-[4-[[1-(2-ethoxyethyl)-2-phenyl-1H-indol-3-yl](4-methylphenyl)methylen]-2,5-cyclohexadien-1-yliden]benzolmethanaminium-trichlorzinkat,
N-Cyclohexyl-N-[4-[[1-(2-ethoxyethyl)-2-phenyl-1H-indol-3-yl](4-methylphenyl)methylen]-2,5-cyclohexadien-1-yliden]benzolmethanaminium,
N-Methyl-N-[4[(1-methyl-2-phenyl-1H-indol-3-yl)phenylmethylen]-2,5-cyclohexadien-1-yliden]-cyclohexanaminium-trichlorzinkat(1-),
N-Methyl-N-[4-[(1-methyl-2-phenyl-1H-indol-3-yl)phenylmethylen]-2,5-cyclohexadien-1-yliden]-cyclohexanaminium-trichlorzinkat,
N-Methyl-N-[4-[(1-methyl-2-phenyl-1H-indol-3-yl)phenylmethylen]-2,5-cyclohexadien-1-yliden]-cyclohexanaminium,
N-[4-[(2-Bromphenyl)(1,2-dimethyl-1H-indol-3-yl)methylen]-2,5-cyclohexadien-1-yliden]-N-ethyl-cyclohexanaminium-trichlorzinkat(1-),
N-[4-[(2-Bromphenyl)(1,2-dimethyl-1H-indol-3-yl)methylen]-2,5-cyclohexadien-1-yliden]-N-ethylcyclohexanaminium-trichlorzinkat,
N-[4-[(2-Bromphenyl)(1,2-dimethyl-1H-indol-3-yl)methylen]-2,5-cyclohexadien-1-yliden]-N-ethyl-cyclohexanaminium,
N-[3-Chlor-4-[(1-methyl-2-phenyl-1H-indol-3-yl)phenylmethylen]-2,5-cyclohexadien-1-yliden]-N-ethyl-ethanaminiumsulfat (1:1),
N-[3-Chlor-4-[(1-methyl-2-phenyl-1H-indol-3-yl)phenylmethylen]-2,5-cyclohexadien-1-yliden]-N-ethyl-ethanaminium,
N-Ethyl-N-[4-[(2-methyl-1 H-indol-3-yl)(2-nitrophenyl)methylen]-2,5-cyclohexadien-1-yliden]-ethanaminiumsulfat (1:1),
N-Ethyl-N-[4-[(2-methyl-1H-indol-3-yl)(2-nitrophenyl)methylen]-2,5-cyclohexadien-1-yliden]-ethanaminium,
N-[4-[(1-Methyl-2-phenyl-1H-indol-3-yl)phenylmethylen]-2,5-cyclohexadien-1-yliden]methanaminsulfat (1:1),
N-4-[(1-Methyl-2-phenyl-1H-indol-3-yl)phenylmethylen]-2,5-cyclohexadien-1-yliden]methanamin,
N-[4-[[4-(Dimethylamino)phenyl]-2-furanylmethylen]-2,5-cyclohexadien-1-yliden]-N-methyl-methanaminium-(T-4)-tetrabromthallat(1-),
N-[4-[[4-(Dimethylamino)phenyl]-2-furanylmethylen]-2,5-cyclohexadien-1-yliden]-N-methyl-methanaminium-(T-4)-tetrabromthallat(1-),
4-[[3,5-Bis(1,1-dimethylethyl)-4-oxo-2,5-cyclohexadien-1-yliden][6-[[3,5-bis(1,1-dimethylethyl)-4-oxo-2,5-cyclohexadien-1-yliden][3,5-bis(1,1-dimethylethyl)-4-oxyphenyl]methyl]-2-pyridinyl]-methyl]-2,6-bis(1,1-dimethylethyl)phenoxy
4,4'-[2,6-Pyridindiylbis[[3,5-bis(1,1-dimethylethyl)-4-hydroxyphenyl]methylidin]]bis[2,6-bis(1,1-dimethylethyl)-2,5-cyclohexadien-1-on],
N-[4-[(2,3-Dihydro-1,5-dimethyl-3-oxo-2-phenyl-1H-pyrazol-4-yl)(3-nitrophenyl)methylen]-2,5-cyclohexadien-1-yliden]-N-methyl-methanaminiumchlorid,
N-[4-[(3-Bromphenyl)(2,3-dihydro-1,5-dimethyl-3-oxo-2-phenyl-1H-pyrazol-4-yl)methylen]-2,5-cyclohexadien-1-yliden]-N-methyl-methanaminiumchlorid,
N-[4-[(2,3-Dihydro-1,5-dimethyl-3-oxo-2-phenyl-1H-pyrazol-4-yl)(3-methoxyphenyl)methylen]-2,5-cyclohexadien-1-yliden]-N-methyl-methanaminiumchlorid,
N-[4-[(2,3-Dihydro-1,5-dimethyl-3-oxo-2-phenyl-1H-pyrazol-4-yl)phenylmethylen]-2,5-cyclohexadien-1-yliden]-N-methyl-methanaminiumchlorid,
[[4-[(1-Methyl-2-phenyl-1H-indol-3-yl)[4-[(sulfophenyl)amino]phenyl]methylen]-2,5-cyclohexadien-1-yliden]amino],
N-[4-[(2,3-Dihydro-1,5-dimethyl-3-oxo-2-phenyl-1H-pyrazol-4-yl)[4-(dimethylamino)phenyl]-methylen]-2,5-cyclohexadien-1-yliden]-N-methyl-methanaminiumchlorid,
4-[(2-[1,1'-Biphenyl]-4-yl-1-butyl-1H-indol-3-yl)[4-[(4-ethoxyphenyl)imino]-2,5-cyclohexadien-1-ylidenmethyl]-N-(4-ethoxyphenyl)benzolamin,
N-Methyl-N-[4-[[4-(methylphenylamino)phenyl](4-oxo-4H-1-benzopyran-3-yl)methylen]-2,5-cyclohexadien-1-yliden]benzolaminium,
N-[4-[[4-(Diethylamino)phenyl](4-oxo-4H-1-benzopyran-3-yl)methylen]-2,5-cyclohexadien-1-yliden]-N-ethyl-ethanaminium,
4-Ethoxy-N-methyl-N-[4-[(1-methyl-2-phenyl-1H-indol-3-yl)phenylmethylen]-2,5-cyclohexadien-1-yliden]benzolaminiumchlorid,
N-[4-[12H-Benzo[a]phenoxazin-5-yl[4-(dimethylamino)phenyl]methylen]-2,5-cyclohexadien-1-yliden]-N-methyl-methanaminiumchlorid,
N-Methyl-N-[4-[(1-methyl-2-phenyl-1H-indol-3-yl)phenylmethylen]-2,5-cyclohexadien-1-yliden]-benzolaminium,
N-[4-[[4-(Diethylamino)phenyl](7-methoxy-2,1,3-benzothiadiazol-4-yl)methylen]-2,5-cyclohexadien-1-yliden]-N-ethyl-ethanaminium,
N-[4-[[4-(Diethylamino)phenyl](5-methoxy-2,1,3-benzothiadiazol-4-yl)methylen]-2,5-cyclohexadien-1-yliden]-N-ethyl-ethanaminium,
N-[4-[[4-(Diethylamino)phenyl](7-methyl-2,1,3-benzothiadiazol-4-yl)methylen]-2,5-cyclohexadien-1-yliden]-N-ethyl-ethanaminium,
N-[4-[2,1,3-Benzothiadiazol-5-yl-[4-(diethylamino)phenyl]methylen]-2,5-cyclohexadien-1-yliden]-N-ethyl-ethanaminium,
N-[4-[2,1,3-Benzothiadiazol-4-yl[4-(diethylamino)phenyl]methylen]-2,5-cyclohexadien-1-yliden]-N-ethyl-ethanaminium,
N-Methyl-N-[4-[(1-methyl-2-phenyl-1H-indol-3-yl)phenylmethylen]-2,5-cyclohexadien-1-yliden]-methanaminiumchlorid,
N-Methyl-N-[4-[(1-methyl-2-phenyl-1H-indol-3-yl)phenylmethylen]-2,5-cyclohexadien-1-yliden]-methanaminium,
N-[4-[[4-(Dimethylamino)phenyl](7-methoxy-2,1,3-benzothiadiazol-4-yl)methylen]-2,5-cyclohexadien-1-yliden]-N-methyl-methanaminium,
N-[4-[[4-(Dimethylamino)phenyl](5-methoxy-2,1,3-benzothiadiazol-4-yl)methylen]-2,5-cyclohexadien-1-yliden]-N-methyl-methanaminium,
N-[4-[[4-(Dimethylamino)phenyl](7-methyl-2,1,3-benzothiadiazol-4-yl)methylen]-2,5-cyclohexadien-1-yliden]-N-methyl-methanaminium,
N-[4-[2,1,3-Benzothiadiazol-5-yl-[4-dimethylamino)phenyl]methylen]-2,5-cyclohexadien-1-yliden]-N-methyl-methanaminium,
N-[4-[2,1,3-Benzothiadiazol-4-yl-[4-(dimethylamino)phenyl]methylen]-2,5-cyclohexadien-1-yliden]-N-methyl-methanaminium,
N-[4-[[4-(Dimethylamino)phenyl]-3-pyridinylmethylen]-2,5-cyclohexadien-1-yliden]-N-ethyl-methanaminium,
N-[4-[[4-(Dimethylamino)phenyl]-4-pyridinylmethylen]-2,5-cyclohexadien-1-yliden]-N-methyl-methanaminium,
N-[4-[[4-(Dimethylamino)phenyl]-2-thienylmethylen]-2,5-cyclohexadien-1-yliden]-N-methyl-methanaminium,
N-[4-[[4-(Dimethylamino)phenyl]-2-furanylmethylen]-2,5-cyclohexadien-1-yliden]-N-methyl-methanaminium,
N-[4-[[4-(Dimethylamino)phenyl]-3-thienylmethylen]-2,5-cyclohexadien-1-yliden]-N-methyl-methanaminium,
[[4-[(1-Methyl-2-phenyl-1H-indol-3-yl)[4-[(sulfophenyl)amino]phenyl]methylen]-2,5-cyclohexadien-1-yliden]amino],
[[4-[[-4[(Ethoxysulfophenyl)amino]phenyl](1-methyl-2-phenyl-1H-indol-3-yl)methylen]-2,5-cyclohexadien-1-yliden]amino]ethoxy,
N-Methyl-4-[[4-(methylimino)-2,5-cyclohexadien-1-yliden](2-methyl-1H-indol-3-yl)methyl]-benzolamin,
N-[4-[[4-(Dimethylamino)phenyl](2-methyl-1H-indol-3-yl)methylen]-2,5-cyclohexadien-1-yliden]-N-methyl-methanaminium,
N-Methyl-4-[[4-(methylimino)-2,5-cyclohexadien-1-yliden](1-methyl-2-phenyl-1H-indol-3-yl)methyl]benzolamin, konjugierte Monosäure,
N-[4-[[4-(Dimethylamino)phenyl](1-methyl-2-phenyl-1H-indol-3-yl)methylen]-2,5-cyclohexadien-1-yliden]-N-methyl-methanaminium,
4-Ethoxy-N-[4-[[4-[(4-ethoxyphenyl)amino]phenyl](2-phenyl-1 H-indol-3-yl)methylen]-2,5-cyclohexadien-1-yliden]benzolamin,
4-Ethoxy-N-[4-[[4-[(4-ethoxyphenyl)amino]phenyl](1-methyl-1H-indol-3-yl)methylen]-2,5-cyclohexadien-1-yliden]benzolamin,
N-[4-[(1,2-Dimethyl-1H-indol-3-yl)[4-[(4-ethoxyphenyl)amino]phenyl]methylen]-2,5-cyclohexadien-1-yliden]-4-ethoxybenzolamin,
4-[(1-Methyl-2-phenyl-1H-indol-3-yl)[4-(phenylimino)-2,5-cyclohexadien-1-yliden]methyl]-N-phenyl-benzolamin, konjugierte Monosäure,
4-Ethoxy-N-[4-[[4-[(4-ethoxyphenyl)amino]phenyl](1-methyl-2-phenyl-1H-indol-3-yl)methylen]-2,5-cyclohexadien-1-yliden]benzolamin,
N-[4-[[4-(Dimethylamino)phenyl](2-phenyl-1H-indol-3-yl)methylen]-2,5-cyclohexadien-1-yliden]-N-methyl-methanaminium,
N-Methyl-N-[4-[[4-(methylphenylamino)phenyl](4-oxo-4H-1-benzopyran-3-yl)methylen]-2,5-cyclohexadien-1-yliden]benzolaminiumchlorid,
N-Methyl-N-[4-[[4-(methylphenylamino)phenyl](4-oxo-4H-1-benzopyran-3-yl)methylen]-2,5-cyclohexadien-1-yliden]benzolaminiumperchlorat,
N-[4-[[4-(Diethylamino)phenyl](4-oxo-4H-1-benzopyran-3-yl)methylen]-2,5-cyclohexadien-1-yliden]-N-ethyl-ethanaminiumperchlorat,
N-[4-[[4-(Diethylamino)phenyl](4-oxo-4H-1-benzopyran-3-yl)methylen]-2,5-cyclohexadien-1-yliden]-N-ethyl-ethanaminiumchlorid,
N-Methyl-N-[4-[[4-(methylphenylamino)phenyl](4-oxo-4H-1-benzopyran-3-yl)methylen]-2,5-cyclohexadien-1-yliden]benzolaminiumacetat,
N-[4-[[4-(Diethylamino)phenyl](4-oxo-4H-1-benzopyran-3-yl)methylen]-2,5-cyclohexadien-1-yliden]-N-ethyl-ethanaminiumacetat,
N-[4-[(2,3-Dihydro-1,5-dimethyl-3-oxo-2-phenyl-1 H-pyrazol-4-yl)(3-nitrophenyl)methylen]-2,5-cyclohexadien-1-yliden]-N-methyl-methanaminium,
N-[4-[(3-Bromphenyl)(2,3-dihydro-1,5-dimethyl-3-oxo-2-phenyl-1H-pyrazol-4-yl)methylen]-2,5-cyclohexadien-1-yliden]-N-methyl-methanaminium,
N-[4-[(2,3-Dihydro-1,5-dimethyl-3-oxo-2-phenyl-1 H-pyrazol-4-yl)(3-methoxyphenyl)methylen]-2,5-cyclohexadien-1-yliden]-N-methyl-methanaminium,
N-[4-[(2,3-Dihydro-1,5-dimethyl-3-oxo-2-phenyl-1H-pyrazol-4-yl)phenylmethylen]-2,5-cyclohexadien-1-yliden]-N-methyl-methanaminium,
N-[4-[(2,3-Dihydro-1,5-dimethyl-3-oxo-2-phenyl-1H-pyrazol-4-yl)(3-methylphenyl)methylen]-2,5-cyclohexadien-1-yliden]-N-methyl-methanaminium,
N-[4-[(1-Ethyl-2-phenyl-1H-indol-3-yl)[4-[(2-methoxyphenyl)amino]phenyl]methylen]-2,5-cyclohexadien-1-yliden]-2-methoxybenzolamin-monohydrochlorid,
2-Hydroxy-5-[[4-[[4-[(4-hydroxy-3-sulfophenyl)amino]phenyl](1-methyl-2-phenyl-1H-indol-3-yl)methylen]-2,5-cyclohexadien-1-yliden]amino],
2-Ethoxy-N-[4-[[-4-(2-ethoxyphenyl)amino]phenyl](1-methyl-2-phenyl-1H-indol-3-yl)methylen]-2,5-cyclohexadien-1-yliden]benzolamin-monohydrochlorid,
N-[4-[[4-(Dimethylamino)phenyl]-2-furanylmethylen]-2,5-cyclohexadien-1-yliden]-N-methyl-methanaminiumperchlorat,
2-Ethoxy-5-[[4-[[4-[(4-ethoxy-3-sulfophenyl)amino]phenyl](1-methyl-2-phenyl-1H-indol-3-yl)methyl]-2,5-cyclohexadien-1-yliden]amino],
[4-[α-[p-(Dimethylamino)phenyl]furfuryliden]-2,5-cyclohexadien-1-yliden]dimethylammonium-tetrachlorgallat(1-),
N-[4-[[4-(Dimethylamino)phenyl]-2-thienylmethyl]-2,5-cyclohexadien-1-yliden]-N-methyl-methanaminiumperchlorat,
[4-[p-(Dimethylamino)-α-2-thienylbenzyliden]-2,5-cyclohexadien-1-yliden]dimethyl-ammonium-perchlorat,
(2-Cyanethyl)[4-[α-[4-[(2-cyanethyl)ethylamino]-o-tolyl]piperonyliden]-3-methyl-2,5-cyclohexadien-1-yliden]ethylammonium,
Dimethyl[4-[α-(1-methyl-2-phenylindol-3-yl)benzyliden]-2,5-cyclohexadien-1-yliden]ammoniumchlorid,
(p-Ethoxyphenyl)methyl[4-[α-(1-methyl-2-phenylindol-3-yl)benzyliden]-2,5-cyclohexadien-1-yliden]ammoniumchlorid,
(p-Ethoxyphenyl)methyl[4-[α-(1-methyl-2-phenylindol-3-yl)benzyliden]-2,5-cyclohexadien-1-yliden]ammoniumchlorid,
[4-[α-2,1,3-Benzothiadiazol-4-yl-p-(dimethylamino)benzyliden]-2,5-cyclohexadien-1-yliden]-dimethylammoniumperchlorat,
[4-[p-(Dimethylamino)-α-(5-methoxy-2,1,3-benzothiadiazol-4-yl)benzyliden]-2,5-cyclohexadien-1-yliden]dimethylammoniumperchlorat,
[4-[p-(Diethylamino)-α-(5-methoxy-2,1,3-benzothiadiazol-4-yl)benzyliden]-2,5-cyclohexadien-1-yliden]diethylammoniumperchlorat,
[4-[p-(Diethylamino)-α-(7-methoxy-2,1,3-benzothiadiazol-4-yl)benzyliden]-2,5-cyclohexadien-1-yliden]diethylammoniumperchlorat,
[4-[p-(Dimethylamino)-α-(7-methoxy-2,1,3-benzothiadiazol-4-yl)benzyliden]-2,5-cyclohexadien-1-yliden]dimethylammoniumperchlorat,
[4-[p-(Diethylamino)-α-(7-methyl-2,1,3-benzothiadiazol-4-yl)benzyliden]-2,5-cyclohexadien-1-yliden]diethylammoniumperchlorat,
[4-[p-(Dimethylamino)-α-(7-methyl-2,1,3-benzothiadiazol-4-yl)benzyliden]-2,5-cyclohexadien-1-yliden]dimethylammoniumperchlorat,
[4-[α-2,1,3-Benzothiadiazol-4-yl-p-(diethylamino)benzyliden]-2,5-cyclohexadien-1-yliden]-diethylammoniumperchlorat,
[4-[α-2,1,3-Benzothiadiazol-5-yl-p-(diethylamino)benzyliden]-2,5-cyclohexadien-1-yliden]-diethylammoniumperchlorat,
[4-[α-2,1,3-Benzothiadiazol-5-yl-p-(dimethylamino)benzyliden]-2,5-cyclohexadien-1-yliden]-dimethylammoniumperchlorat,
Methyl-[4-[α-(1-methyl-2-phenylindol-3-yl)benzyliden]-2,5-cyclohexadien-1-yliden]phenyl-ammonium-trichlorzinkat(1-),
Methyl-[4-[α-(1-methyl-2-phenylindol-3-yl)benzyliden]-2,5-cyclohexadien-1-yliden]phenyl-ammonium-trichlorzinkat,
Dimethyl-[4-[α-(1-methyl-2-phenylindol-3-yl)benzyliden]-2,5-cyclohexadien-1-yliden]-ammonium-trichlorzinkat,
Dimethyl-[4-[α-(1-methyl-2-phenylindol-3-yl)benzyliden]-2,5-cyclohexadien-1-yliden]-ammonium-trichlorzinkat,
3-[α-(4-Imino-2,5-cyclohexadien-1-yliden)benzyl]-1,2-dimethylindol-monohydrochlorid,
3-[α-[4-[(p-Ethoxyphenyl)imino]-2,5-cyclohexadien-1-yliden]benzyl]-1-methyl-2-phenylindol-monohydrochlorid,
[4-[α-[9-(2-Cyanethyl)carbazol-3-yl]-p-(dimethylamino)benzyliden]-2,5-cyclohexadien-1-yliden]dimethylammonium,
[4-[α-Carbazol-3-yl-p-(dimethylamino)benzyliden]-2,5-cyclohexadien-1-yliden]dimethylammonium,
[4-[α-[9-(2-Cyanethyl)carbazol-3-yl]benzyliden]-2,5-cyclohexadien-1-yliden]dimethylammonium,
[4-(α-Carbazol-3-ylbenzyliden)-2,5-cyclohexadien-1-yliden]dimethylammonium, 2,6-Di-tert-butyl-4-(a-morpholinobenzyliden)-2,5-cyclohexadien-1-on,
2,6-Di-tert-butyl-4-[a-(2,2-dimethyl-1-aziridinyl)benzyliden]-2,5-cyclohexadien-1-on,
4-(α-1-Aziridinylbenzyliden)-2,6-di-tert-butyl-2,5-cyclohexadien-1-on,
2,6-Bis(1,1-dimethylethyl)-4-(phenyl-1-piperidinylmethylen)-2,5-cyclohexadien-1-on, 2,6-Di-tert-butyl-4-(a-piperidinobenzyliden)-2,5-cyclohexadien-1-on,
[4-[α-[4-(Diethylamino)-o-tolyl]piperonyl]-3-methyl-2,5-cyclohexadien-1-yliden]diethyl-ammoniumchlorid,
[4-[α-[4-(Diethylamino)-o-tolyl]-2-thenyliden]-3-methyl-2,5-cyclohexadien-1-yliden]diethyl-ammoniumchlorid,
[4-[α-[p-(Dimethylamino)phenyl]-5-nitrofurfuryliden]-2,5-cyclohexadien-1-yliden]dimethyl-ammoniumchlorid,
[4-[5-Brom-α-[p-diethylamino)phenyl]furfuryliden]-2,5-cyclohexadien-1-yliden]diethyl-ammoniumchlorid,
[4-[5-Brom-α-[p-dimethylamino)phenyl]furfuryliden]-2,5-cyclohexadien-1-yliden]dimethyl-ammoniumchlorid,
[4-[α-[p-(Dimethylamino)phenyl]-2-furfuryliden]-2,5-cyclohexadien-1-yliden]diethyl-ammoniumchlorid,
2-Hydroxy-5-[[4-[[4-[(4-hydroxy-3-sulfophenyl)amino]phenyl](1-methyl-2-phenyl-1H-indol-3-yl)methylen]-2,5-cyclohexadien-1-yliden]amino],
[4-[α-12H-Benzo[a]phenoxazin-5-yl-p-(dimethylamino)benzyliden]-2,5-cyclohexadien-1-yliden]dimethylammoniumchlorid, Verbindung mit Zinkchlorid,
[4-[α-12H-Benzo[a]phenoxazin-5-yl-p-(dimethylamino)benzyliden]-2,5-cyclohexadien-1-yliden]dimethylammoniumchlorid,
N,2-Dimethyl-4-[(2-methyl-1 H-indol-3-yl) [3-methyl-4-(methylimino)-2,5-cyclohexadien-1-yliden]methyl]benzolamin-monohydrochlorid,
2-Methyl-3-[3-methyl-4-(methylamino)-α-[3-methyl-4-(methylimino)-2,5-cyclohexadien-1-yliden]benzyl]indol-monohydrochlorid,
N-[4-[[4-(Dimethylamino)phenyl]-2-furanylmethylen]-2,5-cyclohexadien-1-yliden]-N-methyl-methanaminiumchlorid,
[4-[α-[p-Dimethylamino)phenyl]furfuryliden]-2,5-cyclohexadien-1-yliden]dimethylammonium-chlorid,
N-[4-[[2-(4-Chlorphenyl)-4,6-dimethyl-1-(2-methylpropyl)-1H-indol-3-yl](2,4-disulfophenyl)-methylen]-2,5-cyclohexadien-1-yliden]-4-methoxy-N-methyl-benzolaminium,
[4-[α-[2-(p-Chlorphenyl)-1-isobutyl-4,6-dimethylindol-3-yl]-2,4-disulfobenzyliden]-2,5-cyclohexadien-1-yliden](p-methoxyphenyl)methylammoniumhydroxid,
2-Ethoxy-5-[[4-[[4-[(4-ethoxy-3-sulfophenyl)amino]phenyl](1-methyl-2-phenyl-1H-indol-3-yl)methylen]-2,5-cyclohexadien-1-yliden]amino],
5-[(3-Carboxy-5-methyl-4-oxo-2,5-cyclohexadien-1-yliden)(4,5-dihydro-3-methyl-5-oxo-1-phenyl-1H-pyrazol-4-yl)methyl]-2-hydroxy-3-methyl-,
5-[[4-[[2-(4-Chlorphenyl)-4,6-dimethyl-1-(2-methylpropyl)-1H-indol-3-yl][4-[(4-ethoxy-3-sulfophenyl)amino]phenyl]methylen]-2,5-cyclohexadien-1-yliden]amino]-2-ethoxy-,
N-[α-[2-(p-Chlorphenyl)-1-isobutyl-4,6-dimethylindol-3-yl]-α-[4-[(4-ethoxy-3-sulfophenyl)imino]-2,5-cyclohexadien-1-yliden]-p-tolyl]-6-ethoxy-,
6-[(4-Ethoxyphenyl)imino]-3-[(1-methyl-2-phenyl-1H-indol-3-yl)[4-[(2-sulfoethyl)amino]phenyl]-methylen]-,
6-[(p-Ethoxyphenyl)imino]-3-[a-(1-methyl-2-phenylindol-3-yl)-p-[(2-sulfoethyl)amino]-benzyliden]-,
N-[4-[[4-(Dimethylamino)phenyl]-3-thienylmethylen]-2,5-cyclohexadien-1-yliden]-N-methyl-methanaminiumperchlorat,
[4-[α-[p-(Dimethylamino)phenyl]-3-thenyliden]-2,5-cyclohexadien-1-yliden]dimethylammonium-perchlorat,
[4-[α-[p-(Dimethylamino)phenyl]furfuryliden]-2,5-cyclohexadien-1-yliden]dimethylammonium-bromid,
[4-[α-[p-(Dimethylamino)phenyl]furfuryliden]-2,5-cyclohexadien-1-yliden]dimethylammonium-bromid,
N-[4-[[4-(Dimethylamino)phenyl]-3-pyridinylmethylen]-2,5-cyclohexadien-1-yliden]-N-methyl-methanaminiumperchlorat,
[4-[p-(Dimethylamino)-α-3-pyridylbenzyliden]-2,5-cyclohexadien-1-yliden]dimethylammonium-perchlorat,
[4-[p-(Dimethylamino)-α-3-pyridylbenzyliden]-2,5-cyclohexadien-1-yliden]dimethylammonium-perchlorat,
[4-[p-(Dimethylamino)-α-4-pyridylbenzyliden]-2,5-cyclohexadien-1-yliden]dimethylammonium-perchlorat,
[4-[p-(Dimethylamino)-α-4-pyridylbenzyliden]-2,5-cyclohexadien-1-yliden]dimethylammonium-perchlorat,
[4-[p-(Dimethylamino)-α-2-pyridylbenzyliden]-2,5-cyclohexadien-1-yliden]dimethylammonium-chlorid,
[4-[p-(Dimethylamino)-α-2-pyridylbenzyliden]-2,5-cyclohexadien-1-yliden]dimethylammonium-chlorid,
und deren Additionssalze.

6. Zusammensetzung gemäß einem der vorangehenden Ansprüche, **dadurch gekennzeichnet, dass** die Verbindung(en) der Formel (I), (Ibis), (II) oder (IIbis) und/oder das oder die Additionssalz(e) 0,0001 bis 10 Gew.-% des Gesamtgewichts der Färbezusammensetzung, bevorzugt 0,005 bis 10 Gew.-% und weiter bevorzugt 0,01 bis 6 Gew.-% des Gesamtgewichts der Färbezusammensetzung ausmachen.

7. Zusammensetzung gemäß einem der Ansprüche 1 bis 6, **dadurch gekennzeichnet, dass** sie außerdem einen oder mehrere, von den Verbindung(en) der Formeln (I), (Ibis), (II) oder (IIbis) verschiedene Direktfarbstoffe enthält.

8. Zusammensetzung gemäß Anspruch 7, **dadurch gekennzeichnet, dass** der oder die von den Verbindung(en) der Formel (I), (Ibis), (II) oder (IIbis) verschiedene(n) Direktfarbstoff(e) aus den neutralen, sauren oder kationischen, nitrierten Benzoldirektfarbstoffen, den neutralen, sauren oder kationischen Azodirektfarbstoffen, den neutralen, sauren oder kationischen Chinon- und insbesondere Anthrachinondirektfarbstoffen, den Azindirektfarbstoffen, den Triarylmethandirektfarbstoffen, den Indamindirektfarbstoffen und den natürlichen Direktfarbstoffen ausgewählt sind.

9. Zusammensetzung gemäß einem der Ansprüche 7 und 8, **dadurch gekennzeichnet, dass** der oder die von den Verbindung(en) der Formeln (I), (Ibis), (II) oder (IIbis) verschiedene(n) Direktfarbstoff(e) 0,001 bis 20 Gew.-%, bevorzugt 0,005 bis 10 Gewichtsprozent des Gesamtgewichts der Färbezusammensetzung ausmachen.

10. Zusammensetzung gemäß einem der vorangehenden Ansprüche, **dadurch gekennzeichnet, dass** sie außerdem eine oder mehrere Oxidationsbase(n) und gegebenenfalls einen oder mehrere Kuppler enthält.

11. Zusammensetzung gemäß Anspruch 10, **dadurch gekennzeichnet, dass** die Oxidationsbase(n) aus den p-Phenylendiaminen, den Bisphenylalkylendiaminen, den p-Aminophenolen, den o-Aminophenolen, den heterocyclischen Basen und ihren Additionssalzen ausgewählt sind.

12. Zusammensetzung gemäß Anspruch 10, **dadurch gekennzeichnet, dass** der oder die Kuppler aus den m-Phenylendiaminkupplern, den m-Aminophenolkupplern, den m-Diphenolkupplern, den Naphthalinkupplern, den heterocyclischen Kupplern und ihren Additionssalzen ausgewählt sind.

13. Zusammensetzung gemäß einem der Ansprüche 10 bis 12, **dadurch gekennzeichnet, dass** die Oxidationsbase(n) und/oder der oder die Kuppler jeweils in einer Menge von 0,001 bis 10 Gew.-%, bevorzugt von 0,005 bis 6 Gew.-% des Gesamtgewichts der Färbezusammensetzung vorliegen.

14. Zusammensetzung gemäß einem der vorangehenden Ansprüche, **dadurch gekennzeichnet, dass** sie einen pH von 3 bis 12, bevorzugt von 5 bis 11, weiter bevorzugt von 6 bis 8,5 aufweist.

15. Verfahren zur Direktfärbung von Keratinfasern und insbesondere humaner Keratinfasern wie dem Haar, das das Aufbringen wenigstens einer wenigstens eine Verbindung der Formel (I), (Ibis), (II) oder (IIbis) enthaltenden Färbezusammensetzung gemäß einem der Ansprüche 1 bis 14 auf die Keratinfasern und dann die Einhaltung einer Ruhezeit, gefolgt vom Spülen der Fasern umfasst.

16. Verfahren gemäß Anspruch 15, **dadurch gekennzeichnet, dass** die Ruhezeit 3 bis 50 Minuten, bevorzugt 5 bis 30 Minuten beträgt.

17. Verfahren zur Färbung von Keratinfasern und insbesondere humaner Keratinfasern wie dem Haar, bei dem wenigstens eine Färbezusammensetzung, die entweder wenigstens eine Verbindung der Formel (II) oder (IIbis) oder wenigstens eine Verbindung der Formel (I) oder (Ibis) und wenigstens eine Oxidationsbase und gegebenenfalls einen oder mehrere Kuppler umfasst, auf die Fasern aufgebracht wird, wobei die Farbe durch ein Oxidationsmittel bei saurem, neutralem oder alkalischem pH entwickelt wird.

18. Verfahren gemäß Anspruch 17, **dadurch gekennzeichnet, dass** das Oxidationsmittel aus Wasserstoffperoxid, Harnstoffperoxid, den Alkalimetallbromaten, den Persalzen wie etwa den Perboraten und Persulfaten, den Persäuren und den Oxydaseenzymen wie den Peroxydasen, den 2-Elektronen-Oxidreduktionsmitteln wie den Unicasen und den 4-Elektronen-Oxygenasen wie den Laccasen ausgewählt ist.

19. Vorrichtung mit mehren Abteilungen, die eine erste Abteilung, die eine Färbezusammensetzung gemäß einem der Ansprüche 1 bis 14 enthält, die entweder wenigstens eine Verbindung der Formel (II) oder (IIbis) oder wenigstens eine Verbindung der Formel (I) oder (Ibis) und wenigstens eine Oxidationsbase und gegebenenfalls einen oder mehrere Kuppler umfasst, und eine zweite, eine Oxidationszusammensetzung enthaltende Abteilung umfasst.
